# EUROPEAN PATENT SPECIFICATION

(11) **EP 1 274 426 B1**
(45) Date of publication and mention of the grant of the patent: **14.04.2004**
(21) Application number: 01926589.1
(22) Date of filing: 17.04.2001
(51) Int. Cl.: A61K 31/422

(54) **USE OF THIOAMIDE OXAZOLIDINONES FOR THE TREATMENT OF BONE RESORPTION AND OSTEOPOROSIS**
VERWENDUNG VON THIOAMIDOXAZOLIDINONEN ZUR BEHANDLUNG VON KNOCHENRESORPTION UND OSTEOPOROSE
UTILISATION DE THIOAMIDE OXAZOLIDINONES POUR LE TRAITEMENT DE LA RESORPTION OSSEUSE ET DE L'OSTEOPOROSE

(30) Priority: 20.04.2000 US 198688 P
(43) Date of publication of application: 15.01.2003
(73) Proprietor: PHARMACIA & UPJOHN COMPANY, Kalamazoo, Michigan 49001 (US)
(72) Inventor: MESFIN, Gebre-Mariam, Portage, MI 49024 (US); JENSEN, Richard, K., Kalamazoo, MI 49008 (US)
(74) Representative: Perry, Robert Edward
(86) International application number: PCT/US2001/010805
(87) International publication number: WO 2001/080841

(56) References cited:
- EP-A- 0 710 657
- EP-A- 0 741 133
- WO-A-98/54161

## Description

### Background of the Invention

### 1. Field of the Invention

The present invention is directed to a new use for known compounds. More specifically, the invention relates to the use of thioamide oxazolidinones for the treatment of bone resorption, osteoporosis and other bone diseases.

### 2. Technology Description

Bone is not a static tissue. It is subject to constant breakdown and resynthesis in a complex process mediated by osteoblasts, which produce new bone, and osteoclasts, which destroy bone. The activities of these cells are regulated by a large number of cytokines and growth factors, many of which have now been identified and cloned. Mundy has described the current knowledge related to these factors (Mundy, G. R. Clin Orthop 324:24-28, 1996; Mundy, G. R. J Bone Miner Res 8:S505-10, 1993).

Although there is a great deal of information available on the factors which influence the breakdown and resorption of bone, information on growth factors that stimulate the formation of new bone is more limited. Investigators have searched for sources of such activities, and have found that bone tissue itself is a storehouse for factors that have the capacity for stimulating bone cells. Thus, extracts of bovine bone tissue obtained from slaughterhouses contain not only structural proteins which are responsible for maintaining the structural integrity of bone, but also biologically active bone growth factors which can stimulate bone cells to proliferate. Among these latter factors are transforming growth factor .beta., the heparin-binding growth factors (e.g., acidic and basic fibroblast growth factor), the insulin-like growth factors (e.g., insulin-like growth factor I and insulin-like growth factor II), and a recently described family of proteins called bone morphogenetic proteins (BMPs). All of these growth factors have effects on other types of cells, as well as on bone cells.

The BMPs are novel factors in the extended transforming growth factor beta. superfamily. They were first identified by Wozney J. et al. Science (1988) 242:1528-34, using gene cloning techniques, following earlier descriptions characterising the biological activity in extracts of demineralized bone (Urist M. Science (1965) 150:893-99). Recombinant BMP2 and BMP4 can induce new bone formation when they are injected locally into the subcutaneous tissues of rats (Wozney J. Molec Reprod Dev (1992) 32:160-67). These factors arc expressed by normal osteoblasts as they differentiate, and have been shown to stimulate osteoblast differentiation and bone nodule formation in vitro as well as bone formation in vivo (Harris S. et al. J. Bone Miner Res (1994) 9:855-63). This latter property suggests potential usefulness as therapeutic agents in diseases that result in bone loss.

The cells that are responsible for forming bone are osteoblasts. As osteoblasts differentiate from precursors to mature bone-forming cells, they express and secrete a number of enzymes and structural proteins of the bone matrix, including Type-1 collagen, osteocalcin, osteopontin and alkaline phosphatase (Stein G. et al Curr Opin Cell Biol (1990) 2:1018-27; Harris S. et al. (1994), supra). They also synthesize a number of growth regulatory peptides that are stored in the bone matrix, and are presumably responsible for normal bone formation. These growth regulatory peptides include the BMPs (Harris S. et al. (1994), supra). In studies of primary cultures of fetal rat calvarial osteoblasts, BMPs 1, 2, 3, 4, and 6 are expressed by cultured cells prior to the formation of mineralized bone nodules (Harris S. et al. (1994), supra). Like alkaline phosphatase, osteocalcin and osteopontin, the BMPs are expressed by cultured osteoblasts as they proliferate and differentiate.

Although the BMPs are potent stimulators of bone formation in vitro and in vivo, there are disadvantages to their use as therapeutic agents to enhance bone healing. Receptors for the bone morphogenetic proteins have been identified in many tissues, and the BMPs themselves are expressed in a large variety of tissues in specific temporal and spatial patterns. This suggests that BMPs may have effects on many tissues in addition to bone, potentially limiting their usefulness as therapeutic agents when administered systemically. Moreover, since they are peptides, they would have to be administered by injection. These disadvantages impose severe limitations to the development of BMPs as therapeutic agents.

There is a plethora of conditions that are characterized by the need to enhance bone formation. Perhaps the most obvious is the case of bone fractures, where it would be desirable to stimulate bone growth and to hasten and complete bone repair. Agents that enhance bone formation would also be useful in facial reconstruction procedures. Other bone deficit conditions include bone segmental defects, periodontal disease, metastatic bone disease, osteolytic bone disease and conditions where connective tissue repair would be beneficial, such as healing or regeneration of cartilage defects or injury. Also of great significance is the chronic condition of osteoporosis, including age-related osteoporosis and osteoporosis associated with post-menopausal hormone status. Other conditions characterized by the need for bone growth include primary and secondary hyperparathyroidism, disuse osteoporosis, diabetes-related osteoporosis, and glucocorticoid-related osteoporosis.

There are currently no satisfactory pharmaceutical approaches to managing any of these conditions. Bone fractures are still treated exclusively using casts, braces, anchoring devices and other strictly mechanical means. Further bone deterioration associated with post-menopausal osteoporosis has been treated with estrogens or bisphosphonates, which have known side effects.

U.S. Pat. No, 5,280,040 discloses compounds described as useful in the treatment of osteoporosis. These compounds putatively achieve this result by preventing bone resorption.

Wang, G.-J. et al, J Formos Med Assoc (1995) 94:589-592 report that certain lipid clearing agents, exemplified by lovastatin and bezafibrate, were able to inhibit the bone resorption resulting from steroid administration in rabbits. There was no effect on bone formation by these two compounds in the absence of steroid treatment. The mechanism of the inhibition in bone resorption observed in the presence of steroids (and the mechanism of the effect of steroid on bone per se) is said to be unknown. The authors state that steroid-induced bone loss is associated with a decrease in bone formation attributed to an inhibitory effect of corticosteroid on osteoblast activity and an increase in bone absorption due to direct osteoclast stimulation and to an indirect inhibition of intestinal calcium absorption with a secondary increase in parathyroid hormone production. Other mechanisms mentioned include those attributable to lipid abnormalitics and hyperlipidemia which lead to circulatory impairment, obstruction of subchondral vessels, osteocyte necrosis and osteoporosis. In light of the known activities of lovastatin and bezafibrate, the authors attribute the effect on bone loss to their ability to lower lipid levels and overcome the impairment to circulation within the femoral head. There is no suggestion in Wang et al. that lovastatin directly enhances bone formation.

An abstract entitled "Lovastatin Prevents Steroid-Induced Adipogenesis and Osteoporosis" by Cui, Q. et al. appeared in the Reports of the ASBMR 18th Annual Meeting (September 1996) J. Bone Mineral Res. (1996) 11(S1):S510. The abstract reports that lovastatin diminished triglyceride vesicles that accumulated when osteoprogenitor cells cloned from bone marrow stroma of chickens were treated in culture with dexamethasone. Lovastatin was reported to diminish the expression of certain mRNAs and to allow the cells to maintain the osteogenic phenotype after dexamethasone treatment. Further, chickens that had undergone bone loss in the femoral head as a result of dexamethasone treatment were improved by treatment with lovastatin. Again, there is no suggestion that lovastatin directly enhances bone formation in the absence of steroid treatment.

In any event, these data are contrary to reports that dexamethasone and other inducers, such as BMPs, induce osteoblastic differentiation and stimulate osteocalcin mRNA (Bellows, C. G., et al., Develop Biol (1990) 140:132-38; Rickard, D. J., et al., Develop Biol (1994) 161:218-28). In addition, Ducy, P. et al., Nature (1996) 382:448-52 have recently reported that osteocalcin deficient mice exhibit a phenotype marked by increased bone formation and bones of improved flnctional quality, without impairment of bone resorption. Ducy et al. state that their data suggest that osteocalcin antagonists may be of therapeutic use in conjunction with estrogen replacement therapy (for prevention or treatment of osteoporosis).

Other references which may suggest the use of various compounds for such treatments include the following: US 5,773,428; WO97/15561; WO98/08840; WO96/24350 and DE 4338944.

The oxazolidinones are a new class of antibacterial agents useful in treating gram positive bacterial infections. Representative of this class is the compound known as linezolid, developed by Pharmacia & Upjohn. Oxazolidinones having a thiocarbonyl functionality have been described in WO98/54161 and PCT/US98/25308. However, the use of these compounds for the treatment of osteoporosis, bone resorption or other bone diseases has not been disclosed nor suggested.

Despite the above teachings, there still exists a need in the art for compounds useful for stimulating bone formation without the drawbacks associated with presently known treatments for bone deficit conditions.

### Brief Summary of the Invention

In accordance with the present invention a novel method for treating osteoporosis, bone resorption or other bone diseases without the drawbacks associated with presently known treatments for bone deficit conditions is provided. More specifically, the inventive method comprises the administration of oxazolidinones having a thiocarbonyl functionality to the patient in need thereof.

In one embodiment, the present invention provides the use of an effective amount of a compound of formula I or pharmaceutical acceptable salts thereof for treating or preventing osteoporosis, bone resorption or any other bone disease in a vertebrate mammal 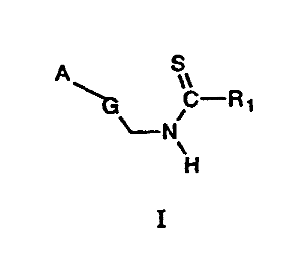
G is 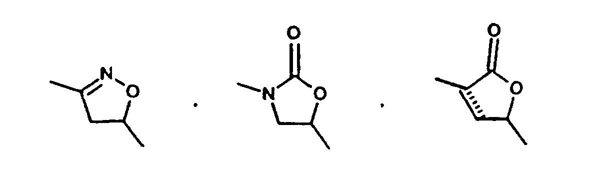
R₁ is
   a) H,
   b) NH₂,
   c) NH-C₁₋₄ alkyl,
   d) C₁₋₄ alkyl,
   e) -OC₁₋₄ alkyl,
   f) -S C₁₋₄ alkyl,
   g) C₁₋₄ alkyl substituted with 1-3 F, 1-2 Cl, CN or -COOC₁₋₄ alkyl,
   h) C₃₋₆ cycloalkyl,
   i) N(C₁₋₄ alkyl)₂ or
   j) N(CH₂)₂₋₅;
A is
   a) 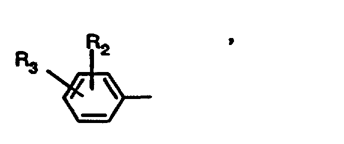
   b) 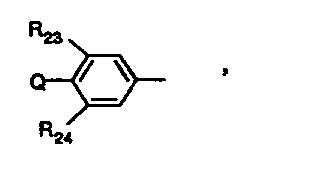
   c) 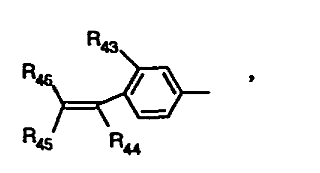
   d) a 5-membered heteroaromatic moiety having one to three atoms selected from the group consisting of S, N, and O,
      wherein the 5-membered heteroaromatic moiety is bonded via a carbon atom,
      wherein the 5-membered heteroaromatic moiety can additionally have a fused-on benzene or naphthyl ring,
      wherein the heteroaromatic moiety is optionally substituted with one to three R₄₈,
   e) a 6-membered heteroaromatic moiety having at least one nitrogen atom,
      wherein the heteroaromatic moiety is bonded via a carbon atom,
      wherein the 6-membered heteroaromatic moiety can additionally have a fused-on benzene or naphthyl ring,
      wherein the heteroaromatic moiety is optionally substituted with one to three R₆₅,
   f) a β-carbolin-3-yl, or indolizinyl bonded via the 6-membered ring, optionally substituted with one to three R₅₅,
   g) 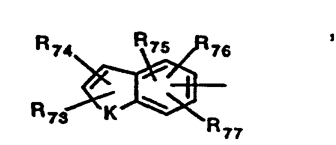 or
   h) 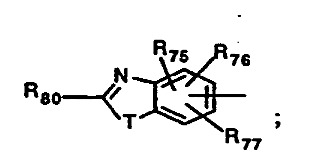
wherein R₂ is
   a) H,
   b) F,
   c) Cl,
   d) Br,
   e) C₁₋₃ alkyl,
   f) NO₂, or
   g) R₂ and R₃ taken together are -O-(CH₂)ₕ-O-;
R₃ is
   a) -S(=O)ᵢ R₄,
   b) -S(=O)₂-N=S(O)ⱼR₅R₆,
   c) -SC(=O)R₇,
   d) -C(=O)R₈,
   e) -C(=O)R₉,
   f) -C(=O)NR₁₀R₁₁,
   g) -C(=NR₁₂)R₈,
   h) -C(R₈)(R₁₁)-OR₁₃,
   i) -C(R₉)(R₁₁)-OR₁₃,
   j) -C(R₈)(R₁₁)-OC(=O)R₁₃,
   k) -C(R₉)(R₁₁)-OC(=O)R₁₃,
   l) -NR₁₀R₁₁,
   m) -N(R₁₀)-C(=O)R₇,
   n) -N(R₁₀)-S(=O)ᵢR₇,
   o) -C(OR₁₄)(OR₁₅)R₈,
   p) -C(R₈)(R₁₆)-NR₁₀R₁₁, or
   q) C₁₋₈ alkyl substituted with one or more =O other than at alpha position, -S(=O)ᵢR₁₇, -NR₁₀R₁₁, C₂₋₅ alkenyl, or C₂₋₅ alkynyl;
R₄ is
   a) C₁₋₄ alkyl optionally substituted with one or more halos, OH, CN, NR₁₀R₁₁, or -CO₂R₁₃,
   b) C₂₋₄ alkenyl,
   c) -NR₁₆R₁₈,
   d) -N₃,
   e) -NHC(=O)R₇,
   f) -NR₂₀C(=O)R₇,
   g) -N(R₁₉)₂,
   h) -NR₁₆R₁₉, or
   i) -NR₁₉R₂₀,
R₅ and R₆ at each occurrence are the same or different and are
   a) C₁₋₂ alkyl, or
   b) R₅ and R₆ taken together are -(CH₂)ₖ-;
R₇ is C₁₋₄ alkyl optionally substituted with one or more halos;
R₈ is
   a) H, or
   b) C₁₋₈ alkyl optionally substituted with one or more halos, or C₃₋₈ cycloalkyl;
R₉ is C₁₋₄ alkyl substituted with one or more
   a) -S(=O)R₁₇,
   b) -OR₁₃,
   c) -OC(=O)R₁₃,
   d) -NR₁₀R₁₁, or
   e) C₁₋₅ alkenyl optionally substituted with CHO;
R₁₀ and R₁₁ at each occurrence are the same or different and are
   a) H,
   b) C₁₋₄ alkyl, or
   c) C₃₋₈ cycloalkyl;
R₁₂ is
   a) -NR₁₀R₁₁,
   b) -OR₁₀; or
   c) -NHC(=O)R₁₀;
R₁₃ is
   a) H, or
   b) C₁₋₄ alkyl;
R₁₄ and R₁₅ at each occurrence are the same or different and are
   a) C₁₋₄ alkyl, or
   b) R₁₄ and R₁₅ taken together are -(CH)₁-;
R₁₆ is
   a) H,
   b) C₁₋₄ alkyl, or
   c) C₃₋₈ cycloalkyl;
R₁₇ is
   a) C₁₋₄ alkyl, or
   b) C₃₋₈ cycloalkyl;
R₁₈ is
   a) H,
   b) C₁₋₄ alkyl,
   c) C₂₋₄ alkenyl,
   d) C₃₋₄ cycloalkyl,
   e) -OR₁₃ or
   f) -NR₂₁R₂₂;
R₁₉ is
   a) Cl,
   b) Br, or
   c) I;
R₂₀ is a physiologically acceptable cation;
R₂₁ and R₂₂ at each occurrence are the same or different and are
   a) H,
   b) C₁₋₄ alkyl, or
   c) -NR₂₁R₂₂ taken together are -(CH₂)ₘ-;
wherein R₂₃ and R₂₄ at each occurrence are the same or different and are
   a) H,
   b) F,
   c) Cl,
   d) C₁₋₂ alkyl,
   e) CN
   f) OH,
   g) C₁₋₂ alkoxy,
   h) nitro, or
   i) amino;
Q is
   a) 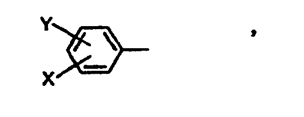
   b) 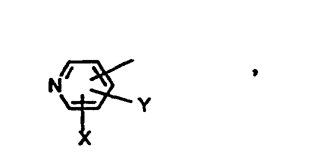
   c) 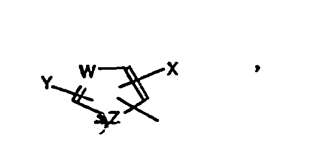
   d) 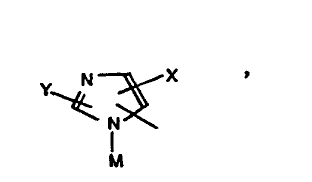
   e) 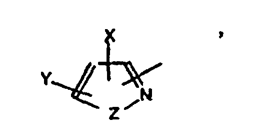
   f) 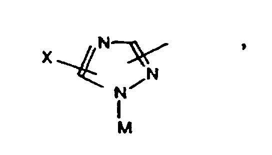
   g) 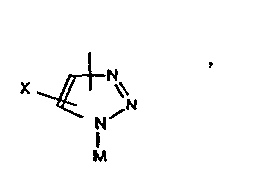
   h) 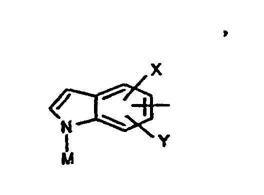
   i) 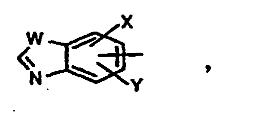
   j) 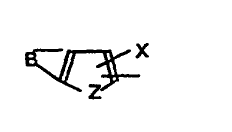
   k) 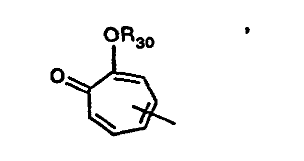
   l) 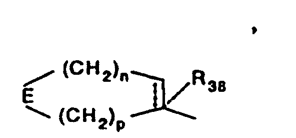
   m) a diazinyl group optionally substituted with X and Y,
   n) a triazinyl group optionally substituted with X and Y,
   o) a quinolinyl group optionally substituted with X and Y,
   p) a quinoxalinyl group optionally substituted with X and Y,
   q) a naphthyridinyl group optionally substituted with X and Y,
   r) 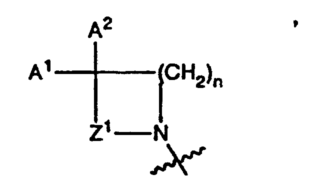
   s) 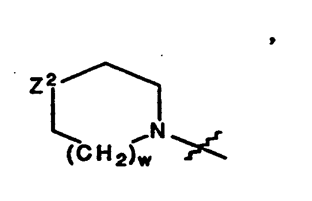
   t) 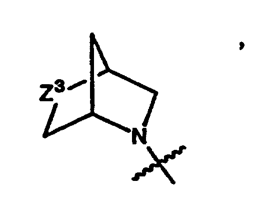
   u) 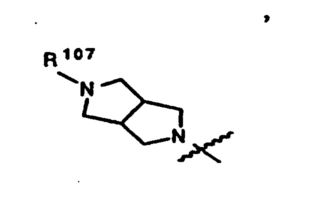
   v) 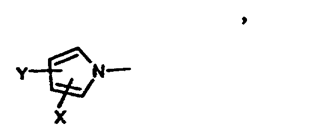
   w) 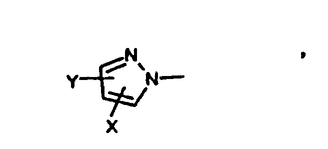
   x) 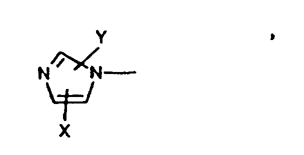
   y) 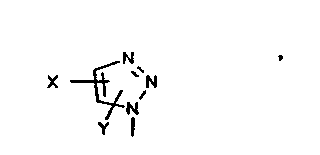
   z)

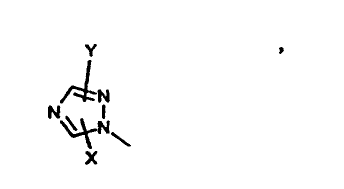

   aa) 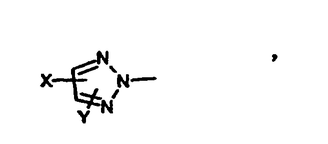
   bb) 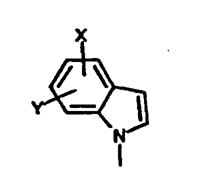 or,
Q and R₂₄ taken together are 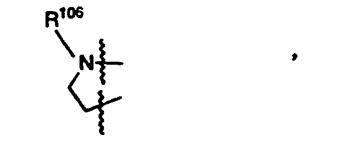
wherein Z¹ is
   a) -CH₂-,
   b) -CH(R¹⁰⁴)-CH₂-,
   c) -C(O)-, or
   d) -CH₂CH₂CH₂-;
wherein Z² is
   a) -O₂S-,
   b) -O-,
   c) -N(R¹⁰⁷)-,
   d) -OS-, or
   e) -S-;
wherein Z³ is
   a) -O₂S-,
   b) -O-,
   c) -OS-, or
   d) -S-;
wherein A¹ is
   a) H-, or
   b) CH₃;
wherein A² is
   a) H-,
   b) HO-,
   c) CH₃-,
   d) CH₃O-,
   e) R¹⁰²O-CH₂-C(O)-NH-
   f) R¹⁰³O-C(O)-NH-,
   g) (C₁-C₂)alkyl-O-C(O)-,
   h) HO-CH₂-,
   i) CH₃O-NH-,
   j) (C₁-C₃)alkyl-O₂C-
   k) CH₃-C(O)-,
   l) CH₃-C(O)-CH₂-,
   m) 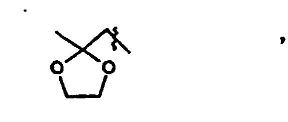 or
   n) 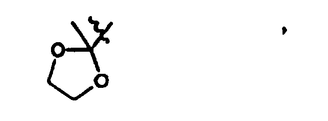
A¹ and A² taken together are:
   a) 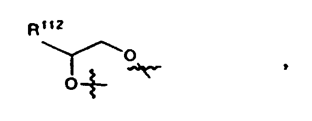
   b) o= , or
   c) 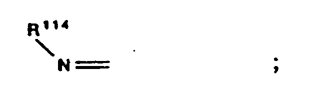
wherein R¹⁰² is
   a) H-,
   b) CH₃-,
   c) phenyl-CH₂-, or
   d) CH₃C(O)-;
wherein R¹⁰³ is
   a) (C₁-C₃)alkyl-, or
   b) phenyl-;
wherein R¹⁰⁴ is
   a) H-, or
   b) HO-;
wherein R¹⁰⁵ is
   a) H-,
   b) (C₁-C₃)alkyl-,
   c) CH₂ = CH-CH₂-, or
   d) CH₃-O-(CH₂)₂-;
wherein R¹⁰⁶ is
   a) CH₃-C(O)-,
   b) H-C(O)-,
   c) Cl₂CH-C(O)-,
   d) HOCH₂-C(O)-,
   e) CH₃SO₂-,
   f) 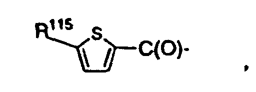
   g) F₂CHC(O)-,
   h) 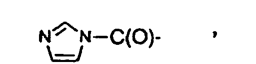
   i) H₃C-C(O)-O-CH₂-C(O)-,
   j) H-C(O)-O-CH₂-C(O)-,
   k) 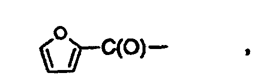
   l) HC≡C-CH₂O-CH₂-C(O)-, or
   m) phenyl-CH₂-O-CH₂-C(O)-;
wherein R¹⁰⁷ is
   a) R¹⁰²O-C(R¹¹⁰)(R¹¹¹)-C(O)-,
   b) R¹⁰³O-C(O)-,
   c) R¹⁰⁸-C(O)-,
   d) 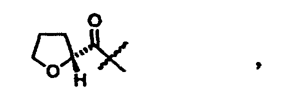
   e) 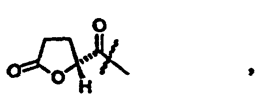
   f) H₃C-C(O)-(CH₂)₂-C(O)-,
   g) R¹⁰⁹-SO₂-,
   h) 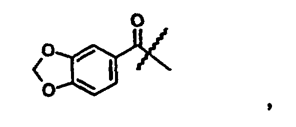
   i) HO-CH₂-C(O)-,
   j) R¹¹⁶-(CH₂)₂-,
   k) R¹¹³-C(O)-O-CH₂-C(O)-,
   l) (CH₃)₂N-CH₂-C(O)-NH-,
   m) NC-CH₂-,
   n) F₂-CH-CH₂-, or
   o) R¹⁵⁰R¹⁵¹NSO₂
wherein R¹⁰⁸ is
   a) H-,
   b) (C₁-C₄)alkyl,
   c) aryl -(CH₂)ₚ,
   d) ClH₂C-,
   e) Cl₂HC-,
   f) FH₂C-,
   g) F₂HC-,
   h) (C₃-C₆)cycloalkyl, or
   i) CNCH₂-.
wherein R¹⁰⁹ is
   a) alkylC₁-C₄,
   b) -CH₂Cl
   c) -CH₂CH=CH₂,
   d) aryl, or
   e) -CH₂CN;
wherein R¹¹⁰ and R¹¹¹ are independently
   a) H-,
   b) CH₃-; or
wherein R¹¹² is
   a) H-,
   b) CH₃O-CH₂O-CH₂-, or
   c) HOCH₂-;
wherein R¹¹³ is
   a) CH₃-,
   b) HOCH₂-,
   c) (CH₃)₂N-phenyl, or
   d) (CH₃)₂N-CH₂-;
wherein R¹¹⁴ is
   a) HO-,
   b) CH₃O-,
   c) H₂N-,
   d) CH₃O-C(O)-O-,
   e) CH₃-C(O)-O-CH₂-C(O)-O-,
   f) phenyl-CH₂-O-CH₂-C(O)-O-,
   g) HO-(CH₂)₂-O-,
   h) CH₃O-CH₂-O-(CH₂)₂-O-, or
   i) CH₃O-CH₂-O-;wherein R¹¹³ is
   a) CH₃-,
   b) HOCH₂-,
   c) (CH₃)₂N-phenyl, or
   d) (CH₃)₂N-CH₂-;
wherein R¹¹⁶ is
   a) H-, or
   b) Cl-;
wherein R¹¹⁶ is
   a) HO-
   b) CH₃O-, or
   c) F;
wherein R¹⁵⁰ and R¹⁵¹ are each H or alkyl C₁-C₄ or R¹⁵⁰ and R¹⁵¹ taken together with the nitrogen atom to which each is attached form a monocyclic heterocyclic ring having from 3 to 6 carbon atoms;
B is an unsaturated 4-atom linker having one nitrogen and three carbons;
M is
   a) H,
   b) C₁₋₈ alkyl,
   c) C₃₋₈ cycloalkyl,
   d) -(CH₂)ₘOR₁₃, or
   e) -(CH₂)ₕ-NR₂₁R₂₂;
Z is
   a) O,
   b) S, or
   c) NM;
W is
   a) CH,
   b) N, or
   c) S or O when Z is NM;
Y is
   a) H,
   b) F,
   c) Cl,
   d) Br,
   e) C₁₋₃ alkyl, or
   f) NO₂;
X is
   a) H,
   b) -CN,
   c) OR₂₇,
   d) halo,
   e) NO₂,
   f) tetrazoyl,
   g) -SH,
   h) -S(=O)ᵢR₄,
   i) -S(=O)₂-N=S(O)ⱼR₅R₆,
   j) -SC(=O)R₇,
   k) -C(=O)R₂₅,
   l) -C(=O)NR₂₇R₂₈,
   m) -C(=NR₂₉)R₂₅,
   n) -C(R₂₅)(R₂₈)-OR₁₃,
   o) -C(R₂₅)(R₂₈)-OC(=O)R₁₃,
   p) -C(R₂₈)(OR₁₃)-(CH₂)ₕ-NR₂₇R₂₈,
   q) -NR₂₇R₂₈,
   r) -N(R₂₇)C(=O)R₇,
   s) -N(R₂₇)-S(=O)ᵢR₇,
   t) -C(OR₁₄)(OR₁₅)R₂₈,
   u) -C(R₂₅)(R₁₆)-NR₂₇R₂₆, or
   v) C₁₋₈ alkyl substituted with one or more halos, OH, =O other than at alpha position, -S(=O)ᵢR₁₇, -NR₂₇R₂₈, C₂₋₅ alkenyl, C₂₋₅ alkynyl, or C₃₋₈ cycloalkyl;
R₄, R₅, R₆, R₇, R₁₃, R₁₄, R₁₅, R₁₆, and R₁₇ are the same as defined above;
R₂₅ is
   a) H,
   b) C₁₋₈ alkyl optionally substituted with one or more halos, C₃₋8 cycloalkyl, C₁₋₄ alkyl substituted with one or more of -S(=O)ᵢR₁₇, -OR₁₃, or OC(=O)R₁₃, NR₂₇R₂₈, or
   c) C₂₋₅ alkenyl optionally substituted with CHO, or CO₂R₁₃;
R₂₆ is
   a) R₂₈, or
   b) NR₂₇N₂₈;
R₂₇ and R₂₈ at each occurrence are the same or different and are
   a) H,
   b) C₁₋₈ alkyl,
   c) C₃₋₈ cycloalkyl,
   d) -(CH₂)ₘOR₁₃,
   e) -(CH₂)ₕ-NR₂₁R₂₂, or
   f) R₂₇ and R₂₈ taken together are -(CH₂)₂O(CH₂)₂-, -(CH₂)ₕCH(COR₇)-, or -(CH₂)₂N(CH₂)₂(R₇);
R₂₉ is
   a) -NR₂₇R₂₈,
   b) -OR₂₇, or
   c) -NHC(=O)R₂₈;
wherein R₃₀ is
   a) H,
   b) C₁₋₈ alkyl optionally substituted with one or more halos, or
   c) C₁₋₈ alkyl optionally substituted with one or more OR, or C₁₋₆ alkoxy;
wherein E is
   a) NR₃₉,
   b) -S(=O)ᵢ, or
   c) O;
R₃₈ is
   a) H,
   b) C₁₋₆ alkyl,
   c) -(CH₂)_{q}-aryl, or
   d) halo;
R₃₉ is
   a) H,
   b) C₁₋₆ alkyl optionally substituted with one or more OH, halo, or -CN,
   c) -(CH₂)_{q}-aryl,
   d) -CO₂R₄₀,
   e) -COR₄₁,
   f) -C(=O)-(CH₂)_{q}-C(=O)R₄₀,
   g) -S(=O)₂-C₁₋₆ alkyl,
   h) -S(=O)₂-(CH₂)_{q}-aryl, or
   i) -(C=O)ⱼ-Het;
R₄₀ is
   a) H,
   b) C₁₋₆ alkyl optionally substituted with one or more OH, halo, or -CN,
   c) -(CH₂)_{q}-aryl, or
   d) -(CH₂)_{q}-OR₄₂;
R₄₁ is
   a) C₁₋₆ alkyl optionally substituted with one or more OH, halo, or -CN,
   b) -(CH₂)_{q}-aryl, or
   c) -(CH₂)_{q}-OR₄₂;
R₄₂ is
   a) H,
   b) C₁₋₆ alkyl,
   c) -(CH₂)_{q}-aryl, or
   d) -C(=O)-C₁₋₆ alkyl;
aryl is
   a) phenyl,
   b) pyridyl, or
   c) napthyl; a to c optionally substituted with one or more halo, -CN, OH, SH, C₁₋₆ alkyl, C₁₋₆ alkoxy, or C₁₋₆ alkylthio;
wherein R₄₃ is
   a) H,
   b) C₁₋₂ alkyl,
   c) F, or
   d) OH;
R₄₄ is
   a) H,
   b) CF₃,
   c) C₁₋₃ alkyl optionally substituted with one or more halo,
   d) phenyl optionally substituted with one or more halo,
   e) R₄₄ and R₄₅ taken together are a 5-, 6-, or 7-membered ring of the formula, 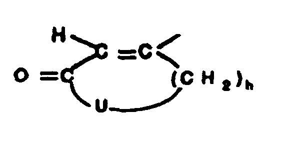 or
   f) R₄₄ and R₄₅ taken together are -(CH₂)ₖ-, when R₄₆ is an electron-withdrawing group;
R₄₅ and R₄₆ at each occurrence are the same or different and are
   a) an electron-withdrawing group,
   b) H,
   c) CF₃,
   d) C₁₋₃ alkyl optionally substituted with one halo,
   e) phenyl, provided at least one of R₄₅ or R₄₆ is an electron-withdrawing group, or
   f) R₄₅ and R₄₆ taken together are a 5-, 6-, 7-membered ring of the formula 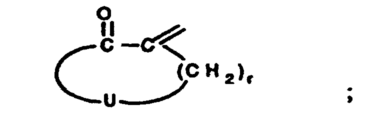
U is
   a) CH₂,
   b) O,
   c) S, or
   d) NR₄₇;
R₄₇ is
   a) H, or
   b) C₁₋₅ alkyl;
wherein R₄₈ is
   a) carboxyl,
   b) halo,
   c) -CN,
   d) mercapto,
   e) formyl,
   f) CF₃,
   g) -NO₂,
   h) C₁₋₆ alkoxy,
   i) C₁₋₆ alkoxycarbonyl,
   j) C₁₋₆ alkythio,
   k) C₁₋₆ acyl,
   l) -NR₄₉ R₅₀,
   m) C₁₋₆ alkyl optionally substituted with OH, C₁₋₅ alkoxy, C₁₋₅ acyl, or -NR₄₉R₅₀,
   n) C₂₋₈ alkenylphenyl optionally substituted with one or two R₅₁,
   o) phenyl optionally substituted with one or two R₅₁,
   p) a 5-, or 6-membered (un)saturated heterocyclic moiety having one to three atoms selected from the group consisting of S, N, and O, optionally substituted with one or two R₅₁, or
   q) 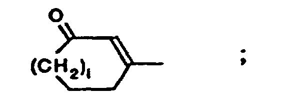
R₄₉ and R₅₀ at each occurrence are the same or different and are
   a) H,
   b) C₁₋₄ alkyl,
   c) C₅₋₆ cycloalkyl, or
   d) R₄₉ and R₅₀ taken together with the nitrogen atom is a 5-, 6-membered saturated heterocyclic moiety which optionally has a further hetero atom selected from the group consisting of S, N, and O, and can in turn be optionally substituted with, including on the further nitrogen atom, C₁₋₃ alkyl, or C₁₋₃ acyl;
R₅₁ is
   a) carboxyl,
   b) halo,
   c) -CN,
   d) mercapto,
   e) formyl,
   f) CF₃,
   g) -NO₂,
   h) C₁₋₆ alkoxy,
   i) C₁₋₆ alkoxycarbonyl,
   j) C₁₋₆ alkythio,
   k) C₁₋₆ acyl,
   l) C₁₋₆ alkyl optionally substituted with OH, C₁₋₆ alkoxy, C₁₋₅ acyl, or -NR₄₉R₅₀,
   m) phenyl,
   n) -C(=O)NR₅₂ R₅₃,
   o) -NR₄₉R₅₀,
   p) -N(R₅₂)(-SO₂R₅₄),
   q) -SO₂-NR₅₂R₅₃, or
   r) -S(=O)ᵢR₅₄;
R₅₂ and R₅₃ at each occurrence are the same or different and are
   a) H,
   b) C₁₋₆ alkyl, or
   c) phenyl;
R₅₄ is
   a) C₁₋₄ alkyl, or
   b) phenyl optionally substituted with C₁₋₄ alkyl;
wherein R₅₅ is
   a) carboxyl,
   b) halo,
   c) -CN,
   d) mercapto,
   e) formyl,
   f) CF₃,
   g) -NO₂,
   h) C₁₋₆ alkoxy,
   i) C₁₋₆ alkoxycarbonyl,
   j) C₁₋₆ alkythio
   k) C₁₋₆ acyl,
   l) -NR₅₆ R₅₇,
   m) C₁₋₆ alkyl optionally substituted with OH, C₁₋₅ alkoxy, C₁₋₅ acyl, or -NR₅₆R₅₇,
   n) C₂₋₈ alkenylphenyl optionally substituted with one or two R₅₈,
   o) phenyl optionally substituted with one or two R₅₈,
   p) a 5- or 6-membered (un)saturated heterocyclic moiety having one to three atoms selected from the group consisting of S, N, and O, optionally substituted with one or two R₅₈, or
   q) 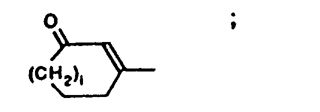
R₅₆ and R₅₇ at each occurrence are the same or different and are
   a) H,
   b) formyl,
   c) C₁₋₄ alkyl,
   d) C₁₋₄ acyl,
   e) phenyl,
   f) C₃₋₆ cycloalkyl, or
   g) R₅₆ and R₅₇ taken together with the nitrogen atom is a 5-, 6-membered saturated heterocyclic moiety which optionally has a further hetero atom selected from the group consisting of S, N, and O, and can in turn be optionally substituted with, including on the further nitrogen atom, phenyl, pyrimidyl, C₁₋₃ alkyl, or C₁₋₃ acyl;
R₅₈ is
   a) carboxyl,
   b) halo,
   c) -CN,
   d) mercapto,
   e) formyl,
   f) CF₃,
   g) -NO₂,
   h) C₁₋₆ alkoxy,
   i) C₁₋₆ alkoxycarbonyl,
   j) C₁₋₆ alkythio,
   k) C₁₋₆ acyl,
   l) phenyl,
   m) C₁₋₆ alkyl optionally substituted with OH, azido, C₁₋₅ alkoxy, C₁₋₅ acyl, -NR₆₅R₆₆, -SR₆₇, -O-SO₂R₆₈, or 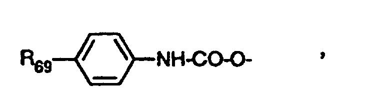
   n) -C(=O)NR₅₉ R₆₀,
   o) -NR₅₆R₆₇,
   p) -N(R₅₉)(-SO₂R₅₄),
   q) -SO₂-NR₅₉R₆₀,
   r) -S(=O)ᵢR₅₄,
   s) -CH=N-R₆₁, or
   t) -CH(OH)-SO₃R₆₄;
R₅₄ is the same as defined above;
R₅₉ and R₆₀ at each occurrence are the same or different and are
   a) H,
   b) C₁₋₆ alkyl,
   c) phenyl, or
   d) tolyl;
R₆₁ is
   a) OH,
   b) benzyloxy,
   c) -NH-C(=O)-NH₂,
   d) -NH-C(=S)-NH₂, or
   e) -NH-C(=NH)-NR₆₂R₆₃;
R₆₂ and R₆₃ at each occurrence are the same or different and are
   a) H, or
   b) C₁₋₄ alkyl optionally substituted with phenyl or pyridyl;
R₆₄ is
   a) H, or
   b) a sodium ion;
R₆₅ and R₆₆ at each occurrence are the same or different and are
   a) H,
   b) formyl,
   c) C₁₋₄ alkyl,
   d) C₁₋₄ acyl,
   e) phenyl,
   f) C₃₋₆ cycloalkyl,
   g) R₆₅ and R₆₆ taken together are a 5-, 6-membered saturated heterocyclic moiety having one to three atoms selected from the group consisting of S, N, and O, optionally substituted with, including on the nitrogen atom, phenyl, pyrimidyl, C₁₋₃ alkyl, or C₁₋₃ acyl,
   h) -P(O)(OR₇₀)(OR₇₁), or
   i) -SO₂-R₇₂;
R₆₇ is 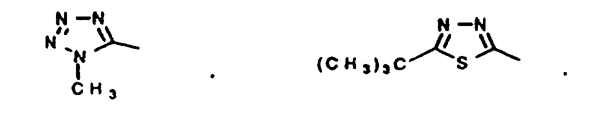 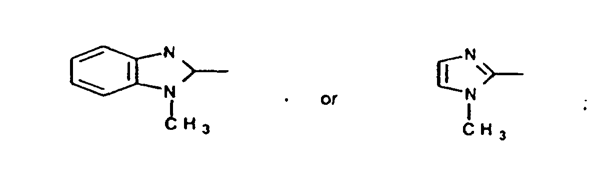
R₆₈ is C₁₋₃ alkyl;
R₆₉ is
   a) C₁₋₆ alkoxycarbonyl, or
   b) carboxyl;
R₇₀ and R₇₁ at each occurrence are the same or different and are
   a) H, or
   b) C₁₋₃ alkyl;
R₇₂ is
   a) methyl,
   b) phenyl, or
   c) tolyl;
wherein K is
   a) O, or
   b) S;
R₇₃, R₇₄, R₇₅, R₇₆, and R₇₇ at each occurrence are the same or different and are
   a) H,
   b) carboxyl,
   c) halo,
   d) -CN,
   e) mercapto,
   f) formyl,
   g) CF₃,
   h) -NO₂,
   i) C₁₋₆ alkoxy,
   j) C₁₋₆ alkoxycarbonyl,
   k) C₁₋₆ alkythio,
   l) C₁₋₆ acyl,
   m) -NR₇₈ R₇₉,
   n) C₁₋₆ alkyl optionally substituted with OH, C₁₋₅ alkoxy, C₁₋₅ acyl, - NR₇₈R₇₉, -N(phenyl)(CH₂-CH₂-OH), -O-CH(CH₃)(OCH₂CH₃), or - O-phenyl-[para-NHC(=O)CH₃],
   o) C₂₋₈ alkenylphenyl optionally substituted with R₅₁,
   p) phenyl optionally substituted with R₅₁, or
   q) a 5-, or 6-membered (un)saturated heterocyclic moiety having one to three atoms selected from the group consisting of S, N, and O, optionally substituted with R₅₁;
R₅₁ is the same as defined above;
R₇₈ and R₇₉ at each occurrence are the same or different and are
   a) H,
   b) C₁₋₄ alkyl,
   c) phenyl, or
   d) R₇₈ and R₇₉ taken together with the nitrogen atom is a 5-, 6-membered saturated heterocyclic moiety which optionally has a further hetero atom selected from the group consisting of S, N, and O, and can in turn be optionally substituted with, including on the further nitrogen atom, C₁₋₃ alkyl, or C₁₋₃ acyl;
wherein T is
   a) O,
   b) S, or
   c) SO₂;
R₇₅, R₇₆, and R₇₇ are the same as defined above;
R₈₀ is
   a) H,
   b) formyl,
   c) carboxyl,
   d) C₁₋₆ alkoxycarbonyl,
   e) C₁₋₈ alkyl,
   f) C₂₋₈ alkenyl,
      wherein the substituents (e) and (f) can be optionally substituted with OH, halo, C₁₋₆ alkoxy, C₁₋₆ acyl, C₁₋₆ alkylthio or C₁₋₆ alkoxycarbonyl, or phenyl optionally substituted with halo,
   g) an aromatic moiety having 6 to 10 carbon atoms optionally substituted with carboxyl, halo, -CN, formyl, CF₃, -NO₂, C₁₋₆ alkyl, C₁₋₆ alkoxy, C₁₋₆ acyl, C₁₋₆ alkylthio, or C₁₋₆ alkoxycarbonyl;
   h) -NR₈₁R₈₂,
   i) -OR₉₀,
   j) -S(=O)ᵢ-R₉₁, or
   k) -SO₂-N(R₉₂)(R₉₃);
R₈₁ and R₈₂ at each occurrence are the same or different and are
   a) H,
   b) C₃₋₆ cycloalkyl,
   c) phenyl,
   d) C₁₋₆ acyl,
   e) C₁₋₈ alkyl optionally substituted with OH, C₁₋₆ alkoxy which can be substituted with OH, a 5-, or 6-membered aromatic heterocyclic moiety having one to three atoms selected from the group consisting of S, N, and O, phenyl optionally substituted with OH, CF₃, halo, -NO₂, C₁₋₄ alkoxy, -NR₈₃R₈₄, or 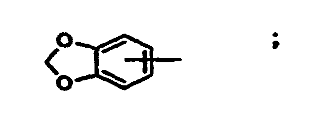
   f) 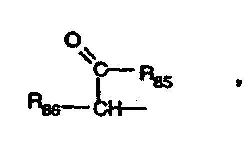 or
   g) 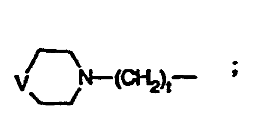
V is
   a) O,
   b) CH₂, or
   c) NR₈₇;
R₈₃ and R₈₄ at each occurrence are the same or different and are
   a) H, or
   b) C₁₋₄ alkyl;
R₈₅ is
   a) OH,
   b) C₁₋₄ alkoxy, or
   c) -NR₈₈ R₈₉;
R₈₆ is
   a) H, or
   b) C₁₋₇ alkyl optionally substituted with indolyl, OH, mercaptyl, imidazoly, methylthio, amino, phenyl optionally substituted with OH, -C(=O)-NH₂, -CO₂H, or -C(=NH)-NH₂;
R₈₇ is
   a) H,
   b) phenyl, or
   c) C₁₋₆ alkyl optionally substituted by OH;
R₈₈ and R₈₉ at each occurrence are the same or different and are
   a) H,
   b) C₁₋₅ alkyl
   c) C₃₋₆ cycloalky, or
   d) phenyl;
R₉₀ is
   a) C₁₋₈ alkyl optionally substituted with C₁₋₆ alkoxy or hydroxy, C₃₋₆ cycloalkyl, a 6-membered aromatic optionally benzo-fused heterocyclic moiety having one to three nitrogen atoms, which can in turn be substituted with one or two -NO₂, CF₃, halo, -CN, OH, C₁₋₅ alkyl, C₁₋₅ alkoxy, or C₁₋₅ acyl;
   b) 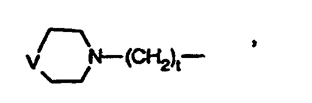
   c) phenyl, or
   d) pyridyl;
R₉₁ is
   a) C₁₋₁₆ alkyl,
   b) C₂₋₁₆ alkenyl,
      wherein the substituents (a) and (b) can be optionally substituted with C₁₋₆ alkoxycarbonyl, or a 5-, 6-, 7-membered aromatic heterocyclic moiety having one to three atoms selected from the group consisting of S, N, and O,
   c) an aromatic moiety having 6 to 10 carbon atoms, or
   d) a 5-, 6-, 7-membered aromatic heterocyclic moiety having one to three atoms selected from the group consisting of S, N, and O,
   wherein the substituents (c) and (d) can be optionally substituted with carboxyl, halo, -CN, formyl, CF₃, -NO₂, C₁₋₆ alkyl, C₁₋₆ alkoxy, C₁₋₆ acyl, C₁₋₆ alkylthio, or C₁₋₆ alkoxycarbonyl;
R₉₂ and R₉₃ at each occurrence are the same or different and are
   a) H,
   b) phenyl,
   c) C₁₋₆ alkyl, or
   d) benzyl;
R₉₄ and R₉₅ at each occurrence are the same or different and are
   a) H,
   b) OH,
   c) C₁₋₆ alkyl optionally substituted with -NR₈₃ R₈₄, or
   d) R₉₄ and R₉₅ taken together are =O;
R₉₆ is
   a) an aromatic moiety having 6 to 10 carbon atoms,
   b) a 5-, or 6-membered aromatic optionally benzo-fused heterocyclic moiety having one to three atoms selected from the group consisting of S, N, and O,
      wherein the substituents (a) and (b) which can in turn be substituted with one or three -NO₂, CF₃, halo, -CN, OH, phenyl, C₁₋₅ alkyl, C₁₋₅ alkoxy, or C₁₋₅ acyl,
   c) morpholinyl,
   d) OH,
   e) C₁₋₆ alkoxy,
   f) -NR₈₃R₈₄,
   g) -C(=O)-R₉₇, or
   h) 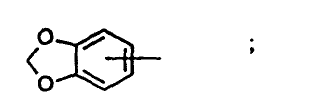
R₉₇ is
   a) morpholinyl,
   b) OH, or
   c) C₁₋₆ alkoxy;
h is 1, 2, or 3;
i is 0, 1, or 2;
j is 0 or 1;
k is 3, 4, or 5;
l is 2 or 3;
m is 4 or 5;
n is 0, 1, 2, 3, 4, or 5;
p is 0, 1, 2, 3, 4, or 5; with the proviso that n and p together are 1, 2, 3, 4, or 5;
q is 1, 2, 3, or 4;
r is 2, 3, or 4;
t is 0, 1, 2, 3, 4, 5, or 6;
u is 1 or 2;
w is 0, 1, 2, or 3.

An object of the present invention is to provide a novel use of a compound of formula I for treating or preventing osteoporosis, bone resorption and other bone diseases.

Still another object of the present invention is to prepare a medicament for treating or preventing osteoporosis, bone resorption and other bone diseases in a mammal

These, and other objects, will readily be apparent to those skilled in the art as reference is made to the detailed description of the preferred embodiment.

### Detailed Description of the Preferred Embodiment

The present invention is directed to the use of the compounds of formula I above to treat osteoporosis, bone resorption or other bone diseases. The compounds, including their synthesis, are described in greater detail in WO98/54161 and PCT/US98/25308. To the extent necessary for completion, the disclosure of these documents is expressly incorporated by reference.

The compounds used in the invention can be prepared using known compounds and intermediates of oxazolidinones, isoxazolines and butyolactones as intermediates and synthetic methods known in the art. Thioamides of the invention can typically be prepared by the reaction of the corresponding amide with Lawesson's reagent.

Compounds disclosed in the following publications are suitable intermediates for preparation of the compounds used in this invention and are hereby incorporated by reference for their disclosure of suitable compounds that can be converted to the subject thiocarbonyl derivatives. U.S. Patents 5,225,565; 5,182,403; 5,164,510; 5,247,090; 5,231,188; 5,565,571; 5,547,950; and 5,523,403. PCT Application and publications PCT/US93/04850, WO94/01110; PCT/US94/08904, WO95/07271; PCT/US95/02972, WO95/25106; PCT/US95/10992, WO96/13502; PCT/US96/05202, WO96/35691; PCT/US96/12766; PCT/US96/13726; PCT/US96/14135; PCT/US96/17120; PCT/US96/19149; PCT/US97/01970; PCT/US95/12751, WO96/15130; and PCT/US96/00718, WO96/23788.

Chemical conversion techniques for converting various intermediates having a CH₂NH₂ on the oxazolidinone ring to CH₂NH-C(S)-CH₃ is disclosed by Hartke, K., Barrmeyer, S., J. prakt. Chem. 1996, 338, 251-6. Similarly, conversion of CH₂NHC(=0)CH₃ to CH₂NHC(S)NHCH₃ is reported by Cava, M.P.; Levinson, M.I., Thionation Reactions of Lawesson's Reagents, Tetrahedron 1985, 41, 5061-87.

For the purpose of the present invention, the carbon content of various hydrocarbon containing moieties is indicated by a prefix designating the minimum and maximum number of carbon atoms in the moiety, i.e., the prefix Cᵢ₋ⱼ defines the number of carbon atoms present from the integer "i" to the integer "j", inclusive. Thus, C₁₋₄ alkyl refers to alkyl of 1-4 carbon atoms, inclusive, or methyl, ethyl, propyl, butyl and isomeric forms thereof.

The terms "C₁₋₂ alkyl", "C₁₋₃ alkyl", "C₁₋₄ alkyl", "C₁₋₅ alkyl", "C₁₋₆ alkyl", "C₁₋₈ alkyl", and "C₁₋₁₆ alkyl" refer to an alkyl group having one to two, one to three, one to four, one to five, one to six, one to eight, or one to sixteen carbon atoms respectively such as, for example, methyl, ethyl, propyl, butyl, pentyl, hexyl. heptyl, octyl, nonyl, decyl, undecyl, dodecyl, tridecyl, tetradecyl and their isomeric forms thereof.

The terms "C₂₋₄ alkenyl", "C₂₋₅ alkenyl", "C₂₋₈ alkenyl", "C₂₋₁₄ alkenyl" and "C₂₋₁₆ alkenyl" refer to at least one double bond alkenyl group having two to four, two to five, two to eight, two to fourteen, or two to sixteen carbon atoms, respectively such as, for example, ethenyl, propenyl, butenyl, pentenyl, pentdienyl, hexenyl, hexdienyl, heptenyl, heptdienyl, octenyl, octdienyl, octatrienyl, nonenyl, nonedienyl, nonatrienyl, undecenyl, undecdienyl, dodecenyl, tridecenyl, tetradecenyl and their isomeric forms thereof.

The terms "C₂₋₅ alkynyl", "C₂₋₈ alkynyl", and "C₂₋₁₀ alkynyl" refer to at least one triple bond alkynyl group having two to five, two to eight, or two to ten carbon atoms respectively such as, for example, ethynyl, propynyl, butynyl, pentynyl, pentdiynyl, hexynyl, hexdiynyl, heptynyl, heptdiynyl, octynyl, octdiynyl, octatriynyl, nonynyl, nonediynyl, nonatriynyl and their isomeric forms thereof.

The terms "C₃₋₄ cycloalkyl", "C₃₋₆ cycloalkyl", "C₅₋₆ cycloalkyl", and "C₃₋₈ cycloalkyl" refer to a cycloalkyl having three to four, three to six, five to six, or three to eight carbon atoms respectively such as, for example, cyclopropyl, cyclobutyl, cyclopentyl, cyclohexyl, cycloheptyl, cyclooctyl, and their isomeric forms thereof.

The terms "C₁₋₄ alkoxy", "C₁₋₆ alkoxy", and "C₁₋₈ alkoxy" refer to an alkyl group having one to four, one to six, or one to eight carbon atoms respectively attached to an oxygen atom such as, for example, methoxy, ethoxy, propyloxy, butyloxy, pentyloxy, hexyloxy, heptyloxy, or octyloxy and their isomeric forms thereof.

The terms "C₁₋₆ alkylamino", and "C₁₋₈ alkylamino" refer to an alkyl group having one to six, or one to eight carbon atoms respectively attached to an amino moiety such as, for example, methylamino, ethylamino, propylamino, butylamino, pentylamino, hexylamino, heptylamino, or octoylamino and their isomeric forms thereof.

The terms "C₁₋₆ dialkylamino", and "C₁₋₈ dialkylamino" refer to two alkyl groups having one to six, or one to eight carbon atoms respectively attached to an amino moiety such as, for example, dimethylamino, methylethylamino, diethylamino, dipropylamino, methypropylamino, ethylpropylamino, dibutylamino, dipentylamino, dihexylamino, methylhecylamino, diheptylamino, or dioctoylamino and their isomeric forms thereof.

The terms "C₁₋₃ acyl", "C₁₋₄ acyl", "C₁₋₅ acyl", "C₁₋₆ acyl", "C₁₋₈ acyl", and "C₂₋₈ acyl" refer to a carbonyl group having an alkyl group of one to three, one to four, one to five, one to six, one to eight, or two to eight carbon atoms.

The terms "C₁₋₄ allcoxycarbonyl", "C₁₋₆ alkoxycarbonyl", and "C₁₋₈ alkoxycarbonyl" refer to an ester group having an alkyl group of one to four, one to six, or one to eight carbon atoms.

The term "C₁₋₈ alkyl phenyl" refers to an alkyl group having one to eight carbon atoms and isomeric forms thereof which is substituted with at least one phenyl radical.

The term "C₂₋₈ alkenyl phenyl" refers to a at least one double bond alkenyl group having one to eight carbon atoms and isomeric forms thereof which is substituted with at least one phenyl radical.

The term "C₁₋₈ alkyl pyridyl" refers to an alkyl group having one to eight carbon atoms and isomeric forms thereof which is substituted with at least one pyridyl radical.

The term "C₁₋₈ hydroxyl" refers to an alkyl group having one to eight carbon atoms and isomeric forms thereof attached to a hydroxy group.

The term "C₁₋₈ alkylsulfonyl" refers to an alkyl group having one to eight carbon atoms and isomeric forms thereof attached to a SO₂ moiety.

The term "C₁₋₆ alkylthio" refers to an alkyl group having one to six carbon atoms and isomeric forms thereof attached to a sulfur atom.

The term "Het" refers to 5 to 10 membered saturated, unsaturated or aromatic heterocyclic rings containing one or more oxygen, nitrogen, and sulfur forming such groups as, for example, pyridine, thiophene, furan, pyrazoline, pyrimidine, 2-pyridyl, 3-pyridyl, 4-pyridyl, 2-pyrimidinyl, 4-pyrimidinyl, 5-pyrimidinyl, 3-pyridazinyl, 4-pyridazinyl, 3-pyrazinyl, 2-quinolyl, 3-quinolyl, 1-isoquinolyl, 3-isoquinolyl, 4-isoquinolyl, 2-quinazolinyl, 4-quinazolinyl, 2-quinoxalinyl, 1-phthalazinyl, 4-oxo-2-imidazolyl, 2-imidazolyl, 4-imidazolyl, 3-isoxazolyl, 4-isoxazolyl, 5-isoxazolyl, 3-pyrazolyl, 4-pyrazolyl, 5-pyrazolyl, 2-oxazolyl, 4-oxazolyl, 4-oxo-2-oxazolyl, 5-oxazolyl, 4,5,-dihydrooxazole, 1,2,3-oxathiole, 1,2,3-oxadiazole, 1,2,4-oxadiazole, 1,2,5-oxadiazole, 1,3,4-oxadiazole, 2-thiazolyl, 4-thiazolyl, 5-thiazolyl, 3-isothiazole, 4-isothiazole, 5-isothiazole, 2-indolyl, 3-indolyl, 3-indazolyl, 2-benzoxazolyl, 2-benzothiazolyl, 2-benzimidazolyl, 2-benzofuranyl, 3-benzofuranyl, benzoisothiazole, benzisoxazole, 2-furanyl, 3-furanyl, 2-thienyl, 3-thienyl, 2-pyrrolyl, 3-pyrrolyl, 3-isopyrrolyl, 4-isopyrrolyl, 5-isopyrrolyl, 1,2,3,-oxathiazole-1-oxide, 1,2,4-oxadiazol-3-yl, 1,2,4-oxadiazol-5-yl, 5-oxo-1,2,4-oxadiazol-3-yl, 1,2,4-thiadiazol-3-yl, 1,2,4-thiadiazol-5-yl, 3-oxo-1,2,4-thiadiazol-5-yl, 1,3,4-thiadiazol-5-yl, 2-oxo-1,3,4-thiadiazol-5-yl, 1,2,4-triazol-3-yl, 1,2,4-triazol-5-yl, 1,2,3,4-tetrazol-5-yl, 5-oxazolyl, 1-pyrrolyl, 1-pyrazolyl, 1,2,3-triazol-1-yl, 1,2,4-triazol-1-yl, 1-tetrazolyl, 1-indolyl, 1-indazolyl, 2-isoindolyl, 7-oxo-2-isoindolyl,1-purinyl, 3-isothiazolyl, 4-isothiazolyl and 5-isothiazolyl, 1,3,4,-oxadiazole, 4-oxo-2-thiazolinyl, or 5-methyl-1,3,4-thiadiazol-2-yl, thiazoledione, 1,2,3,4-thiatriazole, 1,2,4-dithiazolone. Each of these moieties may be substituted as appropriate.

The term halo refers to fluoro, chloro, bromo, or iodo.

When Q is the structure of 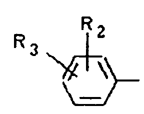 the dotted line in the heterocyclic ring means that this bond can be either single or double. In the case where the dotted line is a double bond, the R₃₉ group will not be present.

The compounds of Formula I of this invention contain a chiral center at C5 of the isoxazoline ring, and as such there exist two enantiomers or a racemic mixture of both. This invention relates to both the enantiomers, as well as mixtures containing both the isomers. In addition, depending on substituents, additional chiral centers and other isomeric forms may be present in any of A or R₁ group, and this invention embraces all possible stereoisomers and geometric forms in these groups.

In even more preferred embodiments, the compounds selected for use are of formula (II): 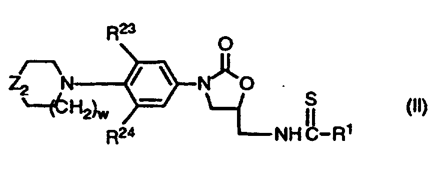 wherein Z₂ is -O₂S-, -O-, -N(R¹⁰⁷)-, -OS-, or -S-;
w is 0, 1, 2, or 3;
R²³ and R²⁴ are the same or different and can be H or F; and
R¹ is H, NH₂, NHalkylC₁-C₄; N(alkylC₁-C₄)₂; 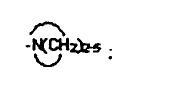 alkylC₁-C₄; OalkylC₁-C₄; SalkylC₁-C₄; alkylC₁-C₄ substituted with 1-3F, 1-2Cl, CN, or -COOalkylC₁-C₄, or cycloalkylC₃-C₆, wherein in each occurrence of the alkyl group may be straight or branched; and
R¹⁰⁷ is
a) R¹⁰²O-C(R¹¹⁰)(R¹¹¹)-C(O)-,
b) R¹⁰³O-C(O)-,
c) R¹⁰⁸-C(O)-,
d) R¹⁰⁹-SO₂-,
e) NC-CH₂-,
f) FCHCH₂-, or
g) R¹⁵⁰R¹⁵¹NSO_{2-;}
wherein R¹⁰² is H, CH₃-, phenyl-CH₂-, or CH₃C(O); each of R¹¹⁰ and R¹¹¹ is selected from H or CH₃; R¹⁰³ is alkylC₁-C₃ or phenyl; R¹⁰⁸ is H, alkylC₁-C₄, aryl(CH₂)₀₋₅, CNCH₂-, ClCH₂-,
Cl₂HC-, FH₂C-, F₂HC-, or cycloalkylC₃-C₆; R¹⁵⁰ and R¹⁵¹ are the same or different and are selected from H, alkylC₁-C₄, or R¹⁵⁰ and R¹⁵¹ taken together with the nitrogen to which each is attached forms a monocyclic heterocyclic ring having from 3 to 6 carbon atoms.

Specifically preferred compounds for use include:
(S)-trans-[[3-(3-Fluoro-4-(tetrahydro-1-oxido-2H-thiopyran-4-yl)phenyl]-2-oxo-5-oxazolidinyl]methyl]thiourea; and
(S)-N-[[3-[3-Fluoro-4-(4-thiomorpholinyl)phenyl]-2-oxo-5-oxazolidinyl]methyl]thioacetamide, thiomorpholine S-oxide.

The compounds used in the present invention can be converted to their salts, where appropriate, according to conventional methods.

The term "pharmaceutically acceptable salts" refers to acid addition salts useful for administering the compounds used in this invention and include hydrochloride, hydrobromide, hydroiodide, sulfate, phosphate, acetate, propionate, lactate, mesylate, maleate, malate, succinate, tartrate, citric acid, 2-hydroxyethyl sulfonate, fumarate and the like. These salts may be in hydrated form.

The compounds are useful for treatment of osteoporosis, bone resorption and other bone diseases in mammals. The term "mammals" is intended to include both humans and non-human vertebrates, including, but not limited to companion animals and food animals. In particularly preferred embodiments, the mammal being treated is not concurrently suffering from an antibacterial infection.

The pharmaceutical compositions used in this invention may be prepared by combining the compounds of formula (I) with a solid or liquid pharmaceutically acceptable carrier and, optionally, with pharmaceutically acceptable adjuvants and excipients employing standard and conventional techniques. Solid form compositions include powders, tablets, dispersible granules, capsules, cachets and suppositories. A solid carrier can be at least one substance which may also function as a diluent, flavoring agent, solubilizer, lubricant, suspending agent, binder, tablet disintegrating agent, and encapsulating agent. Inert solid carriers include magnesium carbonate, magnesium stearate, talc, sugar, lactose, pectin, dextrin, starch, gelatin, cellulosic materials, low melting wax, cocoa butter, and the like. Liquid form compositions include solutions, suspensions and emulsions. For example, there may be provided solutions of the compounds used in this invention dissolved in water and water-propylene glycol and water-polyethylene glycol systems, optionally containing suitable conventional coloring agents, flavoring agents, stabilizers and thickening agents.

Preferably, the pharmaceutical composition is provided employing conventional techniques in unit dosage form containing effective or appropriate amounts of the active component.

The quantity of active component, that is the compound used according to this invention, in the pharmaceutical composition and unit dosage form thereof may be varied or adjusted widely depending upon the particular application, the potency of the particular compound, the desired concentration. Generally, the quantity of active component will range between 0.5% to 90% by weight of the composition.

In therapeutic use for treating or preventing osteoporosis, bone resorption or other bone diseases in vertebrate animals, the compounds or pharmaceutical compositions thereof will be administered orally, nasally, parenterally, topically, transdermally, or rectally at a dosage to obtain and maintain a concentration, that is, an amount, or blood-level of active component in the animal undergoing treatment which will be effective. Generally, such effective amount of dosage of active component will be in the range of about 0.1 to about 100, more preferably about 3.0 to about 50 mg/kg of patient body weight/day. It is to be understood that the dosages may vary depending upon the requirements of the patient, the severity of the condition being treated, and the particular compound being used. Also, it is to be understood that the initial dosage administered may be increased beyond the above upper level in order to rapidly achieve the desired blood-level or the initial dosage may be smaller than the optimum and the daily dosage may be progressively increased during the course of treatment depending on the particular situation. If desired, the daily dose may also be divided into multiple doses for administration, e.g., 2-4 four times per day. Once a day delivery of the compound using osmotic delivery technology such as the OROS system developed by Alza Corp. is also contemplated as falling within the scope of the invention.

When the compounds according to this invention are administered parenterally, i.e., by injection, for example, by intravenous injection or by other parenteral routes of administration, they will generally contain a pharmaceutically acceptable amount of the compound or a soluble salt (acid addition salt or base salt) dissolved in a pharmaceutically acceptable liquid carrier such as, for example, water-for-injection and a buffer to provide a suitably buffered isotonic solution, for example, having a pH of about 3.5-6. Suitable buffering agents include, for example, trisodium orthophosphate, sodium bicarbonate, sodium citrate, N-methylglucamine, L(+)-lysine and L(+)-arginine to name but a few representative buffering agents. The compound of this invention generally will be dissolved in the carrier in an amount sufficient to provide a pharmaceutically acceptable injectable concentration in the range of about 1 mg/mL to about 400 mg/mL of solution. The resulting liquid pharmaceutical composition will be administered so as to obtain the above-mentioned effective amount of dosage. The compounds according to this invention are advantageously administered orally in solid and liquid dosage forms.

The invention is described in greater detail by the following non-limiting examples.

### Example 1

### (S)-trans-[[3-[3-Fluoro-4-(tetrahydro-1-oxido-2H-thiopyran-4-yl)phenyl]-2-oxo-5-oxazolidinyl]methyl]thiourea

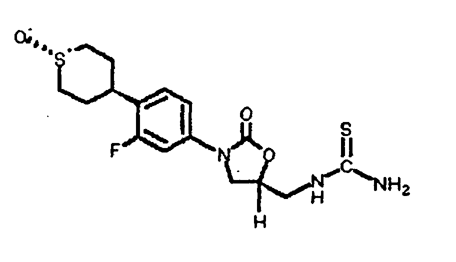

Step 1: A solution of 1,1'-thiocarbonyldi-2(1H)-pyridone (192 mg, 0.827 mmol) in anhydrous methylene chloride (8.3 mL) at 0°C under a nitrogen atmosphere was treated with a solution of (S)-trans-3-[3-fluoro-4-(tetrahydro-1-oxido-2H-thiopyran-4-yl)phenyl]-5-aminomethyl-2-oxazolidinone, as prepared in Example 9 of WO98/54161, Step 1, (225 mg, 0.689 mmol) in anhydrous methylene chloride (28 mL) over 30 minutes. The resulting mixture was stirred at 0°C for 30 minutes and at ambient temperature for 40 minutes and was then diluted with methylene chloride (20 mL), washed with water (15 mL) and brine (15 mL), dried over anhydrous sodium sulfate and concentrated *in vacuo*. The crude product was chromatographed on silica gel (32 - 63 mm, 40 g), eluting with a gradient of acetonitrile/methylene chloride (30/70 - 60/40) under 15 psi N₂, and those fractions with an R_{f} = 0.12 by TLC (acetonitrile/methylene chloride, 30/70) were pooled and concentrated to give (S)-trans-3-[3-Fluoro-4-(tetrahydro-1-oxido-2H-thiopyran-4- yl)phenyl]-5-isothiocyanatomethyl-2-oxazolidinone, mp 165 - 167°C.

Step 2: A solution of (S)-trans-3-[3-fluoro-4-(tetrahydro-1-oxido-2H-thiopyran-4-yl)phenyl]-5-isothiocyanatomethyl-2-oxazolidinone (Step 1, 230 mg, 0.624 mmol) in anhydrous tetrahydrofuran (31.2 mL) at 0°C under a nitrogen atmosphere was treated (bubbled) with a stream of ammonia gas for 5 minutes. The reaction pot was sealed, and the resulting mixture was stirred at 0°C for 1 hour. The excess ammonia was then removed under a stream of nitrogen, and the reaction mixture was concentrated *in vacuo* to give the crude product. Trituration with methanol/methylene chloride/diethyl ether gave the title compound, mp 209 - 210°C (dec.).

### Example 2

### (S)-N-[[3-[3-Fluoro-4-(4-thiomorpholinyl)phenyl]-2-oxo-5-oxazolidinyl]methyl]thioacetamide, thiomorpholine S-oxide (34).

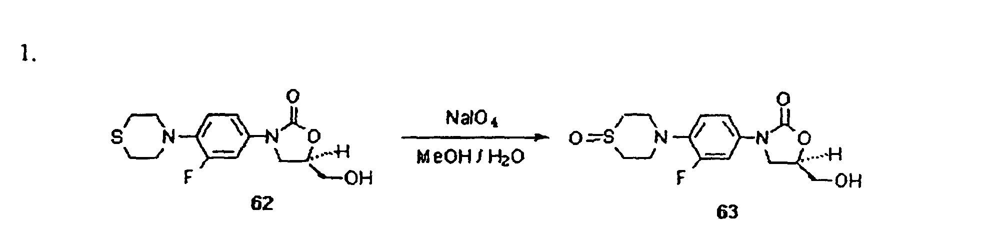

An ice cold, stirred mixture of sodium metaperiodate (1.08 g, 5.05 mmol) and water (12 mL), under nitrogen, was treated with **62** (1.5 g, 4.8 mmol) and MeOH (17 mL) and kept at 6 °C for 18 h and at 4 °C for 3 h. It was then treated with additional sodium metaperiodate (0.1 g), kept at 4°C for 3 h and extracted with CHCl₃. The extract was dried (MgSO₄) and concntrated to give 1.4 g of 63: ¹H NMR [300 MHz, (CD₃)₂SO] d 2.84 (m, 2H), 3.01 (m, 2H), 3.16 (m, 2H), 3.50 (m, 3H), 3.65 (m, 1H), 3.77 (d,d, 1H), 4.03 (t, 1H), 4.66 (m, 1H), 5.18 (t, 1H), 7.16 (m, 2H), 7.52 (m, 1H); MS(ES) *m*/*z* 329 (M+H⁺), 351 (M+Na⁺).

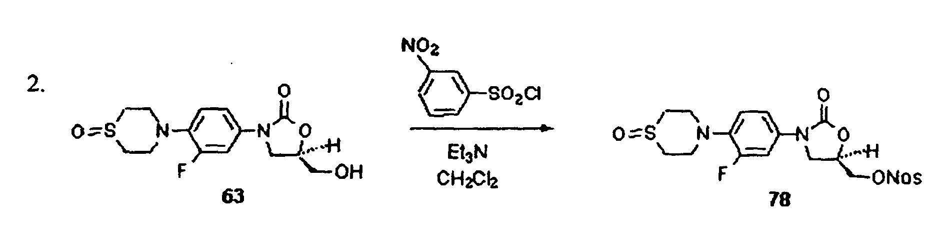

An ice cold, stirred mixture of **63** (1.27 g, 3.87 mmol) and triethylamine (0.732 mL, 5.25 mmol) in CH₂Cl₂ (130 mL), under nitrogen, was treated with *m*-nitrobenzenesulfonyl chloride (1.15 g, 5.19 mmol) and kept at ambient temperature for about 24 h. It was diluted with CH₂Cl₂, washed with water and brine, dried (Na₂SO₄) and concentrated to give **78** which was used in the next reaction without purification.

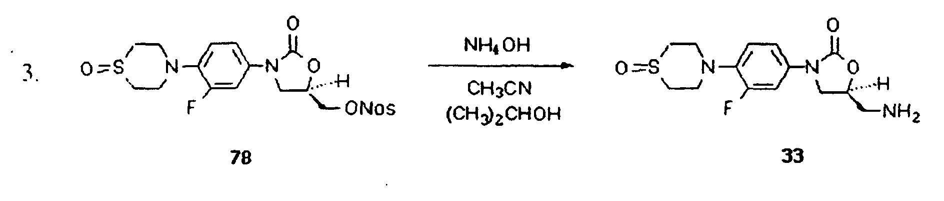

A stirred mixture of the product (**78**) from the previous reaction, acetonitrile (70 mL) and isopropanol (70 mL) was treated with concentrated ammonium hydroxide (70 mL, 29.9% NH₃) and kept at 40 °C for 2 h, at ambient temperature for 18 h and at 40-45 °C for 4 h; it was concentrated to about 50 mL, diluted with water and extracted with CH₂Cl₂. The extracts were washed with water and brine, dried (MgSO₄) and concentrated. Chromatography of the residue on silica gel with 5% MeOH-CHCl₃ gave 0.58 g of 33: MS(ES) *m*/*z* 328 (M+H⁺), 350 (M+Na⁺); ¹H NMR [300 MHz, (CD₃)₂SO] d 2.81 (m, 4H), 3.01 (m, 2H), 3.16 (m, 2H), 3.30 (broad s), 3.49 (m, 2H), 3.80 (d,d, 1H), 4.01 (t, 1H), 4.58 (m, 1H), 7.19 (m, 2H), 7.51 (m, 1H).

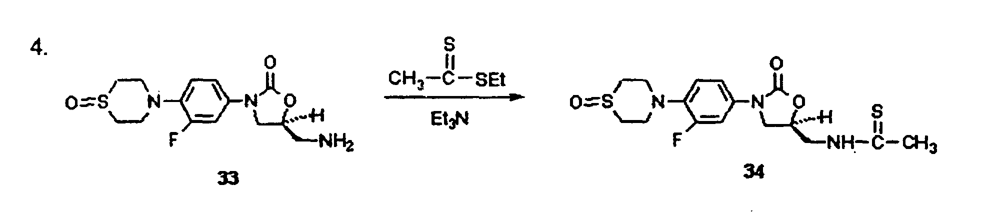

A stirred suspension of **33** (3.7 g, 0.011 mol) and triethylamine (3.5 mL, 0.025 mol) in THF (120 mL) was cooled, in an ice bath, under nitrogen, treated, dropwise during 2 min, with a solution of ethyl dithioacetate (1.47 mL, 0.0128 mol) in THF (2 mL) and kept at ambient temperature for 22 h. The resulting solution was concentrated and the residue crystallized from acetonitrile to give 3.61 g of **34**: mp 176-177 °C ; ¹H NMR [300 MHz, (CD₃)₂SO] d 2.42 (s, 3H), 2.85 (m, 2H), 3.01 (m, 2H), 3.18 (m, 3H), 3.50 (m, 2H), 3.78 (d,d, 1H), 3.89 (broad s, 2H), 4.12 (t, 1H), 4.92 (m, 1H), 7.18 (m, 2H), 7.49 (m, 1H), 10.33 (s, 1H); IR (DRIFT) 3186, 3102, 1741 cm⁻¹; MS(ES) *m*/z 386 (M+H⁺), 408 (M+Na⁺). Anal. calcd for C₁₆H₂₀FN₃O₃S₂°0.5 H₂O: C, 48.71; H, 5.37; N, 10.65; S, 16.26; H₂O, 2.38. Found: C, 48.75; H, 5.17; N, 10.72; S, 16.07; H₂O, 1.72.

### Use of the Compounds

The toxicity of the compound of Example 2 was evaluated in rats treated orally with 30, 60, 120, or 200 mg/kg/day of the compound of Example 2, water, or vehicle (containing 80% propylene glycol, 5% cremophor, and 30-mg/ml povidone) for 4 weeks given in 2 divided doses at 8 hours apart. Reversibility of drug-induced changes was determined 4 and 8 weeks following drug withdrawal. The pathogenesis of the bone changes was evaluated in a study in rats treated with 200 mg/kg/day for 3 or 7 days. TGF-beta1 protein levels in bone marrow supernatants and serum were determined using ELISA at the end of each of the dosing period. Toxicity was evaluated using clinical, hematological, biochemical, and pathologic end points.

All doses, but 200 mg/kg/day, were well tolerated clinically. In addition to anorexia, weight loss, GI effects, hematopoietic suppression and lymphoid depletion, administration of the compound of Example 2, at 120 and 200 mg/kg/day, caused an increase in bone mass and density (hyperostosis) of the trabecular (spongy) bone of the sternum, femur/tibia, vertebrae, and basal cranium. Endosteal cortical bone formation occurred rarely. Hyperostosis, which became evident by Day 7 was characterized by proliferation of osteoblasts and deposition of osteoid within the marrow cavity in a trabecular configuration and/or as deposits of new bone over the existing trabeculae. At Day 28, marrow cavities were traversed by abundant normal looking trabecular bone that was lined by some peri-trabecular osteoblast proliferation and osteoid deposition. There were no significant osseous changes on Day 3 except for some subtle focal proliferation/activation of osteoblasts. The newly formed bone spicules, still present after 4 and 8 weeks of drug withdrawal, were qualitatively indistinguishable from the pre-existing bone. In relatively severe cases, osteoblast proliferation and osteoid accumulation on Day 7 and newly formed trabecular bones on Day 28 or after the recovery periods compromised or obliterated the marrow spaces. However, the bone marrow did not show changes other than hypocellularity related to hematopoietic suppression. TGF-beta1 was modestly but significantly increased in marrow supernatants on Day 3 but not on Day 7 or 28. Serum TGF-beta1 was slightly but significantly decreased on Day 3. Increased levels of TGF-beta1 in marrow supernatant preceded hyperostosis suggesting a possible mechanistic relationship. The compound of Example 2 increased bone mass and density within a short period of time in an orderly manner at a well-tolerated doses. Newly formed bone that persisted for up to 8 weeks after drug withdrawal (the longest period evaluated) was morphologically indistinguishable from normal trabecular bones.

## Claims

1. The use of an effective amount of a compound of formula I or pharmaceutical acceptable salts thereof to prepare a medicament for treating or preventing osteoporosis, bone resorption or other hone disease in a vertebrate mammal, 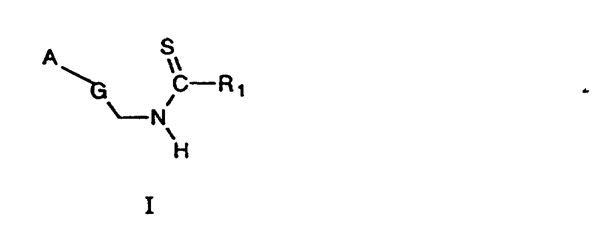 wherein:
G is 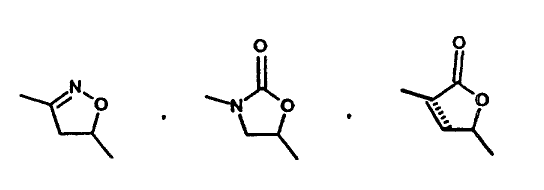
R₁ is
a) H,
b) NH₂,
c) NH-C₁₋₄ alkyl,
d) C₁₋₄ alkyl,
e) -OC₁₋₄ alkyl,
f) -S C₁₋₄ alkyl,
g) C₁₋₄ alkyl substituted with 1-3 F, 1-2 Cl, CN or -COOC₁₋₄ alkyl,
h) C₃₋₆ cycloalkyl,
i) N(C₁₋₄ alkyl)₂ or
j) N(CH₂)₂₋₅;
A is
a) 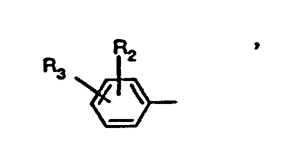
b) 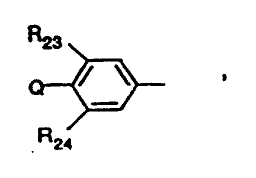
c) 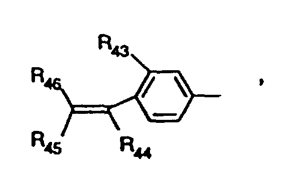
d) a 5-membered heteroaromatic moiety having one to three atoms selected from the group consisting of S, N, and O,
wherein the 5-membered heteroaromatic moiety is bonded via a carbon atom,
wherein the 5-membered heteroaromatic moiety can additionally have a fused-on benzene or naphthyl ring,
wherein the heteroaromatic moiety is optionally substituted with one to three R₄₈,
e) a 6-membered heteroaromatic moiety having at least one nitrogen atom,
wherein the heteroaromatic moiety is bonded via a carbon atom,
wherein the 6-membered heteroaromatic moiety can additionally have a fused-on benzene or naphthyl ring,
wherein the heteroaromatic moiety is optionally substituted with one to three R₅₅,
f) a β-carbolin-3-yl, or indolizinyl bonded via the 6-membered ring, optionally substituted with one to three R₅₅,
g) 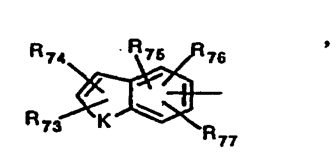 or
h) 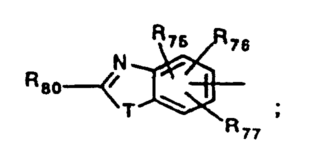
wherein R₂ is
a) H,
b) F,
c) Cl,
d) Br,
e) C₁₋₃ alkyl,
f) NO₂, or
g) R₂ and R₃ taken together are -O-(CH₂)ₕ-O-;
R₃ is
a) -S(=O)ᵢ R₄,
b) -S(=O)₂-N=S(O)ⱼR₅R₆,
c) -SC(=O)R₇,
d) -C(=O)R₈,
e) -C(=O)R₉,
f) -C(=O)NR₁₀R₁₁,
g) -C(=NR₁₂)R₈,
h) -C(R₈)(R₁₁)-OR₁₃,
i) -C(R₉)(R₁₁)-OR₁₃,
j) -C(R₈)(R₁₁)-OC(=O)R₁₃,
k) -C(R₉)(R₁₁)-OC(=O)R₁₃,
l) -NR₁₀R₁₁,
m) -N(R₁₀)-C(=O)R₇,
n) -N(R₁₀)-S(=O)ᵢR₇,
o) -C(OR₁₄)(OR₁₅)R₈,
p) -C(R₈)(R₁₆)-NR₁₀R₁₁, or
q) C₁₋₈ alkyl substituted with one or more =O other than at alpha position, -S(=O)ᵢR₁₇, -NR₁₀R₁₁, C₂₋₅ alkenyl, or C₂₋₅ alkynyl;
R₄ is
a) C₁₋₄ alkyl optionally substituted with one or more halos, OH, CN, NR₁₀R₁₁, or -CO₂R₁₃,
b) C₂₋₄ alkenyl,
c) -NR₁₆R₁₈,
d) -N₃,
e) -NHC(=O)R₇,
f) -NR₂₀C(=O)R₇,
g) -N(R₁₉)₂,
h) -NR₁₆R₁₉, or
i) -NR₁₉R₂₀,
R₅ and R₆ at each occurrence are the same or different and are
a) C₁₋₂ alkyl, or
b) R₅ and R₆ taken together are -(CH₂)ₖ-;
R₇ is C₁₋₄ alkyl optionally substituted with one or more halos;
R₈ is
a) H, or
b) C₁₋₈ alkyl optionally substituted with one or more halos, or C₃₋₈ cycloalkyl;
R₉ is C₁₋₄ alkyl substituted with one or more
a) -S(=O)R₁₇,
b) -OR₁₃,
c) -OC(=O)R₁₃,
d) -NR₁₀R₁₁, or
e) C₁₋₅ alkenyl optionally substituted with CHO;
R₁₀ and R₁₁ at each occurrence are the same or different and are
a) H,
b) C₁₋₄ alkyl, or
c) C₃₋₈ cycloalkyl;
R₁₂ is
a) -NR₁₀R₁₁,
b) -OR₁₀; or
c) -NHC(=O)R₁₀;
R₁₃ is
a) H, or
b) C₁₋₄ alkyl;
R₁₄ and R₁₅ at each occurrence are the same or different and are
a) C₁₋₄ alkyl, or
b) R₁₄ and R₁₅ taken together are -(CH)₁-;
a) H,
b) C₁₋₄ alkyl, or
c) C₃₋₈ cycloalkyl;
R₁₇ is
a) C₁₋₄ alkyl, or
b) C₃₋₈ cycloalkyl;
R₁₈ is
a) H,
b) C₁₋₄ alkyl,
c) C₂₋₄ alkenyl,
d) C₃₋₄ cycloalkyl,
e) -OR₁₃ or
f) -NR₂₁R₂₂;
R₁₉ is
a) Cl,
b) Br, or
c) I;
R₂₀ is a physiologically acceptable cation;
R₂₁ and R₂₂ at each occurrence are the same or different and are
a) H,
b) C₁₋₄ alkyl, or
c) -NR₂₁R₂₂ taken together are -(CH₂)ₘ-;
wherein R₂₃ and R₂₄ at each occurrence are the same or different and are
a) H,
b) F,
c) Cl,
d) C₁₋₂ alkyl,
e) CN
f) OH,
g) C₁₋₂ alkoxy,
h) nitro, or
i) amino;
Q is
a) 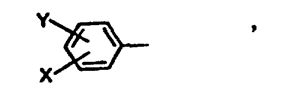
b) 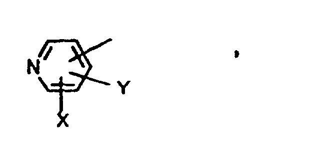
c) 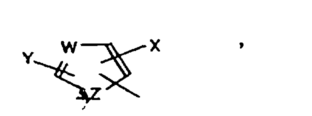
d) 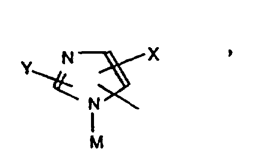
e) 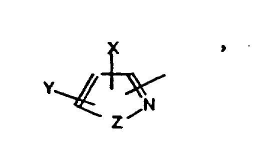
f) 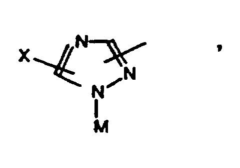
g) 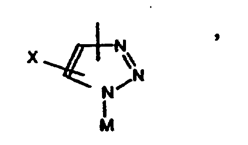
h) 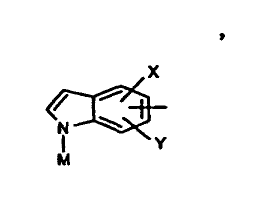
i) 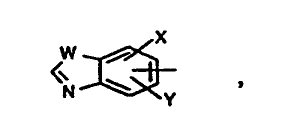
j) 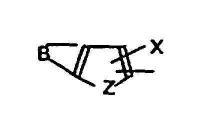
k) 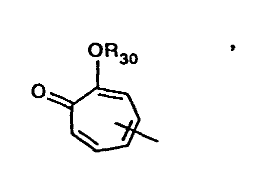
l) 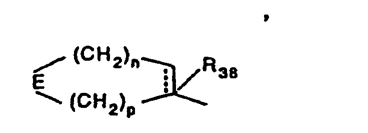
m) a diazinyl group optionally substituted with X and Y,
n) a triazinyl group optionally substituted with X and Y,
o) a quinolinyl group optionally substituted with X and Y,
p) a quinoxalinyl group optionally substituted with X and Y,
q) a naphthyridinyl group optionally substituted with X and Y,
r) 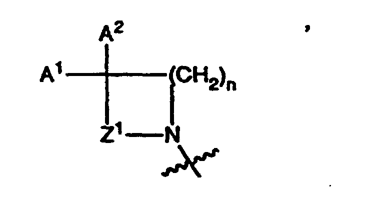
s) 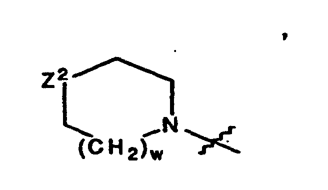
t) 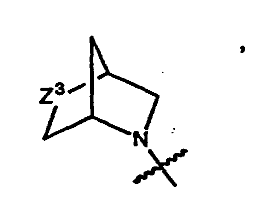
u) 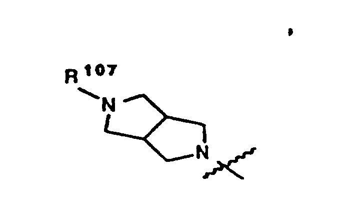
v) 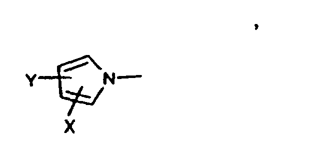
w) 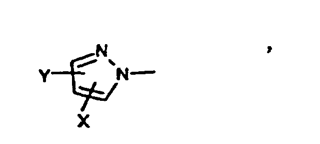
x) 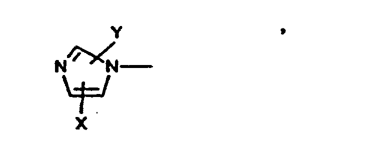
y) 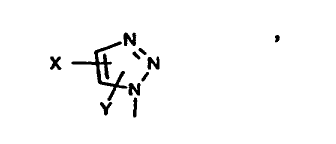
z) 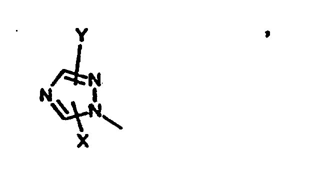
aa) 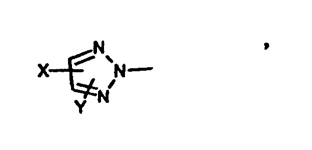
bb) 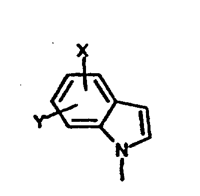 or,
Q and R₂₄ taken together are 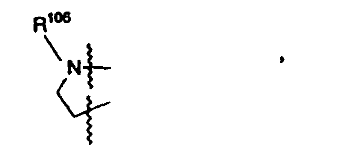
wherein Z¹ is
a) -CH₂-,
b) -CH(R¹⁰⁴)-CH₂-,
c) -C(O)-, or
d) -CH₂CH₂CH₂-;
wherein Z² is
a) -O₂S-,
b) -O-,
c) -N(R¹⁰⁷)-,
d) -OS-, or
e) -S-;
wherein Z³ is
a) -O₂S-,
b) -O-,
c) -OS-, or
d) -S-;
wherein A¹ is
a) H-, or
b) CH₃;
wherein A² is
a) H-,
b) HO-,
c) CH₃-,
d) CH₃O-,
e) R¹⁰²O-CH₂-C(O)-NH-
f) R¹⁰³O-C(O)-NH-,
g) (C₁-C₂)alkyl-O-C(O)-,
h) HO-CH₂-,
i) CH₃O-NH-,
j) (C₁-C₃)alkyl-O₂C-
k) CH₃-C(O)-,
l) CH₃-C(O)-CH₂-,
m) 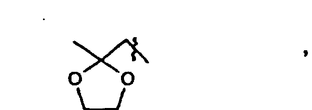 or
n) 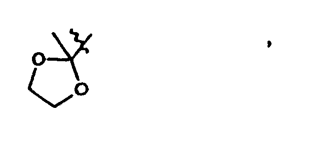
A¹ and A² taken together are:
a) 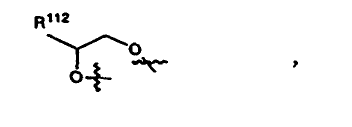
b) o= , or
c) 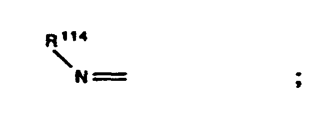
wherein R¹⁰² is
a) H-,
b) CH₃-,
c) phenyl-CH₂-, or
d) CH₃C(O)-;
wherein R¹⁰³ is
a) (C₁-C₃)alkyl-, or
b) phenyl-;
wherein R¹⁰⁴ is
a) H-, or
b) HO-;
wherein R¹⁰⁵ is
a) H-,
b) (C₁-C₃)alkyl-,
c) CH₂ = CH-CH₂-, or
d) CH₃-O-(CH₂)₂-;
wherein R¹⁰⁶ is
a) CH₃-C(O)-,
b) H-C(O)-,
c) Cl₂CH-C(O)-,
d) HOCH₂-C(O)-,
e) CH₃SO₂-,
f) 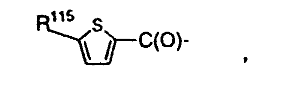
g) F₂CHC(O)-,
h) 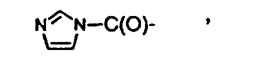
i) H₃C-C(O)-O-CH₂-C(O)-,
j) H-C(O)-O-CH₂-C(O)-,
k) 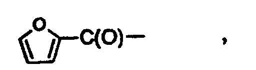
l) HC≡C-CH₂O-CH₂-C(O)-, or
m) phenyl-CH₂-O-CH₂-C(O)-;
wherein R¹⁰⁷ is
a) R¹⁰²O-C(R¹¹⁰)(R¹¹¹)-C(O)-,
b) R¹⁰³O-C(O)-,
c) R¹⁰⁸-C(O)-,
d) 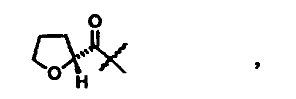
e) 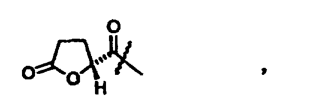
f) H₃C-C(O)-(CH₂)₂-C(O)-,
g) R¹⁰⁹-SO₂-,
h) 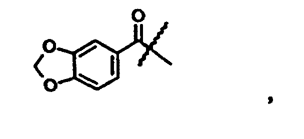
i) HO-CH₂-C(O)-,
j) R¹¹⁶-(CH₂)₂-,
k) R¹¹³-C(O)-O-CH₂-C(O)-,
l) (CH₃)₂N-CH₂-C(O)-NH-,
m) NC-CH₂-,
n) F₂-CH-CH₂-, or
o) R¹⁵⁰R¹⁵¹NSO₂
wherein R¹⁰⁸ is
a) H-,
b) (C₁-C₄)alkyl,
c) aryl -(CH₂)ₚ,
d) ClH₂C-,
e) Cl₂HC-,
f) FH₂C-,
g) F₂HC-,
h) (C₃-C₆)cycloalkyl, or
i) CNCH₂-.
wherein R¹⁰⁹ is
a) alkylC₁-C₄,
b) -CH₂Cl
c) -CH₂CH=CH₂,
d) aryl, or
e) -CH₂CN;
wherein R¹¹⁰ and R¹¹¹ are independently
a) H-,
b) CH₃-; or
wherein R¹¹² is
a) H-,
b) CH₃O-CH₂O-CH₂-, or
c) HOCH₂-;
wherein R¹¹³ is
a) CH₃-,
b) HOCH₂-,
c) (CH₃)₂N-phenyl, or
d) (CH₃)₂N-CH₂-;
wherein R¹¹⁴ is
a) HO-,
b) CH₃O-,
c) H₂N-,
d) CH₂O-C(O)-O-,
e) CH₃-C(O)-O-CH₂-C(O)-O-,
f) phenyl-CH₂-O-CH₂-C(O)-O-,
g) HO-(CH₂)₂-O-,
h) CH₃O-CH₂-O-(CH₂)₂-O-, or
i) CH₃O-CH₂-O-;wherein R¹¹³ is
a) CH₃-,
b) HOCH₂-,
c) (CH₃)₂N-phenyl, or
d) (CH₃)₂N-CH₂-;
wherein R¹¹⁵ is
a) H-, or
b) Cl-;
wherein R¹¹⁶ is
a) HO-
b) CH₃O-, or
c) F;
wherein R¹⁵⁰ and R¹⁵¹ are each H or alkyl C₁-C₄ or R¹⁵⁰ and R¹⁵¹ taken together with the nitrogen atom to which each is attached form a monocyclic heterocyclic ring having from 3 to 6 carbon atoms;
B is an unsaturated 4-atom linker having one nitrogen and three carbons;
M is
a) H,
b) C₁₋₈ alkyl,
c) C₃₋₈ cycloalkyl,
d) -(CH₂)ₘOR₁₃, or
e) -(CH₂)ₕ-NR₂₁R₂₂;
Z is
a) O,
b) S, or
c) NM;
W is
a) CH,
b) N, or
c) S or O when Z is NM;
Y is
a) H,
b) F,
c) Cl,
d) Br,
e) C₁₋₃ alkyl, or
f) NO₂;
X is
a) H,
b) -CN,
c) OR₂₇,
d) halo,
e) NO₂,
f) tetrazoyl,
g) -SH,
h) -S(=O)ᵢR₄,
i) -S(=O)₂-N=S(O)ⱼR₅R₆,
j) -SC(=O)R₇,
k) -C(=O)R₂₅,
l) -C(=O)NR₂₇R₂₈,
m) -C(=NR₂₉)R₂₅,
n) -C(R₂₅)(R₂₈)-OR₁₃,
o) -C(R₂₅)(R₂₈)-OC(=O)R₁₃,
p) -C(R₂₈)(OR₁₃)-(CH₂)ₕ-NR₂₇R₂₈,
q) -NR₂₇R₂₈,
r) -N(R₂₇)C(=O)R₇,
s) -N(R₂₇)-S(=O)ᵢR₇,
t) -C(OR₁₄)(OR₁₅)R₂₈,
u) -C(R₂₅)(R₁₆)-NR₂₇R₂₆, or
v) C₁₋₈ alkyl substituted with one or more halos, OH, =O other than at alpha position, -S(=O)ᵢR₁₇, -NR₂₇R₂₈, C₂₋₅ alkenyl, C₂₋₅ alkynyl, or C₃₋₈ cycloalkyl;
R₄, R₅, R₆, R₇, R₁₃, R₁₄, R₁₅, R₁₆, and R₁₇ are the same as defined above; R₂₅ is
a) H,
b) C₁₋₈ alkyl optionally substituted with one or more halos, C₂₋₆ cycloalkyl, C₁₋₄ alkyl substituted with one or more of -S(=O)_{f}R₁₇, -OR₁₃. or OC(=O)R₁₃, NR₂₇R₂₈, or
c) C₂₋₅ alkenyl optionally substituted with CHO, or CO₂R₁₃;
R₂₆ is
a) R₂₈, or
b) NR₂₇N₂₈;
R₂₇ and R₂₈ at each occurrence are the same or different and are
a) H.
b) C₁₋₈ alkyl,
c) C₃₋₈ cycloalkyl,
d) -(CH₂)ₘOR₁₃,
e) -(CH₂)ₕ-NR₂₁R₂₂, or
f) R₂₇ and R₂₈ taken together are -(CH₂)₂O(CH₂)₂-, -(CH₂)ₕCH(COR₇)-, or -(CH₂)₂N(CH₂)₂(R₇);
R₂₉ is
a) -NR₂₇R₂₈,
b) -OR₂₇, or
c) -NHC(=O)R₂₈;
wherein R₃₀ is
a) H,
b) C₁₋₈ alkyl optionally substituted with one or more halos, or
c) C₁₋₈ alkyl optionally substituted with one or more OH, or C₁₋₆ alkoxy;
wherein E is
a) NR₃₉,
b) -S(=O)ᵢ, or
c) O;
R₃₈ is
a) H,
b) C₁₋₆ alkyl,
c) -(CH₂)_{q}-aryl, or
d) halo;
R₃₃ is
a) H,
b) C₁₋₆ alkyl optionally substituted with one or more OH, halo, or -CN,
c) -(CH₂)_{q}-aryl,
d) -CO₂R₄₀,
e) -COR₄₁,
f) -C(=O)-(CH₂)_{q}-C(=O)R₄₀,
g) -S(=O)₂-C₁₋₆ alkyl,
h) -S(=O)₂-(CH₂)_{q}-aryl, or
i) -(C=O)ⱼ-Het;
R₄₀ is
a) H,
b) C₁₋₆ alkyl optionally substituted with one or more OH, halo, or -CN,
c) -(CH₂)_{q}-aryl, or
d) -(CH₂)_{q}-OR₄₂;
R₄₁ is
a) C₁₋₆ alkyl optionally substituted with one or more OH, halo, or -CN,
b) -(CH₂)_{q}-aryl, or
c) -(CH₂)_{q}-OR₄₂;
R₄₂ is
a) H,
b) C₁₋₆ alkyl,
c) -(CH₂)_{q}-aryl, or
d) -C(=O)-C₁₋₆ alkyl;
aryl is
a) phenyl,
b) pyridyl, or
c) napthyl; a to c optionally substituted with one or more halo, -CN, OH, SH, C₁₋₆ alkyl, C₁₋₆ alkoxy, or C₁₋₆ alkylthio;
wherein R₄₃ is
a) H,
b) C₁₋₂ alkyl,
c) F, or
d) OH;
R₄₄ is
a) H,
b) CF₃,
c) C₁₋₃ alkyl optionally substituted with one or more halo,
d) phenyl optionally substituted with one or more halo,
e) R₄₄ and R₄₅ taken together are a 5-, 6-, or 7-membered ring of the formula, 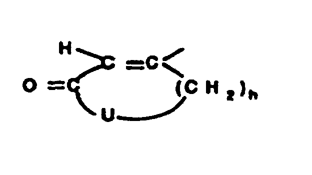 or
f) R₄₄ and R₄₅ taken together are -(CH₂)ₖ-, when R₄₆ is an electron-withdrawing group;
R₄₅ and R₄₆ at each occurrence are the same or different and are
a) an electron-withdrawing group,
b) H,
c) CF₃,
d) C₁₋₃ alkyl optionally substituted with one halo,
e) phenyl, provided at least one of R₄₅ or R₄₆ is an electron-withdrawing group, or
f) R₄₅ and R₄₆ taken together are a 5-, 6-, 7-membered ring of the formula 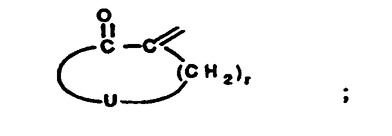
U is
a) CH₂,
b) O,
c) S, or
d) NR₄₇;
R₄₇ is
a) H, or
b) C₁₋₅ alkyl;
wherein R₄₈ is
a) carboxyl,
b) halo,
c) -CN,
d) mercapto,
e) formyl,
f) CF₃,
g) -NO₂,
h) C₁₋₆ alkoxy,
i) C₁₋₆ alkoxycarbonyl,
j) C₁₋₆ alkythio,
k) C₁₋₆ acyl,
l) -NR₄₉ R₅₀,
m) C₁₋₆ alkyl optionally substituted with OH, C₁₋₅ alkoxy, C₁₋₅ acyl, or - NR₄₉R₅₀,
n) C₂₋₈ alkenylphenyl optionally substituted with one or two R₅₁,
o) phenyl optionally substituted with one or two R₅₁,
p) a 5-, or 6-membered (un)saturated heterocyclic moiety having one to three atoms selected from the group consisting of S, N, and O, optionally substituted with one or two R₅₁, or
q) 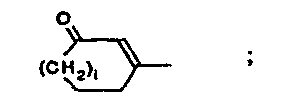
R₄₉ and R₅₀ at each occurrence are the same or different and are
a) H,
b) C₁₋₄ alkyl,
c) C₅₋₆ cycloalkyl, or
d) R₄₉ and R₅₀ taken together with the nitrogen atom is a 5-, 6-membered saturated heterocyclic moiety which optionally has a further hetero atom selected from the group consisting of S, N, and O, and can in turn be optionally substituted with, including on the further nitrogen atom, C₁₋₃ alkyl, or C₁₋₃ acyl;
R₅₁ is
a) carboxyl,
b) halo,
c) -CN,
d) mercapto,
e) formyl,
f) CF₃,
g) -NO₂,
h) C₁₋₆ alkoxy,
i) C₁₋₆ alkoxycarbonyl,
j) C₁₋₆ alkythio,
k) C₁₋₆ acyl,
l) C₁₋₆ alkyl optionally substituted with OH, C₁₋₅ alkoxy, C₁₋₅ acyl, or -NR₄₉R₅₀,
m) phenyl,
n) -C(=O)NR₅₂ R₅₃,
o) -NR₄₉R₅₀,
p) -N(R₅₂)(-SO₂R₅₄),
q) -SO₂-NR₅₂R₅₃, or
r) -S(=O)ᵢR₅₄;
R₅₂ and R₅₃ at each occurrence are the same or different and are
a) H,
b) C₁₋₆ alkyl, or
c) phenyl;
R₅₄ is
a) C₁₋₄ alkyl, or
b) phenyl optionally substituted with C₁₋₄ alkyl;
wherein R₅₅ is
a) carboxyl,
b) halo,
c) -CN,
d) mercapto,
e) formyl,
f) CF₃,
g) -NO₂,
h) C₁₋₆ alkoxy,
i) C₁₋₆ alkoxycarbonyl,
j) C₁₋₆ alkythio
k) C₁₋₆ acyl,
l) -NR₅₆ R₅₇,
m) C₁₋₆ alkyl optionally substituted with OH, C₁₋₅ alkoxy, C₁₋₅ acyl, or -NR₅₆R₅₇,
n) C₂₋₈ alkenylphenyl optionally substituted with one or two R₅₈,
o) phenyl optionally substituted with one or two R₅₈,
p) a 5- or 6-membered (un)saturated heterocyclic moiety having one to three atoms selected from the group consisting of S, N, and O, optionally substituted with one or two R₅₈, or
q) 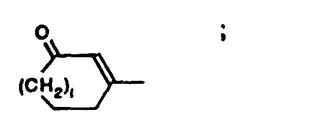
R₅₆ and R₅₇ at each occurrence are the same or different and are
a) H,
b) formyl,
c) C₁₋₄ alkyl,
d) C₁₋₄ acyl,
e) phenyl,
f) C₃₋₆ cycloalkyl, or
g) R₅₆ and R₅₇ taken together with the nitrogen atom is a 5-, 6-membered saturated heterocyclic moiety which optionally has a further hetero atom selected from the group consisting of S, N, and O, and can in turn be optionally substituted with, including on the further nitrogen atom, phenyl, pyrimidyl, C₁₋₃ alkyl, or C₁₋₃ acyl;
R₅₈ is
a) carboxyl,
b) halo,
c) -CN,
d) mercapto,
e) formyl,
f) CF₃,
g) -NO₂,
h) C₁₋₆ alkoxy,
i) C₁₋₆ alkoxycarbonyl,
j) C₁₋₆ alkythio,
k) C₁₋₆ acyl,
l) phenyl,
m) C₁₋₆ alkyl optionally substituted with OH, azido, C₁₋₅ alkoxy, C₁₋₅ acyl, -NR₆₅R₆₆, -SR₆₇, -O-SO₂R₆₈, or 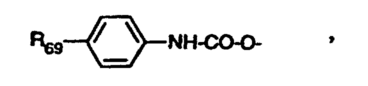
n) -C(=O)NR₅₉ R₆₀,
o) -NR₅₆R₅₇,
p) -N(R₅₉)(-SO₂R₅₄),
q) -SO₂-NR₅₉R₆₀,
r) -S(=O)ᵢR₅₄,
s) -CH=N-R₆₁, or
t) -CH(OH)-SO₃R₆₄;
R₅₄ is the same as defined above;
R₅₉ and R₆₀ at each occurrence are the same or different and are
a) H,
b) C₁₋₆ alkyl,
c) phenyl, or
d) tolyl;
R₆₁ is
a) OH,
b) benzyloxy,
c) -NH-C(=O)-NH₂,
d) -NH-C(=S)-NH₂, or
e) -NH-C(=NH)-NR₆₂R₆₃;
R₆₂ and R₆₃ at each occurrence are the same or different and are
a) H, or
b) C₁₋₄ alkyl optionally substituted with phenyl or pyridyl;
R₆₄ is
a) H, or
b) a sodium ion;
R₆₅ and R₆₆ at each occurrence are the same or different and are
a) H,
b) formyl,
c) C₁₋₄ alkyl,
d) C₁₋₄ acyl,
e) phenyl,
f) C₃₋₆ cycloalkyl,
g) R₆₅ and R₆₆ taken together are a 5-, 6-membered saturated heterocyclic moiety having one to three atoms selected from the group consisting of S, N, and O, optionally substituted with, including on the nitrogen atom, phenyl, pyrimidyl, C₁₋₃ alkyl, or C₁₋₃ acyl,
h) -P(O)(OR₇₀)(OR₇₁), or
i) -SO₂-R₇₂;
R₆₇ is 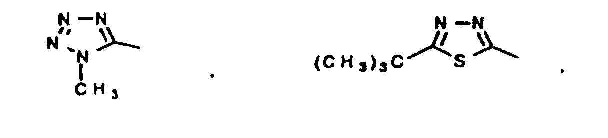 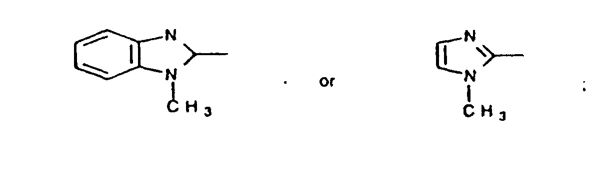
R₆₈ is C₁₋₃ alkyl;
R₆₉ is
a) C₁₋₆ alkoxycarbonyl, or
b) carboxyl;
R₇₀ and R₇₁ at each occurrence are the same or different and are
a) H, or
b) C₁₋₃ alkyl;
R₇₂ is
a) methyl,
b) phenyl, or
c) tolyl;
wherein K is
a) O, or
b) S;
R₇₃, R₇₄, R₇₅, R₇₆, and R₇₇ at each occurrence are the same or different and are
a) H,
b) carboxyl,
c) halo,
d) -CN,
e) mercapto,
f) formyl,
g) CF₃,
h) -NO₂,
i) C₁₋₅ alkoxy,
j) C₁₋₅ alkoxycarbonyl,
k) C₁₋₆ alkythio,
l) C₁₋₆ acyl,
m) -NR₇₈ R₇₉,
n) C₁₋₆ alkyl optionally substituted with OH, C₁₋₅ alkoxy, C₁₋₅ acyl, -NR₇₈R₇₉, -N(phenyl)(CH₂-CH₂-OH), -O-CH(CH₃)(OCH₂CH₃), or - O-phenyl-[para-NHC(=O)CH₃],
o) C₂₋₈ alkenylphenyl optionally substituted with R₅₁,
p) phenyl optionally substituted with R₅₁, or
q) a 5-, or 6-membered (un)saturated heterocyclic moiety having one to three atoms selected from the group consisting of S, N, and O, optionally substituted with R₅₁;
R₅₁ is the same as defined above;
R₇₈ and R₇₉ at each occurrence are the same or different and are
a) H,
b) C₁₋₄ alkyl,
c) phenyl, or
d) R₇₈ and R₇₉ taken together with the nitrogen atom is a 5-, 6-membered saturated heterocyclic moiety which optionally has a further hetero atom selected from the group consisting of S, N, and O, and can in turn be optionally substituted with, including on the further nitrogen atom, C₁₋₃ alkyl, or C₁₋₃ acyl;
wherein T is
a) O,
b) S, or
c) SO₂;
R₇₅, R₇₆, and R₇₇ are the same as defined above;
R₈₀ is
a) H,
b) formyl,
c) carboxyl,
d) C₁₋₆ alkoxycarbonyl,
e) C₁₋₈ alkyl,
f) C₂₋₈ alkenyl,
wherein the substituents (e) and (f) can be optionally substituted with OH, halo, C₁₋₆ alkoxy, C₁₋₆ acyl, C₁₋₆ alkylthio or C₁₋₆ alkoxycarbonyl, or phenyl optionally substituted with halo,
g) an aromatic moiety having 6 to 10 carbon atoms optionally substituted with carboxyl, halo, -CN, formyl, CF₃, -NO₂, C₁₋₆ alkyl, C₁₋₆ alkoxy, C₁₋₆ acyl, C₁₋₆ alkylthio, or C₁₋₆ alkoxycarbonyl;
h) -NR₈₁R₈₂,
i) -OR₉₀,
j) -S(=O)ᵢ-R₉₁, or
k) -SO₂-N(R₉₂) (R₉₃);
R₈₁ and R₈₂ at each occurrence are the same or different and are
a) H,
b) C₃₋₆ cycloalkyl,
c) phenyl,
d) C₁₋₆ acyl,
e) C₁₋₈ alkyl optionally substituted with OH, C₁₋₆ alkoxy which can be substituted with OH, a 5-, or 6-membered aromatic heterocyclic moiety having one to three atoms selected from the group consisting of S, N, and O, phenyl optionally substituted with OH, CF₃, halo, -NO₂, C₁₋₄ alkoxy, -NR₈₃R₈₄, or 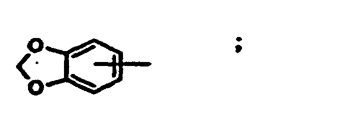
f) 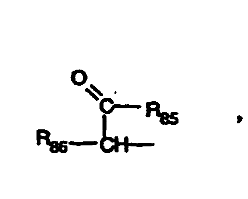 or
g) 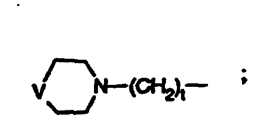
V is
a) O,
b) CH₂, or
c) NR₈₇;
R₈₃ and R₈₄ at each occurrence are the same or different and are
a) H, or
b) C₁₋₄ alkyl;
R₈₅ is
a) OH,
b) C₁₋₄ alkoxy, or
c) -NR₈₈ R₈₉;
R₈₆ is
a) H, or
b) C₁₋₇ alkyl optionally substituted with indolyl, OH, mercaptyl, imidazoly, methylthio, amino, phenyl optionally substituted with OH, -C(=O)-NH₂, -CO₂H, or -C(=NH)-NH₂;
R₈₇ is
a) H,
b) phenyl, or
c) C₁₋₆ alkyl optionally substituted by OH;
R₈₈ and R₈₉ at each occurrence are the same or different and are
a) H,
b) C₁₋₅ alkyl
c) C₃₋₆ cycloalky, or
d) phenyl;
R₉₀ is
a) C₁₋₈ alkyl optionally substituted with C₁₋₆ alkoxy or hydroxy, C₃₋₆ cycloalkyl, a 6-membered aromatic optionally benzo-fused heterocyclic moiety having one to three nitrogen atoms, which can in turn be substituted with one or two -NO₂, CF₃, halo, -CN, OH, C₁₋₅ alkyl, C₁₋₅ alkoxy, or C₁₋₅ acyl;
b) 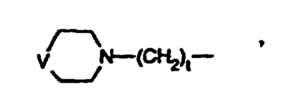
c) phenyl, or
d) pyridyl;
R₉₁ is
a) C₁₋₁₆ alkyl,
b) C₂₋₁₆ alkenyl,
wherein the substituents (a) and (b) can be optionally substituted with C₁₋₆ alkoxycarbonyl, or a 5-, 6-, 7-membered aromatic heterocyclic moiety having one to three atoms selected from the group consisting of S, N, and O,
c) an aromatic moiety having 6 to 10 carbon atoms, or
d) a 5-, 6-, 7-membered aromatic heterocyclic moiety having one to three atoms selected from the group consisting of S, N, and O,
wherein the substituents (c) and (d) can be optionally substituted with carboxyl, halo, -CN, formyl, CF₃, -NO₂, C₁₋₆ alkyl, C₁₋₆ alkoxy, C₁₋₆ acyl, C₁₋₆ alkylthio, or C₁₋₆ alkoxycarbonyl;
R₉₂ and R₉₃ at each occurrence are the same or different and are
a) H,
b) phenyl,
c) C₁₋₆ alkyl, or
d) benzyl;
R₉₄ and R₉₅ at each occurrence are the same or different and are
a) H,
b) OH,
c) C₁₋₆ alkyl optionally substituted with -NR₈₃ R₈₄, or
d) R₉₄ and R₉₅ taken together are =O;
R₉₆ is
a) an aromatic moiety having 6 to 10 carbon atoms,
b) a 5-, or 6-membered aromatic optionally benzo-fused heterocyclic moiety having one to three atoms selected from the group consisting of S, N, and O,
wherein the substituents (a) and (b) which can in turn be substituted with one or three -NO₂, CF₃, halo, -CN, OH, phenyl, C₁₋₅ alkyl, C₁₋₅ alkoxy, or C₁₋₅ acyl,
c) morpholinyl,
d) OH,
e) C₁₋₆ alkoxy,
f) -NR₈₃R₈₄,
g) -C(=O)-R₉₇, or
h) 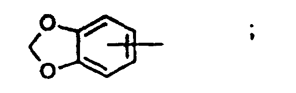
R₉₇ is
a) morpholinyl,
b) OH, or
c) C₁₋₆ alkoxy;
h is 1, 2, or 3;
i is 0, 1, or 2;
j is 0 or 1;
k is 3, 4, or 5;
l is 2 or 3;
m is 4 or 5;
n is 0, 1, 2, 3, 4, or 5;
p is 0, 1, 2, 3, 4, or 5; with the proviso that n and p together are 1, 2, 3, 4, or 5;
q is 1, 2, 3, or 4;
r is 2, 3, or 4;
t is 0, 1, 2, 3, 4, 5, or 6;
u is 1 or 2;
w is 0, 1, 2, or 3.

2. The use according to claim 1 wherein said mammal is a human.

3. The use according to claim 1 wherein the compound is administered in the range of about 0.1 to about 100 mg/kg of mammal body weight/day.

4. The use according to claim 1 wherein the compound is administered orally, nasally, parenterally, topically, transdermally, or rectally.

5. The use according to claim 1 wherein said compound is selected from the group consisting of :
(S)-trans-[[3-[3-Fluoro-4-(tetrahydro-1-oxido-2H-thiopyran-4-yl)phenyl]-2-oxo-5-oxazolidinyl]methyl]thiourea; and
(S)-N-[[3-[3-Fluoro-4-(4-thiomorpholinyl)phenyl]-2-oxo-5-oxazolidinyl]methyl]thioacetamide, thiomorpholine S-oxide; and
pharmaceutically acceptable salts thereof.

6. The use according to claim 1 wherein said mammal is not suffering from a bacterial infection.

7. The use of an effective amount of a compound, of formula II to prepare a medicament for treating or preventing osteoporosis, bone resorption or other bone disease in a vertebrate mammal, 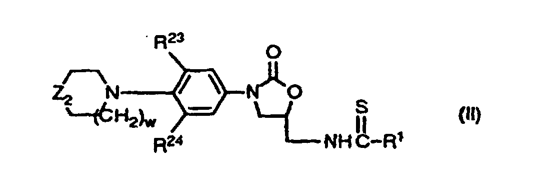 wherein Z₂ is -O₂S-, -O-, -N(R¹⁰⁷)-, -OS-, or -S-;
w is 0, 1, 2, or 3;
R²³ and R²⁴ are the same or different and can be H or F; and
R¹ is H, NH₂, NHalkylC₁-C₄; N(alkylC₁-C₄)₂; 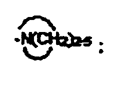 alkylC₁-C₄; OalkylC₁-C₄; SalkylC₁-C₄; alkylC₁-C₄ substituted with 1-3F, 1-2Cl, CN, or -COOalkylC₁-C₄, or cycloalkylC₃-C₆, wherein each occurrence of the alkyl group may be straight or branched; and
R¹⁰⁷ is
a) R¹⁰²O-C(R¹¹⁰)(R¹¹¹)-C(O)-,
b) R¹⁰³O-C(O)-,
c) R¹⁰⁸-C(O)-,
d) R¹⁰⁹-SO₂-,
e) NC-CH₂-,
f) E₂CHCH₂-, or
g) R¹⁵⁰R¹⁵¹NSO₂₋;
wherein R¹⁰² is H, CH₃-, phenyl-CH₂-, or CH₃C(O); each of R¹¹⁰ and R¹¹¹ is selected from H or CH₃; R¹⁰³ is alkylC₁-C₃ or phenyl; R¹⁰⁸ is H, alkylC₁-C₄, aryl(CH₂)₀₋₅, CNCH₂-, ClCH₂-, Cl₂HC-, FH₂C-, F₂HC-, or cycloalkylC₃-C₆; R¹⁵⁰ and R¹⁵¹ are the same or different and are selected from H, alkylC₁-C₄, or R¹⁵⁰ and R¹⁵¹ taken together with the nitrogen to which each is attached forms a monocyclic heterocyclic ring having from 3 to 6 carbon atoms.

8. The use according to claim 7 wherein said mammal is a human.

9. The use according to claim 7 wherein the compound is administered in the range of about 0.1 to about 100 mg/kg of mammal body weight/day.

10. The use according to claim 7 wherein the compound is administered orally, nasally, parenterally, topically, transdermally, or rectally.

11. The use according to claim 7 wherein said compound is selected from the group consisting of :
(S)-trans-[[3-[3-Fluoro-4-(tetrahydro-1-oxido-2H-thiopyran-4-yl)phenyl]-2-oxo-5-oxazolidinyl]methyl]thiourea; and
(S)-N-[[3-[3-Fluoro-4-(4-thiomorpholinyl)phenyl]-2-oxo-5-oxazolidinyl]methyl]thioacetamide, thiomorpholine S-oxide; and
pharmaceutically acceptable salts thereof.

12. The use according to claim 7 wherein said mammal is not suffering from a bacterial infection.

## Patentansprüche

1. Verwendung einer wirksamen Menge einer Verbindung der Formel I oder eines pharmazeutisch akzeptablen Salzes derselben zur Herstellung eines Medikaments zur Behandlung oder Prävention von Osteoporose, Knochenresorption oder einer anderen Knochenerkrankung bei einem Säugerwirbeltier, 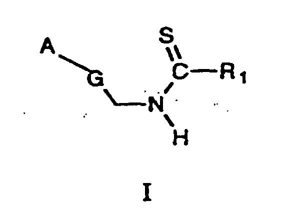 worin:
G 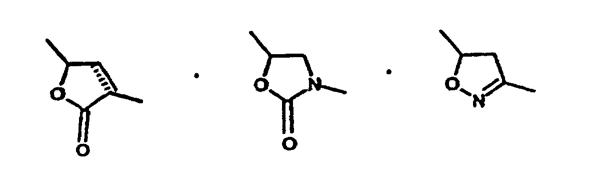 bedeutet;
R₁
a) H,
b) NH₂,
c) NH-C₁₋₄-Alkyl,
d) C₁₋₄-Alkyl,
e) -O-C₁₋₄-Alkyl,
f) -S-C₁₋₄-Alkyl,
g) C₁₋₄-Alkyl, das mit 1-3 F, 1-2 Cl, CN oder -COO-C₁₋₄-Alkyl substituiert ist,
h) C₃₋₆-Cycloalkyl,
i ) N(C₁₋₄-Alkyl)₂ oder
j ) N(CH₂)₂₋₅ bedeutet;
A
a) 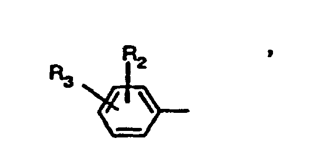
b) 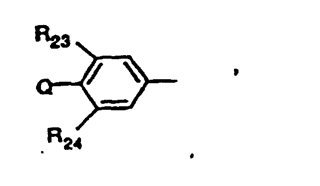
c) 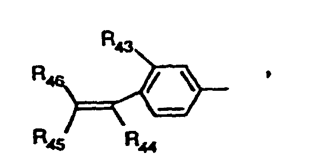
d) eine 5-gliedrige Heteroaromateneinheit, die ein bis drei aus der aus S, N und O bestehenden Gruppe ausgewählte Atome aufweist,
wobei die 5-gliedrige Heteroaromateneinheit über ein Kohlenstoffatom gebunden ist,
wobei die 5-gliedrige Heteroaromateneinheit ferner einen ankondensierten Benzol- oder Naphthylring aufweisen kann,
wobei die Heteroaromateneinheit optional mit einem bis drei Resten R₄₈ substituiert ist,
e) eine 6-gliedrige Heteroaromateneinheit, die mindestens ein Stickstoffatom aufweist,
wobei die Heteroaromateneinheit über ein Kohlenstoffatom gebunden ist,
wobei die 6-gliedrige Heteroaromateneinheit ferner einen ankondensierten Benzol- oder Naphthylring aufweisen kann,
wobei die Heteroaromateneinheit optional mit einem bis drei Resten R₅₅ substituiert ist,
f) ein β-Carbolin-3-yl oder Indolizinyl, das über den 6-gliedrigen Ring gebunden ist, das optional mit einem bis drei Resten R₅₅ substituiert ist,
g) 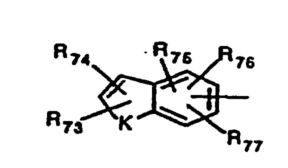 oder
n) 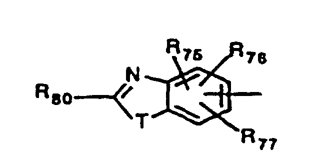 bedeutet;
wobei R₂
a) H,
b) F,
c) Cl,
d) Br,
e) C₁₋₃-Alkyl,
f) NO₂ bedeutet oder
g) R₂ und R₃ zusammengenommen -O-(CH₂)ₕ-O- bedeuten;
R₃
a) -S(=O)ᵢR₄,
b) -S (=O)₂-N=S(O)ⱼR₅R₆,
c) -SC(=O)R₇,
d) -C(=O)R₈,
e ) -C(=O)R₉,
f) -C(=O)NR₁₀R₁₁,
g) -C(=NR₁₂)R₈,
h) -C(R₈)(R₁₁)-OR₁₃,
i) -C(R₉)(R₁₁)-OR₁₃,
j) -C(R₈)(R₁₁)-OC(=O)R₁₃,
k) -C(R₉)(R₁₁)-OC(=O)R₁₃,
l) -NR₁₀R₁₁,
m) -N(R₁₀)-C(=O)R₇,
n) -N (R₁₀)-S (=O)ᵢR₇,
o) -C(OR₁₄)(OR₁₅)R₈,
p) -C(R₈)(R₁₆)-NR₁₀R₁₁ oder
q) C₁₋₈-Alkyl, das mit einem oder mehreren Resten =O mit Ausnahme an der α-Position, -S(=O)ᵢR₁₇, -NR₁₀R₁₁, C₂₋₅-Alkenyl oder C₂₋₅-Alkinyl substituiert ist, bedeutet;
R₄
a) C₁₋₄-Alkyl, das optional mit einem oder mehreren Halogenen, OH, CN, NR₁₀R₁₁ oder -CO₂R₁₃ substituiert ist,
b) C₂₋₄-Alkenyl,
c) -NR₁₆R₁₈,
d) -N₃,
e) -NHC (=O) R₇,
f) -NR₂₀C(=O)R₇,
g) -N(R₁₉)₂,
h) -NR₁₆R₁₉ oder
i) -NR₁₉R₂₀ bedeutet,
wobei R₅ und R₆ bei jedem Vorhandensein gleich oder verschieden sind und
a) C₁₋₂-Alkyl bedeuten oder
b) R₅ und R₆ zusammengenommen -(CH₂)ₖ- bedeuten;
R₇ C₁₋₄-Alkyl, das optional mit einem oder mehreren Halogenen substituiert ist, bedeutet;
R₈
a) H oder
b) C₁₋₈-Alkyl, das optional substituiert ist mit einem oder mehreren Halogenen, oder C₃₋₈-Cycloalkyl, bedeutet;
R₉ C₁₋₄-Alkyl, das substituiert ist mit einem oder mehreren Resten von
a) -S(=O)R₁₇,
b) -OR₁₃,
c) -OC(=O)R₁₃,
d) -NR₁₀R₁₁ oder
e) C₁₋₆-Alkenyl, das optional mit CHO substituiert ist, bedeutet;
R₁₀ und R₁₁ bei jedem Vorhandensein gleich oder verschieden sind und
a) H,
b) C₁₋₄-Alkyl oder
c) C₃₋₈-Cycloalkyl bedeuten;
R₁₂
a) -NR₁₀R₁₁,
b) -OR₁₀ oder
c) -NHC(=O)R₁₀ bedeutet;
R₁₃
a) H oder
b) C₁₋₄-Alkyl bedeutet;
R₁₄ und R₁₅ bei jedem Vorhandensein gleich oder verschieden sind und
a) C₁₋₄-Alkyl bedeuten, oder
b) R₁₄ und R₁₅ zusammengenommen -(CH)₁- bedeuten;
R₁₆
a) H,
b) C₁₋₄-Alkyl oder
c) C₃₋₈-Cycloalkyl bedeutet;
R₁₇
a) C₁₋₄-Alkyl oder
b) C₃₋₈-Cycloalkyl bedeutet;
R₁₈
a) H,
b) C₁₋₄-Alkyl,
c) C₂₋₄-Alkenyl,
d) C₃₋₄-Cycloalkyl,
e) -O₁₃ oder
f) -NR₂₁R₂₂ bedeutet;
R₁₉
a) Cl,
b) Br oder
c) I bedeutet;
R₂₀ ein physiologisch akzeptables Kation ist;
R₂₁ und R₂₂ bei jedem Vorhandensein gleich oder verschieden sind und
a) H,
b) C₁₋₄-Alkyl bedeuten oder
c) -NR₂₁R₂₂ zusammengenommen -(CH₂)ₘ- bedeuten;
wobei R₂₃ und R₂₄ bei jedem Vorhandensein gleich oder verschieden sind und
a) H,
b) F,
c) Cl,
d) C₁₋₂-Alkyl,
e) CN,
f) OH,
g) C₁₋₂-Alkoxy,
h) Nitro oder
i) Amino bedeuten;
Q
a) 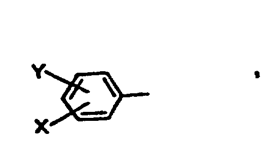
b) 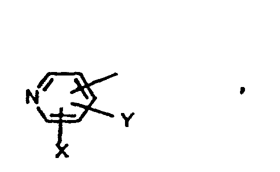
c) 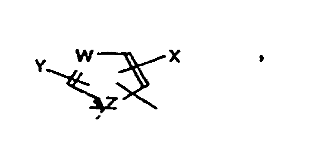
d) 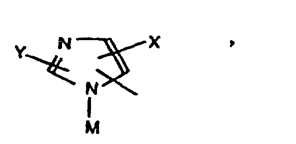
e) 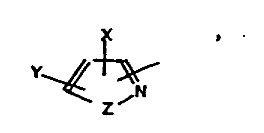
f) 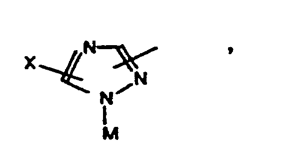
g) 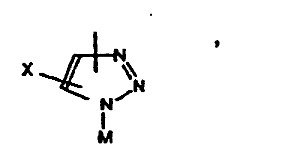
h) 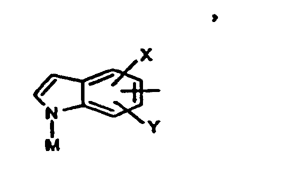
i) 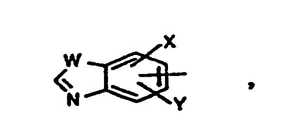
j) 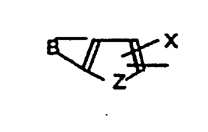
k) 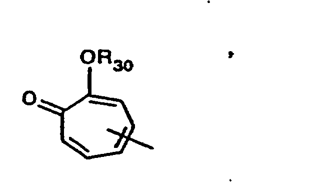
l) 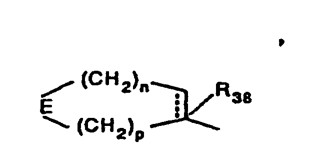
m) eine Diazinylgruppe, die optional mit X und Y substituiert ist,
n) eine Triazinylgruppe, die optional mit X und Y substituiert ist,
o) eine Chinolinylgruppe, die optional mit X und Y substituiert ist,
p) eine Chinoxalinylgruppe, die optional mit X und Y substituiert ist,
q) eine Naphthyridinylgruppe, die optional mit X und Y substituiert ist,
r) 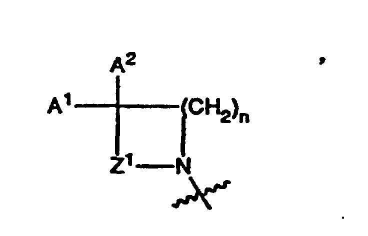
s) 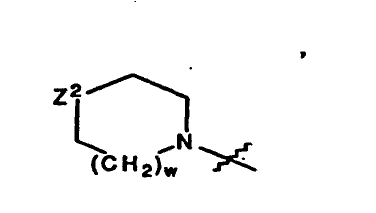
t) 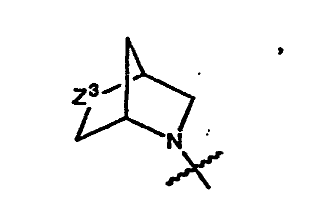
u) 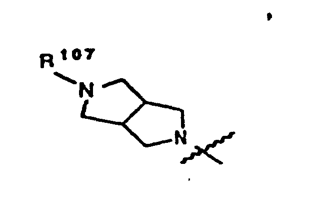
v) 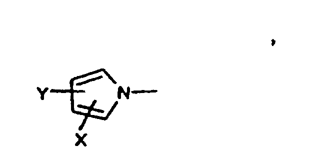
w) 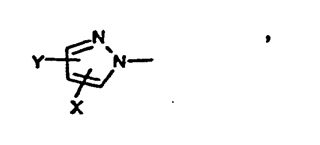
x) 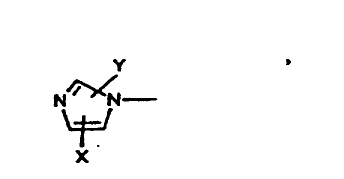
y) 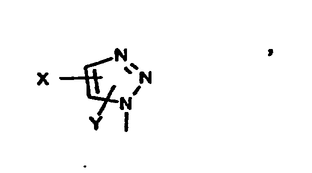
z) 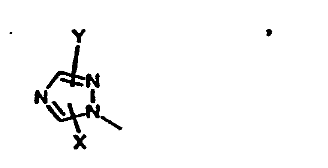
aa) 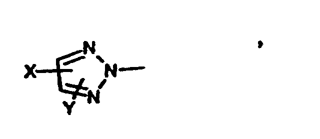
bb) 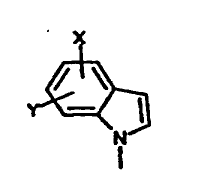 bedeutet oder
Q und R₂₄ zusammengenommen 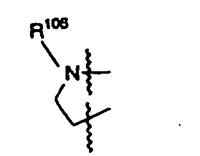 bedeuten,
wobei Z¹
a) -CH₂-,
b) -CH (R¹⁰⁴)-CH₂-,
c) -C(O)- oder
d) -CH₂CH₂CH₂- bedeutet;
wobei Z²
a) -O₂S-,
b) -O-,
c) -N(R¹⁰⁷)-,
d) -OS- oder
e) -S- bedeutet;
wobei Z³
a) -O₂S-,
b) -O-,
c) -OS- oder
d) -S- bedeutet;
wobei A¹
a) H- oder
b) CH₃ bedeutet;
wobei A²
a) H-,
b) HO-,
c) CH₃-,
d) CH₃O-,
e) R¹⁰²O-CH₂-C(O) -NH-,
f) R¹⁰³O-C(O)-NH-,
g) (C₁-C₂)Alkyl-O-C(O)-,
h) HO-CH₂-,
i) CH₃O-NH-,
j) (C₁-C₃)Alkyl-O₂C-,
k) CH₃-C(O)-,
l) CH₃-C(O)-CH₂-,
m) 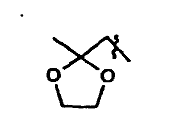 oder
n) 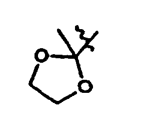 bedeutet,
A¹ und A² zusammengenommen
a) 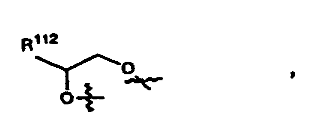
b) O= oder
c) 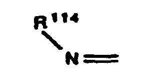 bedeuten;
wobei R¹⁰²
a) H-,
b) CH₃-,
c) Phenyl-CH₂- oder
d) CH₃C(O)- bedeutet;
wobei R¹⁰³
a) (C₁-C₃)Alkyl- oder
b) Phenyl- bedeutet;
wobei R¹⁰⁴
a) H- oder
b) HO- bedeutet;
wobei R¹⁰⁵
a) H-,
b) (C₁-C₃)Alkyl-,
c) CH₂=CH-CH₂- oder
d) CH₃-O-(CH₂)₂- bedeutet;
wobei R¹⁰⁶
a) CH₃-C(O)-,
b) H-C(O)-,
c) Cl₂CH-C(O)-,
d) HOCH₂-C(O)-,
e) CH₃SO₂-,
f) 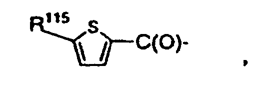
g) F₂CHC(O)-,
h) 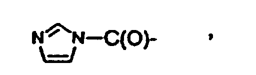
i ) H₃C-C(O)-O-CH₂-C(O)-,
j ) H-C(O)-O-CH₂-C(O)-,
k) 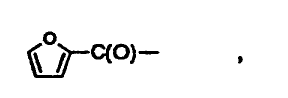
l) HC≡C-CH₂O-CH₂-C(O)- oder
m) Phenyl-CH₂-O-CH₂-C(O) - bedeutet;
wobei R¹⁰⁷
a) R¹⁰²O-C(R¹¹⁰)(R¹¹¹)-C(O)-,
b) R¹⁰³O-C(O)-
c) R¹⁰⁸-C(O)-
d) 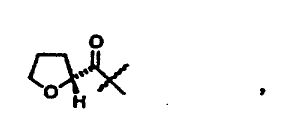
e) 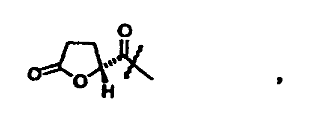
f) H₃C-C(O)-(CH₂)₂-C(O)-,
g) R¹⁰⁹O-SO₂-,
h) 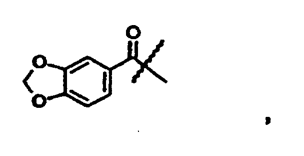
i ) HO-CH₂-C(O)-,
j) R¹¹⁶-(CH₂)₂-,
k) R¹¹³-C(O)-O-CH₂-C(O)-,
l) (CH₃)₂N-CH₂-C(O)-NH-,
m) NC-CH₂-,
n) F₂-CH-CH₂- oder
o) R¹⁵⁰R¹⁵¹NSO₂ bedeutet;
wobei R¹⁰⁸
a) H-,
b) (C₁-C₄)Alkyl,
c) Aryl-(CH₂)ₚ,
d) ClH₂C-,
e) Cl₂HC-,
f) FH₂C-,
g) F₂HC-,
h) (C₃-C₆)Cycloalkyl oder
i) CNCH₂- bedeutet;
wobei R¹⁰⁹
a) Alkyl-C₁-C₄,
b) -CH₂Cl,
c) -CH₂CH=CH₂,
d) Aryl oder
e) -CH₂CN bedeutet;
wobei R¹¹⁰ und R¹¹¹ unabhängig voneinander
a) H- oder
b) CH₃- bedeuten; oder
wobei R¹¹²
a) H-,
b) CH₃O-CH₂O-CH₂- oder
c) HOCH₂- bedeutet;
wobei R¹¹³
a) CH₃-,
b) HOCH₂-,
c) (CH₃)₂N-Phenyl oder
d) (CH₃)₂N-CH₂- bedeutet;
wobei R¹¹⁴
a) HO-,
b) CH₃O-,
c) H₂N-,
d) CH₃O-C(O)-O-,
e) CH₃-C(O)-O-CH₂-C(O)-O-,
f) Phenyl-CH₂-O-CH₂-C(O)-O-,
g) HO-(CH₂)₂-O-,
h) CH₃O-CH₂-O-(CH₂)₂-O- oder
i) CH₃O-CH₂-O- bedeutet;
wobei R¹¹⁵
a) H- oder
b) Cl- bedeutet;
wobei R¹¹⁶
a) HO-,
b) CH₃O- oder
c) F bedeutet;
wobei R¹⁵⁰ und R¹⁵¹ jeweils H oder Alkyl-C₁-C₄ bedeuten oder R¹⁵⁰ und R¹⁵¹ zusammen mit dem Stickstoffatom, an das jeder Rest gebunden ist, einen monocyclischen heterocyclischen Ring mit 3 bis 6 Kohlenstoffatomen bilden;
B eine ungesättigte Verbindungsgruppe mit 4 Atomen mit einem Stickstoff und 3 Kohlenstoffen ist;
M
a) H,
b) C₁₋₈-Alkyl,
c) C₃₋₈-Cycloalkyl,
d) -(CH₂)ₘOR₁₃ oder
e) -(CH₂)ₕNR₂₁R₂₂ bedeutet;
Z
a) O,
b) S oder
c) NM bedeutet;
W
a) CH,
b) N oder
c) S oder O bedeutet, wenn Z gleich NM ist;
Y
a) H,
b) F,
c) Cl,
d) Br,
e) C₁₋₃-Alkyl oder
f) NO₂ bedeutet;
X
a) H,
b) -CN,
c) OR₂₇,
d) Halogen,
e) NO₂,
f) Tetrazoyl,
g) -SH,
h) -S(=O)ᵢR₄,
i) -S (=O)₂-N=S(O) ⱼR₅R₆,
j) -SC(=O)R₇,
k) -C(=O)R₂₅,
l) -C(=O)NR₂₇R₂₈,
m) -C(=NR₂₉)R₂₅,
n) -C(R₂₅)(R₂₈)-OR₁₃,
o) -C (R₂₅)(R₂₈)-OC (=O)R₁₃,
p) -C(R₂₈)(OR₁₃)-(CH₂)ₕ-NR₂₇R₂₈,
q) -NR₂₇R₂₈,
r) -N (R₂₇)C (=O) R₇,
s) -N(R₂₇)-S(=O)ᵢR₇,
t) -C(OR₁₄)(OR₁₅)R₂₈,
u) -C (R₂₅)(R₁₆)-NR₂₇R₂₆ oder
v) C₁₋₈-Alkyl, das mit einem oder mehreren Halogenen, OH, =O mit Ausnahme an der α-Position, -S(=O)ᵢR₁₇, -NR₂₇R₂₈, C₂₋₅-Alkenyl, C₂₋₅-Alkinyl oder C₃₋₈-Cycloalkyl substituiert ist, bedeutet;
R₄, R₅, R₆, R₇, R₁₃, R₁₄, R₁₅, R₁₆ und R₁₇ wie im vorhergehenden definiert sind;
R₂₅
a) H,
b) C₁₋₈-Alkyl, das optional substituiert ist mit einem oder mehreren Halogenen, C₃₋₈-Cycloalkyl, C₁₋₄-Alkyl, das substituiert ist mit einem oder mehreren Resten von -S(=O)ᵢR₁₇, -OR₁₃ oder OC(=O)R₁₃, NR₂₇R₂₈, oder
c) C₂₋₅-Alkenyl, das optional mit CHO oder CO₂R₁₃ substituiert ist, bedeutet;
R₂₆
a) R₂₈ oder
b) NR₂₇N₂₈ bedeutet;
R₂₇ und R₂₈ bei jedem Vorhandensein gleich oder verschieden sind und
a) H,
b) C₁₋₈-Alkyl,
c) C₃₋₈-Cycloalkyl,
d) -(CH₂)ₘOR₁₃,
e) -(CH₂)ₕ-NR₂₁R₂₂ bedeuten oder
f) R₂₇ und R₂₈ zusammengenommen -(CH₂)₂O(CH₂)₂-, -C(CH₂)ₕCH(COR₇)- oder -(CH₂)₂N(CH₂)₂(R₇) bedeuten;
R₂₉
a) -NR₂₇R₂₈,
b) -OR₂₇ oder
c) -NHC(=O)R₂₈ bedeutet;
wobei R₃₀
a) H,
b) C₁₋₈-Alkyl, das optional mit einem oder mehreren Halogenen substituiert ist, oder
c) C₁₋₈-Alkyl, das optional mit einem oder mehreren OH oder C₁₋₆-Alkoxy substituiert ist, bedeutet;
wobei E
a) NR₃₉,
b) -S(=O)ᵢ oder
c) O bedeutet;
R₃₈
a) H,
b) C₁₋₆-Alkyl,
c) -(CH₂)_{q}-Aryl oder
d) Halogen bedeutet;
R₃₉
a) H,
b) C₁₋₆-Alkyl, das optional mit einem oder mehreren OH, Halogen oder -CN substituiert ist,
c) -(CH₂)_{q}-Aryl,
d) -CO₂R₄₀,
e) -COR₄₁,
f) -C(=O)-(CH₂)_{q}-C(=O)R₄₀,
g) -S(=O)₂-C₁₋₆-Alkyl,
h) -S(=O)₂-(CH₂)_{q}-Aryl oder
i) -(C=O)ⱼ-Het bedeutet;
R₄₀
a) H,
b) C₁₋₆-Alkyl, das optional mit einem oder mehreren OH, Halogen oder -CN substituiert ist,
c) -(CH₂)_{q}-Aryl oder
d) -(CH₂)_{q}-OR₄₂ bedeutet;
R₄₁
a) C₁₋₆-Alkyl, das optional mit einem oder mehreren OH, Halogen oder -CN substituiert ist,
b) -(CH₂)_{q}-Aryl oder
c) -(CH₂)_{q}-OR₄₂ bedeutet;
R₄₂
a) H,
b) C₁₋₆-Alkyl,
c) -(CH₂)_{q}-Aryl oder
d) -C(=O)-C₁₋₆-Alkyl bedeutet;
Aryl
a) Phenyl,
b) Pyridyl oder
c) Naphthyl bedeutet; wobei a bis c optional mit einem oder mehreren Resten Halogen, -CN, OH, SH, C₁₋₆-Alkyl, C₁₋₆-Alkoxy oder C₁₋₆-Alkylthio substituiert sind;
wobei R₄₃
a) H,
b) C₁₋₂-Alkyl,
c) F oder
d) OH bedeutet;
R₄₄
a) H,
b) CF₃,
c) C₁₋₃-Alkyl, das optional mit einem oder mehreren Halogenen substituiert ist,
d) Phenyl, das optional mit einem oder mehreren Halogenen substituiert ist, bedeutet,
e) R₄₄ und R₄₅ zusammengenommen einen 5-, 6- oder 7-gliedrigen Ring der Formel 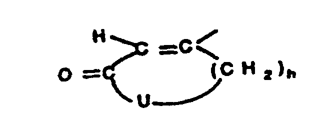 bedeuten, oder
f) R₄₄ und R₄₅ zusammengenommen -(CH₂)ₖ- bedeuten, wenn R₄₆ eine elektronenziehende Gruppe ist;
R₄₅ und R₄₆ bei jedem Vorhandensein gleich oder verschieden sind und
a) eine elektronenziehende Gruppe,
b) H,
c) CF₃,
d) C₁₋₃-Alkyl, das optional mit einem Halogen substituiert ist,
e) Phenyl, wobei mindestens einer der Reste R₄₅ oder R₄₆ eine elektronenziehende Gruppe ist, bedeuten, oder
f) R₄₅ und R₄₆ zusammengenommen einen 5-, 6- oder 7-gliedrigen Ring der Formel 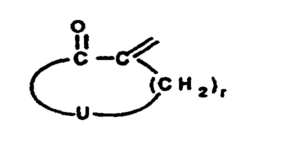 bedeuten;
U
a) CH₂,
b) O,
c) S oder
d) NR₄₇ bedeutet;
R₄₇
a) H oder
b) C₁₋₅-Alkyl bedeutet;
wobei R₄₈
a) Carboxyl,
b) Halogen,
c) -CN,
d) Mercapto,
e) Formyl,
f) CF₃,
g) -NO₂,
h) C₁₋₆-Alkoxy,
i) C₁₋₆-Alkoxycarbonyl,
j) C₁₋₆-Alkylthio,
k) C₁₋₆-Acyl,
l) -NR₄₉R₅₀,
m) C₁₋₆-Alkyl, das optional mit OH, C₁₋₅-Alkoxy, C₁₋₅-Acyl oder -NR₄₉R₅₀ substituiert ist,
n) C₂₋₈-Alkenylphenyl, das optional mit 1 oder 2 Resten R₅₁ substituiert ist,
o) Phenyl, das optional mit 1 oder 2 Resten R₅₁ substituiert ist,
p) eine 5- oder 6-gliedrige (un)gesättigte heterocyclische Einheit mit 1 bis 3 Atomen, die aus der aus S, N und O bestehenden Gruppe ausgewählt sind, die optional mit 1 oder 2 Resten R₅₁ substituiert ist, oder
q) 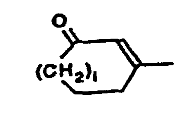 bedeutet;
R₄₉ und R₅₀ bei jedem Vorhandensein gleich oder verschieden sind und
a) H,
b) C₁₋₄-Alkyl,
c) C₅₋₆-Cycloalykl bedeuten, oder
d) R₄₉ und R₅₀ zusammengenommen mit dem Stickstoffatom eine 5-, 6-gliedrige gesättigte heterocyclische Einheit, die optional ein weiteres Heteroatom aufweist, das aus der aus S, N und O bestehenden Gruppe ausgewählt ist und wiederum optional mit C₁₋₃-Alkyl oder C₁₋₃-Acyl einschließlich am weiteren Stickstoffatom substituiert sein kann, bedeuten;
R₅₁
a) Carboxyl,
b) Halogen,
c) -CN,
d) Mercapto,
e) Formyl,
f) CF₃,
g) -NO₂,
h) C₁₋₆-Alkoxy,
i) C₁₋₆-Alkoxycarbonyl,
j) C₁₋₆-Alkylthio,
k) C₁₋₆-Acyl,
l) C₁₋₆-Alkyl, das optional mit OH, C₁₋₅-Alkoxy, C₁₋₅-Acyl oder -NR₄₉R₅₀ substituiert ist,
m) Phenyl,
n) -C(=O)NR₅₂R₅₃,
o) -NR₄₉R₅₀,
p) -N(R₅₂)(-SO₂R₅₄)
q) -SO₂-NR₅₂R₅₃ oder
r) -S(=O)ᵢR₅₄ bedeutet;
R₅₂ und R₅₃ bei jedem Vorhandensein gleich oder verschieden sind und
a) H,
b) C₁₋₆-Alkyl oder
c) Phenyl bedeuten;
R₅₄
a) C₁₋₄-Alkyl oder
b) Phenyl, das optional mit C₁₋₄-Alkyl substituiert ist, bedeutet;
wobei R₅₅
a) Carboxyl,
b) Halogen,
c) -CN,
d) Mercapto,
e) Formyl,
f) CF₃,
g) -NO₂,
h) C₁₋₆-Alkoxy,
i) C₁₋₆-Alkoxycarbonyl,
j) C₁₋₆-Alkylthio,
k) C₁₋₆-Acyl,
l) -NR₅₆R₅₇,
m) C₁₋₆-Alkyl, das optional mit OH, C₁₋₅-Alkoxy, C₁₋₅-Acyl oder -NR₅₆R₅₇ substituiert ist,
n) C₂₋₈-Alkenylphenyl, das optional mit einem oder zwei Resten R₅₈ substituiert ist,
o) Phenyl, das optional mit einem oder zwei Resten R₅₈ substituiert ist,
p) eine 5- oder 6-gliedrige (un)gesättigte heterocyclische Einheit mit 1 bis 3 Atomen, die aus der aus S, N und O bestehenden Gruppe ausgewählt sind, die optional mit einem oder zwei Resten R₅₈ substituiert ist, oder
q) 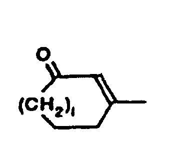 bedeutet;
R₅₆ und R₅₇ bei jedem Vorhandensein gleich oder verschieden sind und
a) H,
b) Formyl,
c) C₁₋₄-Alkyl,
d) C₁₋₄-Acyl,
e) Phenyl,
f) C₃₋₆-Cycloalkyl bedeuten, oder
g) R₅₆ und R₅₇ zusammengenommen mit dem Stickstoffatom eine 5-, 6-gliedrige gesättigte heterocyclische Einheit, die optional ein weiteres Heteroatom, das aus der aus S, N und O bestehenden Gruppe ausgewählt ist, aufweist und wiederum optional einschließlich am weiteren Stickstoffatom mit Phenyl, Pyrimidyl, C₁₋₃-Alkyl oder C₁₋₃-Acyl substituiert sein kann, bedeuten;
R₅₈
a) Carboxyl,
b) Halogen,
c) -CN,
d) Mercapto,
e) Formyl,
f) CF₃,
g) -NO₂,
h) C₁₋₆-Alkoxy,
i) C₁₋₆-Alkoxycarbonyl,
j) C₁₋₆-Alkylthio,
k) C₁₋₆-Acyl,
l) Phenyl,
m) C₁₋₆-Alkyl, das optional substituiert ist mit OH, Azido, C₁₋₅-Alkoxy, C₁₋₅-Acyl, -NR₆₅R₆₆, -SR₆₇, -O-SO₂R₆₈ oder 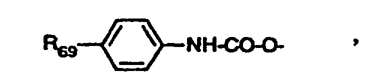
n) -C(=O)NR₅₉R₆₀,
o) -NR₅₆R₅₇,
p) -N (R₅₉)(SO₂R₅₄),
q) -SO₂-NR₅₉R₆₀,
r ) -S (=O)ᵢR₅₄,
s) -CH=N-R₆₁ oder
t) -CH(OH)-SO₃R₆₄ bedeutet;
R₅₄ wie im vorhergehenden definiert ist;
R₅₉ und R₆₀ bei jedem Vorhandensein gleich oder verschieden sind und
a) H,
b) C₁₋₆-Alkyl,
c) Phenyl oder
d) Tolyl bedeuten;
R₆₁
a) OH,
b) Benzyloxy,
c) -NH-C(=O)-NH₂,
d) -NH-C(=S)-NH₂ oder
e) -NH-C (=NH)-NR₆₂R₆₃ bedeutet;
R₆₂ und R₆₃ bei jedem Vorhandensein gleich oder verschieden sind und
a) H oder
b) C₁₋₄-Alkyl, das optional mit Phenyl oder Pyridyl substituiert ist, bedeuten;
R₆₄
a) H oder
b) ein Natriumion bedeutet;
R₆₅ und R₆₆ bei jedem Vorhandensein gleich oder verschieden sind und
a) H,
b) Formyl,
c) C₁₋₄-Alkyl,
d) C₁₋₄-Acyl,
e) Phenyl,
f) C₃₋₆-Cycloalkyl bedeuten,
g) R₆₅ und R₆₆ zusammengenommen eine 5-, 6-gliedrige gesättigte heterocyclische Einheit mit 1 bis 3 Atomen, die aus der aus S, N und O bestehenden Gruppe ausgewählt sind, die optional einschließlich am Stickstoffatom mit Phenyl, Pyrimidyl, C₁₋₃-Alkyl oder C₁₋₃-Acyl substituiert ist, bedeuten,
h) -P(O) (OR₇₀) (OR₇₁) oder
i) -SO₂-R₇₂ bedeuten;
R₆₇ 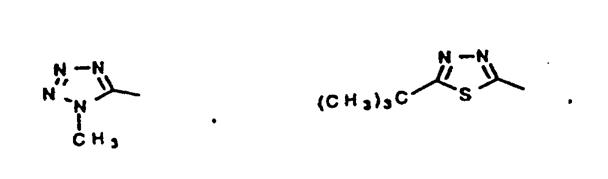 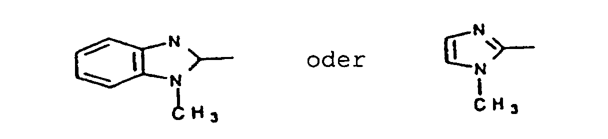 bedeutet;
R₆₈ C₁₋₃-Alkyl ist;
R₆₉
a) C₁₋₆-Alkoxycarbonyl oder
b) Carboxyl bedeutet;
R₇₀ und R₇₁ bei jedem Vorhandensein gleich oder verschieden sind und
a) H oder
b) C₁₋₃-Alkyl bedeuten;
R₇₂
a) Methyl,
b) Phenyl oder
c) Tolyl bedeutet;
wobei K
a) O oder
b) S bedeutet;
R₇₃, R₇₄, R₇₅, R₇₆ und R₇₇ bei jedem Vorhandensein gleich oder verschieden sind und
a) H,
b) Carboxyl,
c) Halogen,
d) -CN,
e) Mercapto,
f) Formyl,
g) CF₃,
h) -NO₂,
i) C₁₋₆-Alkoxy,
j) C₁₋₆-Alkoxycarbonyl,
k) C₁₋₆-Alkylthio,
l) C₁₋₆-Acyl,
m) -NR₇₈R₇₉,
n) C₁₋₆-Alkyl, das optional substituiert ist mit OH, C₁₋₅-Alkoxy, C₁₋₅-Acyl, -NR₇₈R₇₉, -N(Phenyl)(CH₂-CH₂-OH), -O-CH(CH₃)(OCH₂CH₃) oder -O-Phenyl-[para-NHC(=O)CH₃],
o) C₂₋₈-Alkenylphenyl, das optional mit R₅₁ substituiert ist,
p) Phenyl, das optional mit R₅₁ substituiert ist, oder
q) eine 5- oder 6-gliedrige (un)gesättigte heterocyclische Einheit mit 1 bis 3 Atomen, die aus der aus S, N und O bestehenden Gruppe ausgewählt sind, die optional mit R₅₁ substituiert ist, bedeuten;
R₅₁ wie im vorhergehenden definiert ist;
R₇₈ und R₇₉ bei jedem Vorhandensein gleich oder verschieden sind und
a) H,
b) C₁₋₄-Alkyl,
c) Phenyl bedeuten, oder
d) R₇₈ und R₇₉ zusammengenommen mit dem Stickstoff eine 5-, 6-gliedrige gesättigte heterocyclische Einheit, die optional ein weiteres Heteratom, das aus der aus S, N und O bestehenden Gruppe ausgewählt ist, aufweist und wiederum optional einschließlich am weiteren Stickstoffatom mit C₁₋₃-Alkyl oder C₁₋₃-Acyl substituiert sein kann, bedeuten;
wobei T
a) 0,
b) S oder
c) SO₂ bedeutet;
R₇₅, R₇₆ und R₇₇ wie im vorhergehenden definiert sind;
R₈₀
a) H,
b) Formyl,
c) Carboxyl,
d) C₁₋₆-Alkoxycarbonyl,
e) C₁₋₈-Alkyl,
f) C₂₋₈-Alkenyl,
wobei die Substituenten (e) und (f) optional mit OH, Halogen, C₁₋₆-Alkoxy, C₁₋₆-Acyl, C₁₋₆-Alkylthio oder C₁₋₆-Alkoxycarbonyl oder einem mit Halogen optional substituierten Phenyl substituiert sein können,
g) eine aromatische Einheit mit 6 bis 10 Kohlenstoffatomen, die optional mit Carboxyl, Halogen, -CN, Formyl, CF₃, -NO₂, C₁₋₆-Alkyl, C₁₋₆-Alkoxy, C₁₋₆-Acyl, C₁₋₆-Alkylthio oder C₁₋₆-Alkoxycarbonyl substituiert ist,
h) -NR₈₁R₈₂,
i) -OR₉₀,
j) -S(=O)ᵢ-R₉₁,
k) -SO₂-N(R₉₂)(R₉₃) oder
l) einen Rest der im folgenden angegebenen Formeln bedeutet;
R₈₁ und R₈₂ bei jedem Vorhandensein gleich oder verschieden sind und
a) H,
b) C₃₋₆-Cycloalkyl,
c) Phenyl,
d) C₁₋₆-Acyl,
e) C₁₋₈-Alkyl, das optional substituiert ist mit OH, C₁₋₆-Alkoxy, das mit OH substituiert sein kann, einer 5- oder 6-gliedrigen aromatischen heterocyclischen Einheit mit 1 bis 3 Atomen, die aus der aus S, N und 0 bestehenden Gruppe ausgewählt sind, Phenyl, das optional substituiert ist mit OH, CF₃, Halogen, -NO₂, C₁₋₄-Alkoxy, -NR₈₃R₈₄ oder 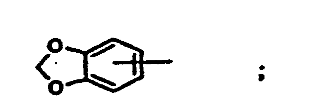
f) 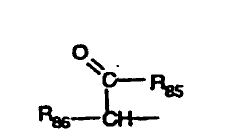 oder
g) 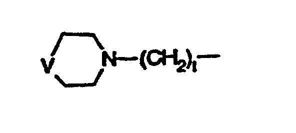 bedeuten;
V
a) O,
b) CH₂ oder
c) NR₈₇ bedeutet;
R₈₃ und R₈₄ bei jedem Vorhandensein gleich oder verschieden sind und
a) H oder
b) C₁₋₄-Alkyl bedeuten;
R₈₅
a) OH,
b) C₁₋₄-Alkoxy oder
c) -NR₈₈R₈₉ bedeutet;
R₈₆
a) H oder
b) C₁₋₇-Alkyl, das optional substituiert ist mit Indolyl, OH, Mercaptyl, Imidazolyl, Methylthio, Amino, Phenyl, das optional substituiert ist mit OH, -C(=O)-NH₂, -CO₂H oder -C(=NH)-NH₂, bedeutet;
R₈₇
a) H,
b) Phenyl oder
c) C₁₋₆-Alkyl, das optional mit OH substituiert ist, bedeutet;
R₈₈ und R₈₉ bei jedem Vorhandensein gleich oder verschieden sind und
a) H,
b) C₁₋₅-Alkyl,
c) C₃₋₆-Cycloalkyl oder
d) Phenyl bedeuten;
R₉₀
a) C₁₋₈-Alkyl, das optional substituiert ist mit C₁₋₆-Alkoxy oder C₁₋₆-Hydroxy, C₃₋₆-Cycloalkyl, einer 6-gliedrigen aromatischen optional benzokondensierten heterocyclischen Einheit mit einem bis drei Stickstoffatomen, die wiederum mit einem oder zwei -NO₂, CF₃, Halogen, -CN, OH, C₁₋₅-Alkyl, C₁₋₅-Alkoxy oder C₁₋₅-Acyl substituiert sein kann,
b) 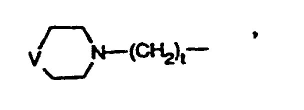
c) Phenyl oder
d) Pyridyl bedeutet;
R₉₁
a) C₁₋₁₆-Alkyl,
b) C₂₋₁₆-Alkenyl,
wobei die Substituenten (a) und (b) optional mit C₁₋₆-Alkoxycarbonyl oder einer 5-, 6-, 7-gliedrigen aromatischen heterocyclischen Einheit mit einem bis drei Atomen, die aus der aus S, N und O bestehenden Gruppe ausgewählt sind, substituiert sein können,
c) eine aromatische Einheit mit 6 bis 10 Kohlenstoffatomen oder
d) eine 5-, 6-, 7-gliedrige aromatische heterocyclische Einheit mit einem bis drei Atomen, die aus der aus S, N und O bestehenden Gruppe ausgewählt sind, wobei die Substituenten (c) und (d) optional mit Carboxyl, Halogen, - CN, Formyl, CF₃, -NO₂, C₁₋₆-Alkyl, C₁₋₆-Alkoxy, C₁₋₆-Acyl, C₁₋₆-Alkylthio oder C₁₋₆-Alkoxycarbonyl substituiert sein können, bedeutet;
R₉₂ und R₉₃ bei jedem Vorhandensein gleich oder verschieden sind und
a) H,
b) Phenyl,
c) C₁₋₆-Alkyl oder
d) Benzyl bedeuten;
R₉₄ und R₉₅ bei jedem Vorhandensein gleich oder verschieden sind und
a) H,
b) OH,
c) C₁₋₆-Alkyl, das optional mit -NR₈₃R₈₄ substituiert ist, bedeuten oder
d) R₉₄ und R₉₅ zusammengenommen =O bedeuten;
R₉₆
a) eine aromatische Einheit mit 6 bis 10 Kohlenstoffatomen,
b) eine 5- oder 6-gliedrige aromatische optional benzokondensierte heterocyclische Einheit mit einem bis drei Atomen, die aus der aus S, N und O bestehenden Gruppe ausgewählt sind,
wobei die Substituenten (a) und (b) wiederum mit einem oder drei Resten -NO₂, CF₃, Halogen, -CN, OH, Phenyl, C₁₋₅-Alkyl, C₁₋₅-Alkoxy oder C₁₋₅-Acyl substituiert sein können,
c) Morpholinyl,
d) OH,
e) C₁₋₆-Alkoxy,
f) -NR₈₃R₈₄,
g) -C(=O)-R₉₇ oder
h) 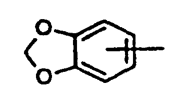 bedeutet;
R₉₇
a) Morpholinyl,
b) OH oder
c) C₁₋₆-Alkoxy bedeutet;
h 1, 2 oder 3 bedeutet;
i 0, 1 oder 2 bedeutet;
j 0 oder 1 bedeutet;
k 3, 4 oder 5 bedeutet;
l 2 oder 3 bedeutet;
m 4 oder 5 bedeutet;
n 0, 1, 2, 3, 4 oder 5 bedeutet;
p 0, 1, 2, 3, 4 oder 5 bedeutet; wobei n und p zusammen 1, 2, 3, 4 oder 5 sind;
q 1, 2, 3 oder 4 bedeutet;
r 2, 3 oder 4 bedeutet;
t 0, 1, 2, 3, 4, 5 oder 6 bedeutet;
u 1 oder 2 bedeutet;
w 0, 1, 2 oder 3 bedeutet.

2. Verwendung nach Anspruch 1, wobei der Säuger ein Mensch ist.

3. Verwendung nach Anspruch 1, wobei die Verbindung in einem Bereich von etwa 0,1 bis etwa 100 mg/kg Körpergewicht des Säugers/Tag verabreicht wird.

4. Verwendung nach Anspruch 1, wobei die Verbindung oral, nasal, parenteral, topisch, transdermal oder rektal verabreicht wird.

5. Verwendung nach Anspruch 1, wobei die Verbindung aus der Gruppe von:
(S)-trans-[[3-[3-Fluor-4-(tetrahydro-1-oxido-2Hthiopyran-4-yl)phenyl]-2-oxo-5-oxazolidinyl]methyl]thioharnstoff und
(S)-N-[[3-[3-Fluor-4-(4-thiomorpholinyl)phenyl]-2-oxo-5-oxazolidinyl]methyl]thioacetamid-thiomorpholin-Soxid
und pharmazeutisch akzeptablen Salzen derselben ausgewählt ist.

6. Verwendung nach Anspruch 1, wobei der Säuger nicht an einer antibakteriellen Infektion leidet.

7. Verwendung einer wirksamen Menge einer Verbindung der Formel II zur Herstellung eines Medikaments zur Behandlung oder Prävention von Osteoporose, Knochenresorption oder einer anderen Knochenerkrankung bei einem Säugerwirbeltier, 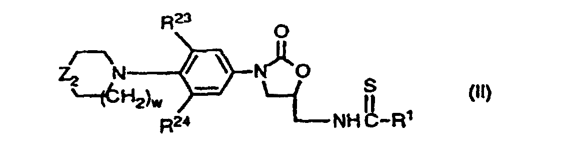 worin Z₂ -O₂S-, -O-, -N(R¹⁰⁷)-, -OS- oder -S- bedeutet; w 0, 1, 2 oder 3 bedeutet;
R²³ und R²⁴ gleich oder verschieden sind und H oder F sein können; und
R¹ H, NH₂, NH-Alkyl-C₁-C₄, N(Alkyl-C₁-C₄)₂,
Alkyl-C₁-C₄, O-Alkyl-C₁-C₄, S-Alkyl-C₁-C₄, Alkyl-C₁-C₄, das substituiert ist mit 1-3 F, 1-2 Cl, CN oder -COO-Alkyl-C₁-C₄, oder Cycloalkyl-C₃-C₆, wobei bei jedem Vorhandensein der Alkylgruppe diese gerade oder verzweigt sein kann, bedeutet; und
R¹⁰⁷
a) R¹⁰²O-C(R¹¹⁰)(R¹¹¹)-C(O)-,
b) R¹⁰³O-C(O)-,
c) R¹⁰⁸-C(O)-,
d) R¹⁰⁹-SO₂-,
e) NC-CH₂-,
f) F₂CHCH₂- oder
g) R¹⁵⁰R¹⁵¹NSO₂- bedeutet;
wobei R¹⁰² H, CH₃-, Phenyl-CH₂- oder CH₃C(O) bedeutet; jeder Rest von R¹¹⁰ und R¹¹¹ aus H oder CH₃ ausgewählt ist;
R¹⁰³ Alkyl-C₁-C₃ oder Phenyl bedeutet;
R¹⁰⁸ H, Alkyl-C₁-C₄, Aryl(CH₂)₀₋₅, CNCH₂-, ClCH₂-, Cl₂HC-, FH₂C-, F₂HC- oder Cycloalkyl-C₃-C₆ bedeutet;
R¹⁵⁰ und R¹⁵¹ gleich oder verschieden sind und aus H, Alkyl-C₁-C₄ ausgewählt sind oder
R¹⁵⁰ und R¹⁵¹ zusammen mit dem Stickstoff, an den jeder Rest gebunden ist, einen monocyclischen heterocyclischen Ring mit 3 bis 6 Kohlenstoffatomen bilden.

8. Verwendung nach Anspruch 7, wobei der Säuger ein Mensch ist.

9. Verwendung nach Anspruch 7, wobei die Verbindung in einem Bereich von etwa 0,1 bis etwa 100 mg/kg Körpergewicht des Säugers/Tag verabreicht wird.

10. Verwendung nach Anspruch 7, wobei die Verbindung oral, nasal, parenteral, topisch, transdermal oder rektal verabreicht wird.

11. Verwendung nach Anspruch 7, wobei die Verbindung aus der Gruppe von:
(S)-trans-[[3-[3-Fluor-4-(tetrahydro-1-oxido-2Hthiopyran-4-yl)phenyl]-2-oxo-5-oxazolidinyl]methyl]thioharnstoff und
(S)-N-[[3-[3-Fluor-4-(4-thiomorpholinyl)phenyl]-2-oxo-5-oxazolidinyl]methyl]thioacetamid-thiomorpholin-Soxid
und pharmazeutisch akzeptablen Salzen derselben ausgewählt ist.

12. Verwendung nach Anspruch 7, wobei der Säuger nicht an einer antibakteriellen Infektion leidet.

## Revendications

1. Utilisation d'une quantité efficace d'un composé de formule I ou de ses sels pharmaceutiquement acceptables pour préparer un médicament destiné au traitement ou la prévention de l'ostéoporose, de la résorption osseuse ou d'une autre maladie osseuse chez un mammifère vertébré, 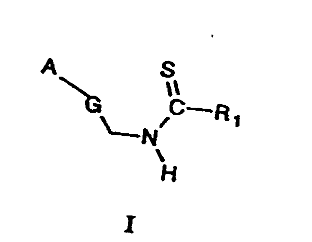 formule dans laquelle ;
G représente un groupe 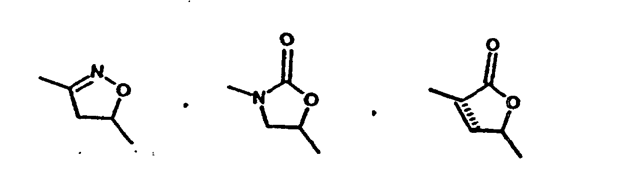
R₁ représente un groupe
a) H,
b) NH₂,
c) NH- (alkyle en C₁ à C₄),
d) alkyle en C₁ à C₄,
e) -O(alkyle en C₁ à C₄),
f) -S(alkyle en C₁ à C₄),
g) alkyle en C₁ à C₄ substitué avec 1 à 3 substituants F, 1 à 2 substituants Cl, CN ou COO(alkyle en C₁ à C₄),
h) cycloalkyle en C₃ à C₆,
i) N(alkyle en C₁ à C₄)₂, ou
j) N(CH₂)₂₋₅ ;
A représente un groupe
a) 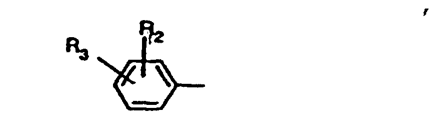
b) 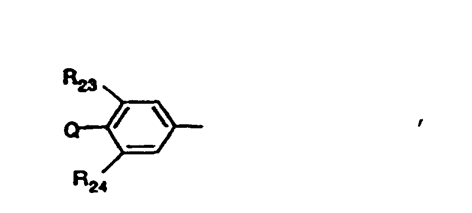
c) 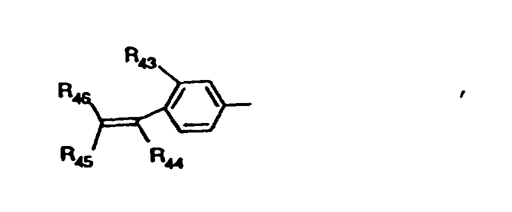
d) un groupement hétéroaromatique pentagonal comprenant un à trois atomes choisis dans le groupe consistant en S, N et O,
ledit groupement hétéroaromatique hexagonal étant lié par un atome de carbone,
ledit groupement hétéroaromatique hexagonal pouvant comprendre en outre un noyau benzène ou naphtyle condensé,
ledit groupement hétéroaromatique étant facultativement substitué avec un à trois substituants R₄₈,
e) un groupement hétéroaromatique hexagonal comprenant au moins un atome d'azote, ledit groupement hétéroaromatique étant lié par un atome de carbone,
ledit groupement hétéroaromatique hexagonal pouvant comprendre en outre un noyau benzène ou naphtyle condensé,
ledit groupement hétéroaromatique étant facultativement substitué avec un à trois substituants R₅₅,
f) un groupe β-carbolin-3-yle, ou indoizinyle lié par le noyau hexagonal, facultativement substitué avec un à trois substituants R₅₅,
g) 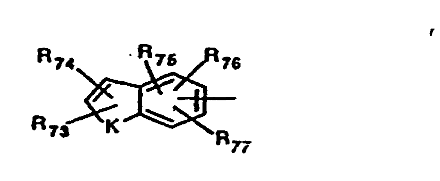 ou
h) 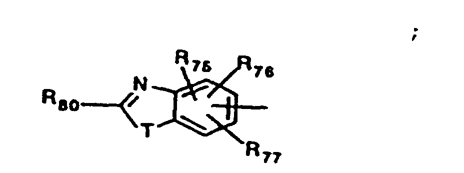
où R₂ représente
a) H
b) F,
c) Cl,
d) Br,
e) alkyle en C₁ à C₃,
f) NO₂, ou
g) R₂ et R₃, pris conjointement, représentent un groupe -O-(CH₂)ₕ-O- ;
R₃ représente un groupe
a) -S(=O)ᵢ R₄,
b) -S (=O)₂-N=S(O)ⱼR₅R₆,
c) -SC(=O)R₇,
d) -C(=O)R₈,
e) -C(=O)R₉,
f) -C(=O)NR₁₀R₁₁,
g) -C(=NR₁₂)R₈,
h) -C (R₈)(R₁₁)-OR₁₃,
i) -C(R₉)(R₁₁)-OR₁₃,
j) -C(R₈)(R₁₁)-OC(=O)R₁₃,
k) -C(R₉)(R₁₁)-OC(=O)R₁₃,
l) -NR₁₀R₁₁,
m) -N(R₁₀)-C(=O)R₇,
n) -N(R₁₀)-S(=O)R₇,
o) -C(OR₁₄)(OR₁₅)R₈,
p) -C(R₈)(R₁₆)-NR₁₀R₁₁, ou
q) alkyle en C₁ à C₈ substitué avec un ou plusieurs substituants =O à des positions autres qu'une position alpha, -S(=O)ᵢR₁₇, -NR₁₀R₁₁, alcényle en C₂ à C₅ ou alcynyle en C₂ à C₅ ;
R₄ représente un groupe
a) alkyle en C₁ à C₄ facultativement substitué avec un ou plusieurs substituants halogéno, OH, CN, NR₁₀R₁₁, ou -CO₂R₁₃,
b) alcényle en C₂ à C₄,
c) -NR₁₆R₁₈,
d) -N₃,
e) -NHC(=O)R₇,
f) -NR₂₀C(=O)R₇,
g) -N(R₁₉)₂,
h) -NR₁₆R₁₉, ou
i) -NR₁₉R₂₀,
R₅ et R₆, à chaque emplacement où ils sont présents, sont identiques ou différents et représentent
a) un groupe alkyle en C₁ ou C₂, ou
b) R₅ et R₆, pris conjointement, représentent un groupe -(CH₂)ₖ- ;
R₇ représente un groupe alkyle en C₁ à C₄ facultativement substitué avec un ou plusieurs substituants halogéno ;
R₈ représente un groupe
a) H, ou
b) alkyle en C₁ à C₈ facultativement substitué avec un plusieurs substituants halogéno, ou cycloalkyle en C₃ à C₈ ;
R₉ représente un groupe alkyle en C₁ à C₄ substitué avec ou plusieurs substituants
a) -S(=O)R₁₇,
b) -OR₁₃,
c) -OC(=O)R₁₃,
d) -NR₁₀R₁₁, ou
e) alcényle en C₁ à C₅ facultativement substitué avec un substituant CHO ;
R₁₀ et R₁₁, à chaque emplacement où ils sont présents, sont identiques ou différents et représentent un groupe
a) H,
b) alkyle en C₁ à C₄, ou
c) cycloalkyle en C₃ à C₈ ;
R₁₂ représente un groupe
a) -NR₁₀R₁₁,
b) -OR₁₀ ; ou
c) -NHC(=O)R₁₀ ;
R₁₃ représente un groupe
a) -H, ou
b) -alkyle en C₁ à C₄ ;
R₁₄ et R₁₅, à chaque emplacement où ils sont présents, sont identiques ou différents et représentent un groupe
a) alkyle en C₁ à C₄, ou
b) R₁₄ et R₁₅, pris conjointement, représentent un groupe -(CH)₁- ;
a) H,
b) alkyle en C₁ à C₄, ou
c) cycloalkyle en C₃ à C₈ ;
R₁₇ représente un groupe
a) alkyle en C₁ à C₄, ou
b) cycloalkyle en C₃ à C₈ ;
R₁₈ représente un groupe
a) H,
b) alkyle en C₁ à C₄,
c) alcényle en C₂ à C₄,
d) cycloalkyle en C₃ ou C₄,
e) -OR₁₃, ou
f) -NR₂₁R₂₂ ;
R₁₉ représente un groupe
a) Cl,
b) Br, ou
c) I ;
R₂₀ représente un cation physiologiquement acceptable ;
R₂₁ et R₂₂, à chaque emplacement où ils sont présents, sont identiques ou différents et représentent un groupe
a) H,
b) alkyle en C₁ à C₄, ou
c) pris conjointement, un groupe -NR₂₁R₂₂ représentant un groupe -(CH₂)ₘ- ;
où R₂₃ et R₂₄, à chaque emplacement où ils sont présents sont identiques ou différents et représentent un groupe
a) H,
b) F,
c) Cl;
d) alkyle en C₁ ou C₂,
e) CN,
f) OH,
g) alkoxy en C₁ ou C₂,
h) nitro, ou
i) amino ;
Q représente un groupe
a) 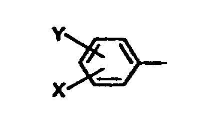
b) 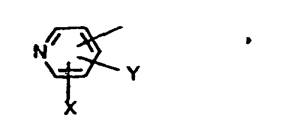
c) 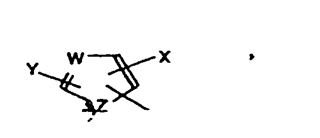
d) 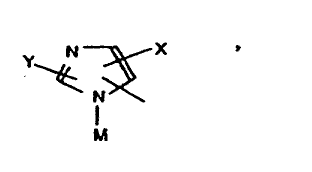
e) 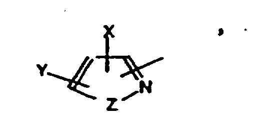
f) 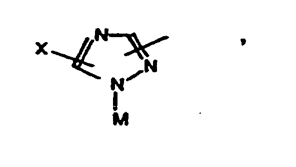
g) 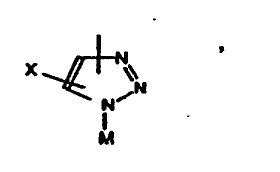
h) 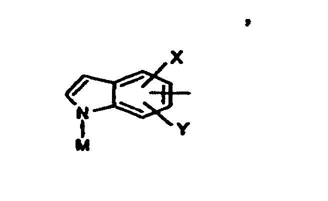
i) 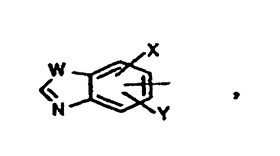
j) 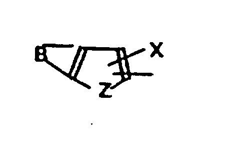
k) 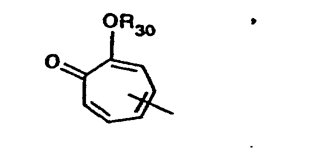
l) 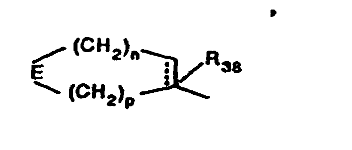
m) un groupe diazinyle facultativement substitué avec des substituants X, Y,
n) un groupe triazinyle facultativement substitué avec des substituants X et Y,
o) un groupe quinolinyle facultativement substitué avec des substituants X et Y,
p) un groupe quinoxalinyle facultativement substitué avec des substituants X et Y,
q) un groupe naphthyridinyle facultativement substitué avec des substituants X et Y,
r) 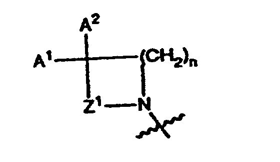
s) 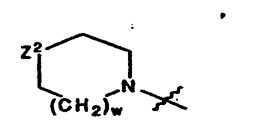
t) 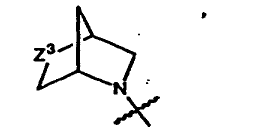
u) 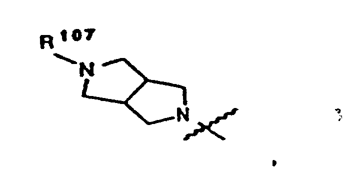
v) 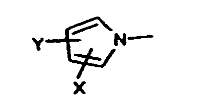
w) 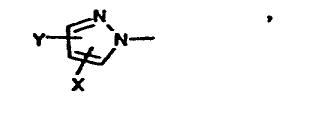
x) 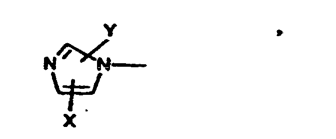
y) 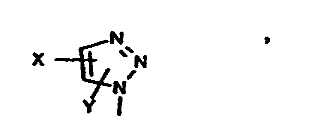
z) 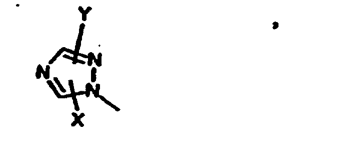
aa) 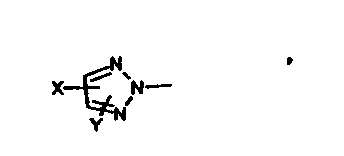
bb) 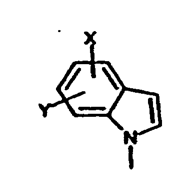
ou,
Q et R₂₄, pris conjointement, représentent un groupe 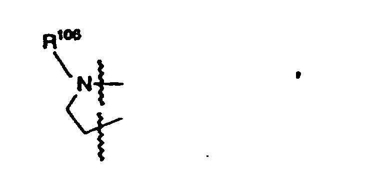
Z¹ représente un groupe
a) -CH₂-,
b) -CH(R¹⁰⁴)-CH₂-,
c) -C(O)- ou
d) -CH₂CH₂CH₂- ;
Z² représente un groupe
a) -O₂S-,
b) -O-,
c) -N(R¹⁰⁷)-,
d) -OS-, ou
e) -S- ;
Z³ représente un groupe
a) -O₂S-,
b) -O-,
c) -OS-, ou
d) -S- ;
A¹ représente un groupe
a) H-, ou
b) CH₃ ;
A² représente un groupe
a) H-,
b) HO-,
c) CH₃,
d) CH₃ O-,
e) R¹⁰²O-CH₂-C(O)-NH-
f) R¹⁰³O-C(O)-NH-,
g) (alkyle en C₁ ou C₂)-O-C(O)-,
h) HO-CH₂-,
i) CH₃O-NH-,
j) (alkyle en C₁ à C₃)-O₂C-,
k) CH₃-C(O)-,
l) CH₃-C(O)-CH₂-,
m) 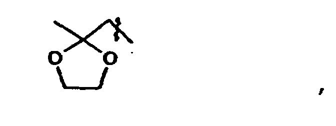 ou
n) 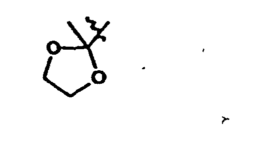
A¹ et A², pris conjointement, représentent un groupe :
a) 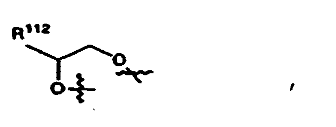
b) o= , ou
c) 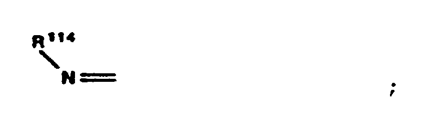
où R¹⁰² représente un groupe
a) H-,
b) CH3-,
c) phényl-CH₂-, ou
d) CH₃C(O)- ;
où R¹⁰³ représente un groupe
a) alkyle en C₁ à C₃-, ou
b) phényle- ;
où R¹⁰⁴ représente un groupe
a) H-, ou
b) HO- ;
où R¹⁰⁵ représente un groupe
a) H-,
b) alkyle en C₁ à C₃,
c) CH₂=CH-CH₂-, ou
d) CH₃-O-(CH₂)₂- ;
où R¹⁰⁶ représente un groupe
a) CH₃C(O)- ,
b) H-C(O)-,
c) Cl₂CH-C(O)-,
d) HOCH₂-C(O)-,
e) CH₃SO₂-,
f) 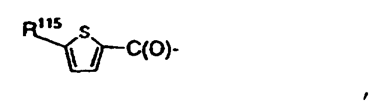
g) F₂CHC(O)-,
h) 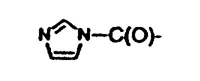
i) H₃C-C(O)-O-CH₂-C(O)-,
j ) H-C(O)-O-CH₂-C(O)-,
k) 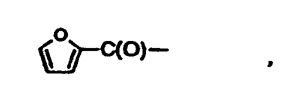
l) HC≡C-CH₂O-CH₂-C(O)-, ou
m) phényl-CH₂-O-CH₂-C(O)- ;
où R¹⁰⁷ représente un groupe
a) R¹⁰²O-C(R¹¹⁰)(R¹¹¹)-C(O)-,
b) R¹⁰³O-C(O)-,
c) R¹⁰⁶-C(O)-,
d) 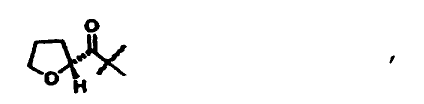
e) 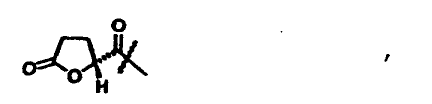
f) H₃C-C(O)-(CH₂)₂-C(O)-,
g) R¹⁰⁹-SO₂-,
h) 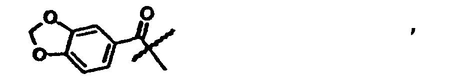
i) HO-CH₂-C(O)-,
j) R¹¹⁶-(CH₂)₂-,
k) R¹¹³-C(O)-O-CH₂-C(O)-,
l) (CH₃)₂N-CH₂-C(O)-NH
m) NC-CH₂-,
n) F₂-CH-CH₂-, ou
o) R¹⁵⁰R¹⁵¹NSO₂
où R¹⁰⁸ représente un groupe
a) H-,
b) alkyle en C₁ à C₄,
c) aryl-(CH₂)ₚ,
d) ClH₂C-,
e) Cl₂HC-,
f) FH₂C-,
g) F₂HC-,
h) cycloalkyle en C₃ à C₆, ou
i) CNCH₂-
où R¹⁰⁹ représente un groupe
a) alkyle en C₁ à C₄,
b) -CH₂Cl,
c) -CH₂CH=-CH₂,
d) aryle, ou
e) -CH₂CN ;
où R¹¹⁰ et R¹¹¹ représentent indépendamment un groupe
a) H-,
b) CH₃- ; ou
où R¹¹² représente un groupe
a) H-,
b) -CH₃O--CH₂O--CH₂-, ou
c) HO-CH₂- ;
où R¹¹³ représente un groupe
a) CH₃-,
b) HOCH₂-
c) (CH₃)₂N-phényle, ou
d) (CH₃)₂N-CH₂- ;
où R¹¹⁴ représente un groupe
a) HO-,
b) CH₃O-,
c) H₂N-,
d) CH₃O-C(O)-O-,
e) CH₃O-C(O)-O-CH₂-C(O)-O-,
f) phényl-CH₂-O-CH₂-C(O)-O-,
g) HO-(CH₂)₂-O-,
h) CH₃-O-CH₂-O-(CH₂)₂-O-, ou
i) CH₃O-CH₂-O- ; où R¹¹³ représente un groupe
a) CH₃-,
b) HOCH₂-,
c) (CH₃)₂N-phényl, ou
d) (CH₃)₂N-CH₂- ;
R¹¹⁵ représente un groupe
a) H-, ou
b) Cl- ;
R¹¹⁶ représente un groupe
a) HO-
b) CH₃-O-, ou
c) F ;
R¹⁵⁰ et R¹⁵¹ représentent chacun H ou un groupe alkyle en C₁ à C₄, ou bien R¹⁵⁰ et R¹⁵¹, pris conjointement avec l'atome d'azote auquel chacun est fixé, forment un noyau hétérocyclique monocyclique ayant 3 à 6 atomes de carbone ;
B représente un groupe de liaison insaturé à 4 atomes comprenant un atome d'azote et trois atomes de carbone ;
M représente un groupe
a) H-,
b) alkyle en C₁ à C₈,
c) cycloalkyle en C₃ à C₈,
d) - (CH₂)ₘOR₁₃, ou
e) -(CH₂)ₕ-NR₂₁R₂₂ ;
Z représente un groupe
a) O,
b) S, ou
c) NM ;
W représente un groupe
a) CH,
b) N, ou
c) S ou O lorsque Z représente un groupe NM ;
Y représente un groupe
a) H,
b) F,
c) Cl,
d) Br,
e) alkyle en C₁ à C₃, ou
f) NO₂ ;
X représente un groupe
a) H,
b) -CN,
c) OR₂₇,
d) halogéno,
e) NO₂,
f) tétrazoyle,
g) -SH,
h) -S(=O)ᵢR₄,
i) -S(=O)₂-N=S(O)ⱼR₅R₆,
j) -SC(=O)R₇,
k) -C(=O)R₂₅,
l) -C(=O)NR₂₇R₂₈,
m) -C(=NR₂₉)R₂₅,
n) -C(R₂₅)(R₂₈)-OR₁₃,
o) -C(R₂₅)(R₂₈)-OC(=O)R₁₃,
p) -C(R₂₈)(OR₁₃)-(CH₂)ₕNR₂₇R₂₈,
q) -NR₂₇R₂₈,
r) -N(R₂₇)C(=O)R₇,
s) -N(R₂₇)-S(=O)ᵢR₇,
t) -C(OR₁₄)(OR₁₅)R₂₈,
u) -C(R₂₅) (R₁₆)-NR₂₇R₂₆, ou
v) alkyle en C₁ à C₈ substitué avec un ou plusieurs substituants halogéno, OH, =O autre qu'en position alpha, -S(=O)ᵢR₁₇, -NR₂₇R₂₈, alcényle en C₂ à C₅, alcynyle en C₂ à C₅ ou cycloalkyle en C₃ à C₈ ;
R₄, R₅, R₆, R₇, R₁₃, R₁₄, R₁₅, R₁₆ et R₁₇ répondent aux définitions précitées ;
R₂₅ représente un groupe
a) H,
b) alkyle en C₁ à C₈ facultativement substitué avec un ou plusieurs substituants halogéno, cycloalkyle en C₃ à C₈, alkyle en C₁ à C₄ substitués avec un ou plusieurs substituants - S(=O)ᵢR₁₇, -OR₁₃ ou OC(=O)R₁₃, NR₂₇R₂₈, ou
c) alcényle en C₂ à C₅ facultativement substitué avec un substituant CHO, ou CO₂R₁₃ ;
R₂₆ représente un groupe
a) R₂₈, ou
b) NR₂₇N₂₈ ;
R₂₇ et R₂₈, à chaque emplacement où ils sont présents, sont identiques ou différents et représentent un groupe
a) H,
b) alkyle en C₁ à C₈ ;
c) cycloalkyle en C₃ à C₈ ;
d) -(CH₂)ₘOR₁₃,
e) - (CH₂)ₕ-NR₂₁R₂₂, ou
f) R₂₇ et R₂₈, pris conjointement, représentent un groupe -(CH₂)₂O(CH₂)₂-, -(CH₂)ₕCH(COR₇)-, ou -(CH₂)₂N(CH₂)₂(R₇) ;
R₂₉ représente un groupe
a) -NR₂₇R₂₈,
b) -OR₂₇, ou
c) -NHC(=O)R₂₈ ;
où R₃₀ représente un groupe
a) H,
b) alkyle en C₁ à C₈ facultativement substitué avec un ou plusieurs substituants halogéno, ou
c) alkyle en C₁ à C₈ facultativement substitué avec un ou plusieurs OH, ou alkoxy en C₁ à C₆ ;
où E représente un groupe
a) NR₃₉,
b) -S(=O)ᵢ, ou
c) O ;
R₃₈ représente un groupe
a) H,
b) alkyle en C₁ à C₆ ,
c) -(CH₂)_{q}-aryle, ou
d) halogène ;
R₃₉ représente un groupe
a) H,
b) alkyle en C₁ à C₆ facultativement substitué avec un ou plusieurs substituants OH, halogéno, ou -CN,
c) -(CH₂)_{q}-aryle,
d) -CO₂R₄₀,
e) -COR₄₁,
f) -C(=O)-(CH₂)_{q}-C(=O)R₄₀,
g) -S(=O)₂-(alkyle en C₁ à C₆),
h) -S(=O)₂-(CH₂)_{q}-aryle, ou
i) -(C=O)ⱼ-Het ;
R₄₀ représente un groupe
a) H,
b) alkyle en C₁ à C₆ facultativement substitué avec un ou plusieurs substituants OH, halogéno ou -CN,
c) -(CH₂)_{q}- aryle, ou
d) -(CH₂)_{q}-OR₄₂ ;
R₄₁ représente un groupe
a) alkyle en C₁ à C₆ facultativement substitué avec un ou plusieurs substituants OH, halogéno, ou -CN
b) -(CH₂)_{q}-aryle, ou
c) -(CH₂)_{q}-OR₄₂ ;
R₄₂ représente un groupe
a) H,
b) alkyle en C₁ à C₆,
c) -(CH₂)_{q}-aryle, ou
d) -C(=O)-alkyle en C₁ à C₆ ;
le groupe aryle est un groupe
a) phényle,
b) pyridyle, ou
c) naphtyle ; les groupes a à c étant facultativement substitués avec un ou plusieurs substituants halogéno, -CN, OH, SH, alkyle en C₁ à C₆, alkoxy en C₁ à C₆ ou alkylthio en C₁ à C₆ ;
où R₄₃ représente un groupe
a) H,
b) alkyle en C₁ ou C₂,
c) F, ou
d) OH ;
R₄₄ représente un groupe
a) H,
b) CF₃,
c) alkyle en C₁ à C₃ facultativement substitué avec un ou plusieurs substituants halogéno,
d) phényle facultativement substitué avec un ou plusieurs substituants halogéno,
e) R₄₄ et R₄₅, pris conjointement, représentent un noyau penta-, hexa- ou heptagonal de formule 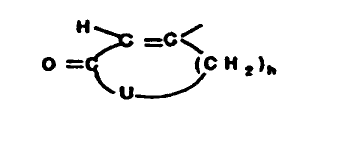
f) R₄₄ et R₄₅, pris conjointement, représentent un groupe -(CH₂)ₖ-, lorsque R₄₆ représente un groupe électrophile ;
R₄₄ et R₄₆, à chaque emplacement où ils sont présents, sont identiques ou différents et représentent
a) un groupe électrophile,
b) H,
c) CF₃,
d) un groupe alkyle en C₁ à C₃ facultativement substitué avec un substituant halogéno,
e) un groupe phényle, sous réserve qu'au moins un des groupes R₄₅ et R₄₆ représente un groupe électrophile, ou
f) R₄₅ et R₄₆, pris conjointement, représentent un noyau penta, hexa ou heptagonal de formule 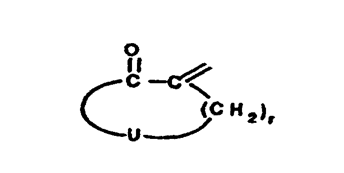
U représente un groupe
a) CH₂,
b) O,
c) S, ou
d) NR₄₇ ;
R₄₇ représente un groupe
a) H, ou
b) alkyle en C₁ à C₅ ;
où R₄₈ représente un groupe
a) carboxyle,
b) halogéno,
c) -CN,
d) mercapto,
e) formyle,
f) CF₃,
g) -NO₂,
h) alkoxy en C₁ à C₆,
i) (alkoxy en C₁ à C₆) carbonyle,
j) alkylthio en C₁ à C₆,
k) acyle en C₁ à C₆,
l) -NR₄₉R₅₀,
m) alkyle en C₁ à C₆ facultativement substitué avec un substituant OH, alkoxy en C₁ à C₅, acyle en C₁ à C₅, ou -NR₄₉R₅₀,
n) (alcényle en C₂ à C₈)phényle facultativement substitué avec un ou deux substituants R₅₁,
o) phényle facultativement substitué avec un ou deux substituants R₅₁,
p) un groupement hétérocyclique penta- ou hexagonal (in) saturé comprenant un à trois atomes choisis dans le groupe consistant en S, N et O, facultativement substitué avec un ou deux substituants R₅₁, ou
q) 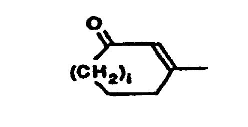
R₄₉ et R₅₀, à chaque emplacement où ils sont présents, sont identiques ou différents, et représentent un groupe
a) H,
b) alkyle en C₁ à C₄ ;
c) cycloalkyle en C₅ ou C₆, ou
d) R₄₉ et R₅₀, pris conjointement avec l'atome d'azote, représentent un groupement hétérocyclique penta- ou hexagonal saturé qui possède facultativement un hétéroatome supplémentaire choisi dans le groupe consistant en S, N et O, et qui peut lui-même être facultativement substitué avec, y compris sur l'atome d'azote supplémentaire, un substituant alkyle en C₁ à C₃ ou acyle en C₁ à C₃ ;
R₅₁ représente un groupe
a) carboxyle,
b) halogéno,
c) -CN,
d) mercapto,
e) formyle,
f) CF₃,
g) -NO₂,
h) alkoxy en C₁ à C₆,
i) (alkoxy en C₁ à C₆)carbonyle,
j) alkylthio en C₁ à C₆,
k) acyle en C₁ à C₆,
l) alkyle en C₁ à C₆ facultativement substitué avec un substituant OH, alkoxy en C₁ à C₅, acyle en C₁ à C₅, ou -NR₄₉R₅₀,
m) phényle,
n) -C(=O)NR₅₂R₅₃,
o) NR₄₉R₅₀,
p) -N (R₅₂)(-SO₂R₅₄),
q) -SO₂-NR₅₂R₅₃, ou
r) -S(=O)ᵢR₅₄ ;
R₅₂ et R₅₃, à chaque emplacement où ils sont présents, sont identiques ou différents et représentent un groupe
a) H,
b) alkyle en C₁ à C₆, ou
c) phényle ;
R₅₄ représente un groupe
a) alkyle en C₁ à C₄, ou
b) phényle facultativement substitué avec un substituant alkyle en C₁ à C₄ ;
où R₅₅ représente un groupe
a) carboxyle,
b) halogéno,
c) -CN,
d) mercapto,
e) formyle,
f) CF₃,
g) -NO₂,
h) alkoxy en C₁ à C₆,
i) (alkoxy en C₁ à C₆)carbonyle,
j) alkylthio en C₁ à C₆,
k) acyle en C₁ à C₆,
l) -NR₅₆R₅₇,
m) alkyle en C₁ à C₆ facultativement substitué avec un substituant OH, alkoxy en C₁ à C₅, acyle en C₁ à C₅ ou -NR₅₆R₅₇,
n) (alcényle en C₂ à C₈)phényle facultativement substitué avec un ou deux substituants R₅₈,
o) phényle facultativement substitué avec un ou deux substituants R₅₈,
p) un groupement hétérocyclique penta- ou hexagonal (in)saturé comprenant un à trois hétéroatomes choisis dans le groupe consistant en S, N et O, facultativement substitué avec un ou deux substituants R₅₈, ou
q) 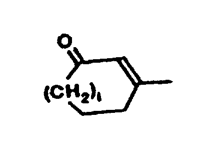
R₅₆ et R₅₇, à chaque emplacement où ils sont présents, sont identiques ou différents et représentent un groupe
a) H,
b) formyle,
c) alkyle en C₁ à C₄,
d) acyle en C₁ à C₄,
e) phényle,
f) cycloalkyle en C₃ à C₆, ou
g) R₅₆ et R₅₇, pris conjointement avec l'atome d'azote, représentent un groupement hétérocyclique penta- ou hexagonal saturé qui possède facultativement un hétéroatome supplémentaire choisi dans le groupe consistant en S, N et 0, et qui peut lui-même être facultativement substitué avec, y compris sur l'atome d'azote supplémentaire, un substituant phényle, pyrimidyle, alkyle en C₁ à C₃ ou acyle en C₁ à C₃ ;
R₅₈ représente un groupe
a) carboxyle,
b) halogéno,
c) -CN,
d) mercapto,
e) formyle,
f) CF₃,
g) NO₂,
h) alkoxy en C₁ à C₆,
i) (alkoxy en C₁ à C₆)carbonyle,
j) alkylthio en C₁ à C₆,
k) acyle en C₁ à C₆,
l) phényle,
m) alkyle en C₁ à C₆ facultativement substitué avec un substituant OH, azido, alkoxy en C₁ à C₅, -NR₆₅R₆₆, -SR₆₇, -O-SO₂R₆₈, ou 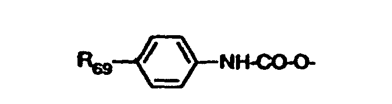
n) -C(=O) NR₅₉R₆₀,
o) -NR₅₆R₅₇,
p) -N(R₅₉)(-SO₂R₆₄),
q) -SO₂-NR₅₉R₆₀,
r) -S(=O)ᵢR₅₄,
s) -CH=N-R₆₁, ou
t) -CH(OH)-SO₃R₆₄ ;
R₅₄ répond à la définition précitée ;
R₅₉ et R₆₀, à chaque emplacement où ils sont présents, sont identiques ou différents et représentent un groupe
a) H,
b) alkyle en C₁ à C₆,
c) phényle, ou
d) tolyle ;
R₆₁ représente un groupe
a) OH,
b) benzyloxy,
c) -NH-C(=O)-NH₂,
d) -NH-C(=S)-NH₂, ou
e) -NH-C(=NH)-NR₆₂R₆₃ ;
R₆₂ et R₆₃, à chaque emplacement où ils sont présents, sont identiques ou différents et représentent un groupe
a) H, ou
b) alkyle en C₁ à C₄, facultativement substitué avec un substituant phényle ou pyridyle ;
R₆₄ représente
a) H, ou
b) un ion sodium ;
R₆₅ et R₆₆, à chaque emplacement où ils sont présents, sont identiques ou différents et représentent un groupe
a) H,
b) formyle,
c) alkyle en C₁ à C₄,
d) acyle en C₁ à C₄,
e) phényle,
f) cycloalkyle en C₃ à C₆,
g) R₆₅ et R₆₆, pris conjointement, représentent un groupement hétérocyclique penta- ou hexagonal saturé comprenant un à trois atomes choisis dans le groupe consistant en S, N et O, facultativement substitué avec, y compris sur l'atome d'azote, un substituant phényle, pyrimidyle, alkyle en C₁ à C₃ ou acyle en C₁ à C₃,
h) -P(O)(OR₇₀)(OR₇₁), ou
i) -SO₂-R₇₂ ;
R₆₇ représente un groupe 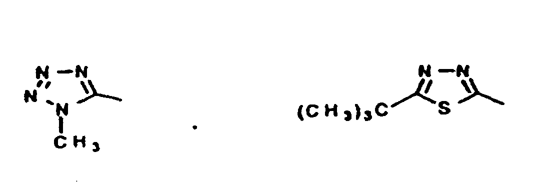 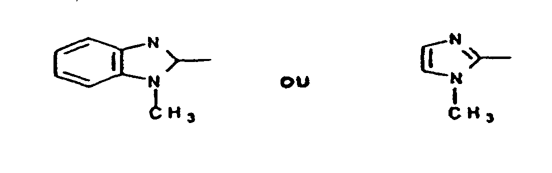
R₆₈ représente un groupe alkyle en C₁ à C₃ ;
R₆₉ représente un groupe
a) (alkoxy en C₁ à C₆) carbonyle, ou
b) carboxyle ;
R₇₀ et R₇₁, à chaque emplacement où ils sont présents, sont identiques ou différents et représentent un groupe
a) H, ou
b) alkyle en C₁ à C₃ ;
R₇₂ représente un groupe
a) méthyle,
b) phényle, ou
c) tolyle ;
où K représente un groupe
a) O, ou
b) S ;
R₇₃, R₇₄, R₇₅, R₇₆ et R₇₇, à chaque emplacement où ils sont présents, sont identiques ou déférents et représentent un groupe
a) H,
b) carboxyle,
c) halogéno,
d) -CN,
e) mercapto,
f) formyle,
g) CF₃,
h) -NO₂,
i) alkoxy en C₁ à C₆,
j) (alkoxy en C₁ à C₆) carbonyle,
k) alkylthio en C₁ à C₆,
l) acyle en C₁ à C₆,
m) -NR₇₈R₇₉,
n) alkyle en C₁ à C₆ facultativement substitué avec un substituant OH, alkoxy en C₁ à C₅, acyle en C₁ à C₅, -NR₇₈R₇₉, -N(phényl)-(CH₂-CH₂-OH), -O-CH(CH₃)(OCH₂CH₃), ou -O-phényl- [para-NHC (=O)CH₃],
o) (alcényle en C₂ à C₈) phényle facultativement substitué avec un substituant R₅₁,
p) phényle facultativement substitué avec un substituant R₅₁, ou
q) un groupement hétérocyclique penta- ou hexagonal (in)saturé possédant un à trois atomes choisis dans le groupe consistant en S, N et O, facultativement substitué avec un substituant R₅₁ ;
R₅₁ répond à la définition précitée ;
R₇₈ et R₇₉, à chaque emplacement où ils sont présents, sont identiques ou différents et représentent un groupe
a) H,
b) alkyle en C₁ à C₄,
c) phényle, ou
d) R₇₈ et R₇₉, pris conjointement avec l'atome d'azote, représentent un groupement hétérocyclique penta ou hexagonal saturé qui possède facultativement un hétéroatome supplémentaire choisi dans le groupe consistant en S, N et 0, et qui peut lui-même être facultativement substitué avec, y compris sur l'atome d'azote supplémentaire, un substituant alkyle en C₁ à C₃ ou acyle en C₁ à C₃,
T représente un groupe
a) O,
b) S, ou
C) SO₂ ;
R₇₅, R₇₆ et R₇₇ répondent aux définitions précitées ;
R₈₀ représente un groupe
a) H,
b) formyle,
c) carboxyle,
d) (alkoxy en C₁ à C₆) carbonyle,
e) alkyle en C₁ à C₈,
f) alcényle en C₂ à C₈,
où les substituants (e) et (f) peuvent être facultativement substitués avec des substituants OH, halogéno, alkoxy en C₁ à C₆, acyle en C₁ à C₆, alkylthio en C₁ à C₆ ou (alkoxy en C₁ à C₆) carbonyle, ou phényle facultativement substitué avec un substituant halogéno,
g) un groupement aromatique ayant 6 à 10 atomes de carbone, facultativement substitué avec un substituant carboxyle, halogéno, -CN, formyle, -NO₂, alkyle en C₁ à C₆, alkoxy en C₁ à C₆, acyle en C₁ à C₆, alkylthio en C₁ à C₆, ou (alkoxy en C₁ à C₆,) carbonyle ;
h) -NR₈₁R₈₂,
i) -OR₉₀,
j) -S(=O)ᵢ-R₉₁, ou
k) -SO₂-N(R₉₂)(R₉₃) ;
R₈₁ et R₈₂, à chaque emplacement où ils sont présents, sont identiques ou différents et représentent un groupe
a) H,
b) cycloalkyle en C₃ à C₆,
c) phényle,
d) acyle en C₁ à C₆,
e) alkyle en C₁ à C₈ facultativement substitué avec un substituant OH, alkoxy en C₁ à C₆ qui peut être substitué avec un substituant OH, un groupement hétérocyclique penta- ou hexagonal aromatique comprenant un à trois atomes choisis dans le groupe consistant en S, N et O, phényle facultativement substitué avec un substituant OH, CF₃, halogéno, -NO₂, alkoxy en C₁ à C₄, -NR₈₃R₈₄, ou 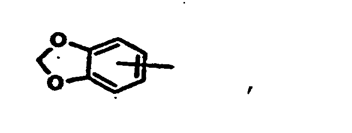
f) 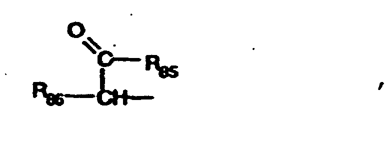 ou
g) 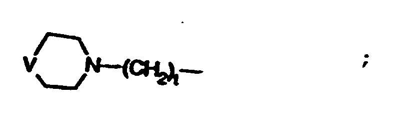
V représente un groupe
a) O,
b) CH₂, ou
c) NR₈₇ ;
R₈₃ et R₈₄, à chaque emplacement où ils sont présents, sont identiques ou différents et représentent un groupe
a) H, ou
b) alkyle en C₁ à C₄ ;
R₈₅ représente un groupe
a) OH,
b) alkoxy en C₁ à C₄, ou
c) -NR₈₈R₈₉ ;
R₈₆ représente un groupe
a) H, ou
b) alkyle en C₁ à C₇ facultativement substitué avec un substituant indonyle, OH, mercaptyle, imidazolyle, méthylthio, amino, phényle facultativement substitué avec un substituant OH, -C(=O)-NH₂, -CO₂H, ou -C(=NH)-NH₂ ;
R₈₇ représente un groupe
a) H,
b) phényle, ou
c) alkyle en C₁ à C₆ facultativement substitué avec un substituant OH ;
R₈₈ et R₈₉, à chaque emplacement où ils sont présents, sont identiques ou différents et représentent un groupe
a) H,
b) alkyle en C₁ à C₆,
c) cycloalkyle en C₃ à C₆, ou
d) phényle ;
R₉₀ représente un groupe
a) alkyle en C₁ à C₈ facultativement substitué avec un substituant alkoxy en C₁ à C₆ ou hydroxy, cycloalkyle en C₃ à C₆, un groupement hétérocyclique aromatique hexagonal, facultativement benzocondensé, comprenant un à trois atomes d'azote, qui peut lui-même être substitué avec un ou deux substituants -NO₂, CF₃, halogéno, -CN, OH, alkyle en C₁ à C₅, alkoxy en C₁ à C₅ ou acyle en C₁ à C₅ ;
b) 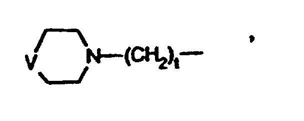
c) phényle, ou
d) pyridyle ;
R₉₁ représente un groupe
a) alkyle en C₁ à C₁₆,
b) alcényle en C₂ à C₁₆,
où les substituants (a) et (b) peuvent être facultativement substitués avec un substituant (alkoxy en C₁ à C₆) carbonyle, ou un groupement hétérocyclique aromatique penta-, hexa- ou heptagonal comprenant un à trois atomes choisis dans le groupe consistant en S, N et O,
c) un groupement aromatique ayant 6 à 10 atomes de carbone, ou
d) un groupement hétérocyclique aromatique penta-, hexa- ou heptagonal comprenant un à trois atomes choisis dans le groupe consistant en S, N et O,
où les substituants (c) et (d) pouvant être facultativement substitués avec des substituants carboxyle, halogéno, -CN, formyle, CF₃, -NO₂, alkyle en C₁ à C₆, alkoxy en C₁ à C₆, acyle en C₁ à C₆, alkylthio en C₁ à C₆, ou (alkoxy en C₁ à C₆) carbonyle ;
R₉₂ et R₉₃, à chaque emplacement où ils sont présents, sont identiques ou différents et représentent un groupe
a) H,
b) phényle,
c) alkyle en C₁ à C₆, ou
d) benzyle ;
R₉₄ et R₉₅, à chaque emplacement où ils sont présents, sont identiques ou différents et représentent un groupe
a) H,
b) OH,
c) alkyle en C₁ à C₆ facultativement substitué avec un substituant -NR₈₃R₈₄, ou
d) R₉₄ et R₉₅, pris conjointement, représentent un groupe =O ;
R₉₆ représente
a) un groupement aromatique ayant 6 à 10 atomes de carbone,
b) un groupement aromatique penta- ou hexagonal, facultativement benzocondensé, comprenant un à trois atomes choisis dans le groupe consistant en S, N et O,
les substituants (a) et (b) pouvant être eux-mêmes substitués avec un ou trois substituants -NO₂, CF₃, halogéno, -CN, OH, phényle, alkyle en C₁ à C₅, alkoxy en C₁ à C₅, ou acyle en C₁ à C₅,
c morpholinyle,
d) OH,
e) alkoxy en C₁ à C₆,
f) -NR₈₃R₈₄,
g ) -C(=O)-R₉₇, ou
h) 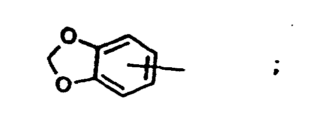
R₉₇ représente un groupe
a) morpholinyle,
b) OH, ou
c) alkoxy en C₁ à C₆,
h est égal à 1, 2 ou 3 ;
i est égal à 0, 1 ou 2 ;
j est égal à 0 ou 1 ;
k est égal à 3, 4 ou 5 ;
l est égal à 2 ou 3 ;
m est égal à 4 ou 5 ;
n est égal à 0, 1, 2, 3, 4 ou 5 ;
p est égal à 0, 1, 2, 3, 4, ou 5 ; sous réserve que n et p, pris conjointement, soient égaux à 1, 2, 3, 4 ou 5 ;
q est égal à 1, 2, 3 ou 4 ;
r est égal à 2, 3 ou 4 ;
t est égal à 0, 1, 2, 3, 4, 5 ou 6 ;
u est égal à 1 ou 2 ;
w est égal à 0, 1, 2 ou 3.

2. Utilisation suivant la revendication 1, dans laquelle ledit mammifère est un être humain.

3. Utilisation suivant la revendication 1, dans laquelle le composé est administré dans l'intervalle d'environ 0,1 à environ 100 mg/Kg de poids corporel du mammifère/jour.

4. Utilisation suivant la revendication 1, dans laquelle le composé est administré par voie orale, nasale, parentérale, topique, transdermique ou rectale.

5. Utilisation suivant la revendication 1, dans laquelle ledit composé est choisi dans le groupe consistant en :
(S)-trans-[[3-[3-fluoro-4-(tétrahydro-1-oxydo-2H-thiopyran-4-yl)phényl]-2-oxo-5-oxazolidinyl]méthyl]-thio-urée ; et
(S)-N-[[3-[3-fluoro-4-(4-thiomorpholinyl)phényl]-2-oxo-5-oxazolidinyl]méthyl]thioacétamide, thiomorpholine S-oxyde ; et
leurs sels pharmaceutiquement acceptables.

6. Utilisation suivant la revendication 1, dans laquelle ledit mammifère ne souffre pas d'une infection bactérienne.

7. Utilisation d'une quantité efficace d'un composé de formule II pour la préparation d'un médicament destiné au traitement ou à la prévention de l'ostéoporose, de la résorption osseuse ou d'une autre maladie osseuse chez un mammifère vertébré, 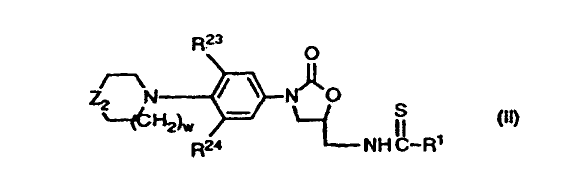 formule dans laquelle Z₂ représente un groupe -O₂S-, -O-, -N(R¹⁰⁷)-, -OS-, ou -S- ;
w est égal à 0, 1, 2 ou 3 ;
R²³ et R²⁴ sont identiques ou différents et peuvent représenter H ou F ; et
R¹ représente H, un groupe NH₂, NH(alkyle en C₁ à C₄) ; N(alkyle en C₁ à C₄)₂ ; 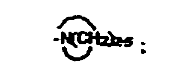 alkyle en C₁ à C₄ ; O(alkyle en C₁ à C₄) ; S(alkyle en C₁ à C₄) ; alkyle en C₁ à C₄ substitué avec 1 à 3 substituants F, 1 à 2 substituants Cl, CN, ou -COO(alkyle en C₁ à C₄), ou cycloalkyle en C₃ à C₆, dans lesquels, à chaque emplacement où il se trouve, le groupe alkyle peut être droit ou ramifié ;
R¹⁰⁷ représente un groupe
a) R¹⁰²O-C(R¹¹⁰)(R¹¹¹)-C(O)-,
b) R¹⁰³O-C(O)-,
c) R¹⁰⁸-C(O)-,
d) R¹⁰⁹-SO₂-,
e) NC-CH₂-,
f) F₂CHCH₂-, ou
g) R¹⁵⁰R¹⁵¹NSO₂₋ ;
où R¹⁰² représente H, un groupe CH₃-, phényl-CH₂-, ou CH₃C(O) ; chacun des groupes R¹¹⁰ ou R¹¹¹ est choisi entre H et un groupe CH₃ ; R¹⁰³ représente un groupe alkyle en C₁ à C₃ ou phényle ; R¹⁰⁸ représente H, un groupe alkyle en C₁ à C₄, aryl(CH₂)₀₋₅, CNCH₂-, ClCH₂-, Cl₂HC-, FH₂HC-, F₂HC-, ou cycloalkyle en C₃ à C₆ ; R¹⁵⁰ et R¹⁵¹ sont identiques ou différents et sont choisis entre H et un groupe alkyle en C₁ à C₄, ou bien R¹⁵⁰ et R¹⁵¹, pris conjointement avec l'atome d'azote auquel chacun est fixé, forment un noyau hétérocyclique, monocyclique ayant 3 à 6 atomes de carbone.

8. Utilisation suivant la revendication 7, dans laquelle ledit mammifère est un être humain.

9. Utilisation suivant la revendication 7, dans laquelle le composé est administré dans l'intervalle d'environ 0,1 à environ 100 mg/Kg de poids corporel du mammifère/jour.

10. Utilisation suivant la revendication 7, dans laquelle le composé est administré par voie orale, nasale, parentérale, topique, transdermique ou rectale.

11. Utilisation suivant la revendication 7, dans laquelle ledit composé est choisi dans le groupe consistant en :
(S)-trans-[[3-[3-fluoro-4-(tétrahydro-1-oxydo-2H-thiopyran-4-yl)phényl]-2-oxo-5-oxazolidinyl]méthyl]-thio-urée ; et
(S)-N-[[3-[3-fluoro-4-(4-thiomorpholinyl)phényl]-2-oxo-5-oxazolidinyl]méthyl]thioacétamide, thiomorpholine S-oxyde ; et
leurs sels pharmaceutiquement acceptables.

12. Utilisation suivant la revendication 7, dans laquelle ledit mammifère ne souffre pas d'une infection bactérienne.
